(19) Europäisches Patentamt
European Patent Office
Office européen des brevets

(11) EP 1 853 261 B1

(12) # EUROPÄISCHE PATENTSCHRIFT

(45) Veröffentlichungstag und Bekanntmachung des Hinweises auf die Patenterteilung:
**11.01.2017 Patentblatt 2017/02**

(21) Anmeldenummer: **06708611.6**

(22) Anmeldetag: **02.03.2006**

(51) Int Cl.:
***A61K 31/4406*** (2006.01) ***A61K 31/4709*** (2006.01)
***A61K 31/4164*** (2006.01) ***A61K 31/505*** (2006.01)
***C07D 213/16*** (2006.01) ***C07D 213/30*** (2006.01)
***C07D 213/55*** (2006.01) ***C07D 213/56*** (2006.01)
***C07D 213/57*** (2006.01) ***C07D 233/54*** (2006.01)
***C07D 239/30*** (2006.01) ***A61P 5/40*** (2006.01)

(86) Internationale Anmeldenummer:
**PCT/EP2006/060410**

(87) Internationale Veröffentlichungsnummer:
**WO 2006/092430 (08.09.2006 Gazette 2006/36)**

(54) **SELEKTIVE HEMMSTOFFE HUMANER CORTICOIDSYNTHASEN**
SELECTIVE INHIBITORS OF HUMAN CORTICOSTEROID SYNTHASES
INHIBITEURS SELECTIFS DE SYNTHASES DE CORTICOIDES HUMAINES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **03.03.2005 DE 102005009705**
**24.06.2005 DE 102005029372**

(43) Veröffentlichungstag der Anmeldung:
**14.11.2007 Patentblatt 2007/46**

(73) Patentinhaber: **Universität des Saarlandes**
**66123 Saarbrücken (DE)**

(72) Erfinder:
- **HARTMANN, Rolf W.**
  **66133 Saarbrücken (DE)**
- **VOETS, Marieke**
  **66424 Homburg/saar (DE)**
- **MÜLLER-VIEIRA, Ursula**
  **66386 St. Ingbert (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) Entgegenhaltungen:
**WO-A-91/17987** **WO-A-92/11240**
**WO-A-2004/046145** **DE-A1- 3 420 592**
**US-A- 3 118 895** **US-A- 3 165 525**
**US-A- 3 541 109** **US-A- 3 719 759**
**US-A- 4 510 149** **US-A- 5 656 643**

- **RAMAN P B ET AL: "Studies on aldosterone biosynthesis in vitro." BIOCHEMISTRY. JUN 1966, Bd. 5, Nr. 6, Juni 1966 (1966-06), Seiten 1795-1804, XP002386728 ISSN: 0006-2960**
- **TEMPLE T E ET AL: "INHIBITORS OF ADRENAL STEROID BIOSYNTHESIS" ELLIOTT, HENRY W. (EDITED BY). ANNUAL REVIEW OF PHARMACOLOGY. VOL. 10. VII + 505P. ILLUS. ANNUAL REVIEWS, INC.: PALO ALTO, CALIF., U.S.A, 1970, Seiten 199-218, XP009068032**
- **CHART J J ET AL: "Inhibitors of adrenal corticosteroid 17alpha-hydroxylation." ENDOCRINOLOGY. SEP 1962, Bd. 71, September 1962 (1962-09), Seiten 479-486, XP009068029 ISSN: 0013-7227**
- **BENCZE W L ET AL: "SELECTIVE ADRENAL CORTICAL AND GONADAL INHIBITORS." JOURNAL OF MEDICINAL AND PHARMACEUTICAL CHEMISTRY. NOV 1962, Bd. 91, November 1962 (1962-11), Seiten 1298-1306, XP009067968 ISSN: 0095-9065**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



**(Forts. nächste Seite)**

- BENCZE W L ET AL: "ADRENAL CORTICAL INHIBITORS AND POTENT SYNTHETIC ESTROGENS." JOURNAL OF MEDICINAL CHEMISTRY. MAR 1965, Bd. 56, März 1965 (1965-03), Seiten 213-214, XP002386730 ISSN: 0022-2623
- HARTMANN R W ET AL: "SYNTHESIS AND EVALUATION OF AZOLE-SUBSTITUTED TETRAHYDRONAPHTHALENES AS INHIBITORS OF P450 AROM, P450 17, AND P450 TXA2" ARCHIV DER PHARMAZIE, VCH VERLAGSGESELLSCHAFT MBH, WEINHEIM, DE, Bd. 329, Nr. 5, Mai 1996 (1996-05), Seiten 251-261, XP009002190 ISSN: 0365-6233
- JOHNSON A L ET AL: "THE SYNTHESIS OF 1 ARYL IMIDAZOLES A NEW CLASS OF STEROID HYDROXYLATION INHIBITORS" JOURNAL OF MEDICINAL CHEMISTRY, Bd. 12, Nr. 6, 1969, Seiten 1024-1028, XP002386731 ISSN: 0022-2623
- DATABASE WPI Section Ch, Week 200035 Derwent Publications Ltd., London, GB; Class B02, AN 2000-411909 XP002386736 & WO 00/31036 A1 (TORII PHARM CO LTD) 2. Juni 2000 (2000-06-02) & WO 00/31036 A (TORII PHARMACEUTICAL CO., LTD; ASHIZAWA, HIROSHI; ITO, YOSHIHARU; WATA) 2. Juni 2000 (2000-06-02)
- MARTIN P MAGUIRE ET AL: "A new series of PDGF receptor tyrosine kinase inhibitors: 3-substituted quinoline derivatives" JOURNAL OF MEDICINAL CHEMISTRY, AMERICAN CHEMICAL SOCIETY. WASHINGTON, US, Bd. 37, 1994, Seiten 2129-2137, XP002133783 ISSN: 0022-2623
- DATABASE CA [Online] CHEMICAL ABSTRACTS SERVICE, COLUMBUS, OHIO, US; COATES, H. ET AL: "New therapeutic agents of the quinoline series. Introduction and I. Monopyridylquinolines" XP002386734 gefunden im STN Database accession no. 1944:518 & JOURNAL OF THE CHEMICAL SOCIETY 401-4 CODEN: JCSOA9; ISSN: 0368-1769, 1943,
- PIGINI ET AL: "Imidazoline Receptors: Qualitative Structure-Activity Relationships and Discovery of Tracizoline and Benazoline. Two Ligands with High Affinity and Unprecedented Selectivity" BIOORGANIC & MEDICINAL CHEMISTRY, ELSEVIER SCIENCE LTD, GB, Bd. 5, Nr. 5, 1997, Seiten 833-841, XP002122337 ISSN: 0968-0896
- HARTMANN R W ET AL: "Discovery of selective CYP11B2 (aldosterone synthase) inhibitors for the therapy of congestive heart failure and myocardial fibrosis" EUROPEAN JOURNAL OF MEDICINAL CHEMISTRY, EDITIONS SCIENTIFIQUE ELSEVIER, PARIS, FR, Bd. 38, Nr. 4, April 2003 (2003-04), Seiten 363-366, XP004425009 ISSN: 0223-5234
- ULMSCHNEIDER S ET AL: "Synthesis and Evaluation of Imidazolylmethylenetetrahydronaphthalenes and Imidazolylmethyleneindanes: Potent Inhibitors of Aldosterone Synthase" JOURNAL OF MEDICINAL CHEMISTRY, AMERICAN CHEMICAL SOCIETY. WASHINGTON, US, Bd. 48, Nr. 6, 2005, Seiten 1796-1805, XP008060860 ISSN: 0022-2623
- ULMSCHNEIDER ET AL: "Development and evaluation of a pharmacophore model for inhibitors of aldosterone synthase (CYP11B2)" BIOORGANIC & MEDICINAL CHEMISTRY LETTERS, OXFORD, GB, Bd. 16, Nr. 1, 1. Januar 2006 (2006-01-01), Seiten 25-30, XP005168853 ISSN: 0960-894X
- VOETS MARIEKE ET AL: "Heteroaryl-substituted naphthalenes and structurally modified derivatives: selective inhibitors of CYP11B2 for the treatment of congestive heart failure and myocardial fibrosis" JOURNAL OF MEDICINAL CHEMISTRY, Bd. 48, Nr. 21, Oktober 2005 (2005-10), Seiten 6632-6642, XP002386732 ISSN: 0022-2623
- VOETS MARIEKE ET AL: "Synthesis and evaluation of heteroaryl-substituted dihydronaphthalenes and indenes: potent and selective inhibitors of aldosterone synthase (CYP11B2) for the treatment of congestive heart failure and myocardial fibrosis." JOURNAL OF MEDICINAL CHEMISTRY. 6 APR 2006, Bd. 49, Nr. 7, 6. April 2006 (2006-04-06), Seiten 2222-2231, XP002386733 ISSN: 0022-2623
- Anthony G. M. Barrett ET AL: "Oxazole Synthesis with Minimal Purification:? Synthesis and Application of a ROMPgel Tosmic Reagent +", Organic Letters, vol. 3, no. 2, 1 January 2001 (2001-01-01) , pages 271-273, XP55123747, ISSN: 1523-7060, DOI: 10.1021/ol006912n
- Drefahl: "4-Aryl-pyrimidine", Journal für praktische Chemie, vol. 23, 1 January 1964 (1964-01-01), pages 324-328, XP055123821,
- Schubert: "Alpha und Beta-Naphthyl-imidazole", Journal für praktische Chemie, vol. 16, 1 January 1962 (1962-01-01), pages 1-7, XP055123830,


Bemerkungen:

Die Akte enthält technische Angaben, die nach dem Eingang der Anmeldung eingereicht wurden und die nicht in dieser Patentschrift enthalten sind.

## Beschreibung

Die Erfindung betrifft Verbindungen zur selektiven Hemmung der humanen Corticoidsynthasen CYP11B1 und CYP11B2, deren Herstellung und Verwendung zur Behandlung von Hypercortisolismus, Diabetes mellitus, Hyperaldosteronismus, Herzinsuffizienz, Myokardfibrose, Depression, altersbedingten kognitiven Einbußen und des metabolischen Syndroms.

## Hintergrund der Erfindung

Die Nebennierendrüsen des Menschen sind in zwei Bereiche untergliedert, das Nebennierenmark und die Nebennierenrinde. Letztere sekretiert eine Reihe von Hormonen, die als Corticoide bekannt sind und in zwei Kategorien fallen. Glucocorticoide (vor allem Hydrocortison bzw. Cortisol) wirken primär auf den Kohlehydrat- und Glucosemetabolismus, sekundär können sie die Wundheilung durch Eingreifen in das Entzündungsgeschehen und die Bildung von fibrosem Gewebe verzögern. Die zweite Kategorie, die Mineralcorticoide, sind primär an der Retention von Natrium und der Exkretion von Kalium beteiligt. Das wichtigste und wirksamste Mineralcorticoid ist Aldosteron.

Die Glucocorticoidbiosynthese wird u. a. von Adrenocorticotropin (ACTH) gesteuert. Steroid-11β-Hydroxylase (CYP11B1) ist das Schlüsselenzym der Biosynthese der Glucocorticoide beim Menschen. In allen Erkrankungen, die mit erhöhter CortisolBildung einhergehen, könnte diesem Enzym somit eine Schlüsselrolle zukommen. Zu diesen Krankheitsbildern zählen Hypercortisolismus, insbesondere das Cushing-Syndrom sowie eine spezielle Form des Diabetes mellitus, die durch einen extremen morgendlichen Anstieg des Cortisolplasmaspiegels gekennzeichnet ist.

Im Falle von Morbus Cushing erfolgt die Therapie in der Regel in Abhängigkeit von der Ursache der Erkrankung. Man unterscheidet zwischen hypophysärhypothalämischem oder adrenal bedingtem Cushing-Syndrom, welches sich aufgrund von Corticoid-produzierenden Tumoren der Nebennierenrinde entwickelt. Zur Therapie des hypophysär-hypothalämischem Cushing-Syndroms werden in der Regel neuromodulatorische Substanzen eingesetzt, wie Bromocriptin, Cyproheptin, Somastatin oder Valproinsäure, die durch ihren Einfluss auf die ACTH-Freisetzung die Cortisolproduktion reduzieren sollen. Diese Therapie erwies sich in der Vergangenheit als nur wenig effektiv.

Beim adrenalen Cushing-Syndrom erfolgt im besonderen dann, wenn eine chirurgische Entfernung des Primärtumors nicht möglich ist, eine Therapie mit Inhibitoren der Steroidbiosynthese. Zum Einsatz kommen die unspezifischen CYP-Enzym-Hemmstoffe Aminoglutethimid, Metyrapon, Ketoconazol und Mitotane, die häufig in Form einer Kombinationstherapie angewendet werden. Die Wirkung auf die Steroidogenese beruht jedoch im Falle von Aminoglutethimid auf einem Angriff an CYP11A1, der Desmolase, bzw. im Falle von Ketoconazol auf der Inhibition von CYP17. Auch die weiteren genannten Verbindungen wirken unspezifisch. Sowohl die Kombination mehrerer unselektiver Inhibitoren der steroidogenen CYP-Enzyme als auch die hohen Dosen, die eingesetzt werden müssen, sind therapeutisch nicht unbedenklich. Dies ist vor allem in Hinblick darauf von Bedeutung, dass die Therapie lebenslang durchgeführt werden muss und aufgrund der mangelnden Selektivität der genannten Verbindungen mit schwerwiegenden Nebenwirkungen behaftet ist (Nieman, L. K., Pituitary 5:77-82 (2002)). Ein Lösungsansatz stellt hier die Therapie mit hochselektiven Inhibitoren des Schlüsselenzyms der Glucocorticoidbiosynthese, CYP11B1 dar. Damit es nicht, wie in der Vergangenheit beschrieben, zu Nebenwirkung insbesondere auf die Androgenbildung beim Mann (Ketoconazol) oder auf die Mineralcorticoidbiosynthese kommt, ist auch hier eine Selektivität der Verbindungen erwünscht.

Erhöhte Cortisolspiegel werden auch mit neurodegenerativen Erkrankungen in Zusammenhang gebracht. Die Abnahme des Erinnerungs- und Lernvermögens nach Exposition mit erhöhten Konzentrationen sowohl exogen zugeführter als auch endogener Glucocorticoide (Cortisol) ist beschrieben (Heffelfinger et al., Dev. Psychopathol. 13:491-513 (2001)).

Bei einer speziellen Form des stressabhängigen Diabetes mellitus kommt es zu einem schnellen morgendlichem Anstieg der Plasma-Cortisolkonzentration. Dieses sog. "Dawn-Phänomen" tritt häufig bei Typ 2-Diabetikern auf und ist durch eine verminderte Glukosetoleranz und eine Abnahme der Insulinsensitivität in den frühen Morgenstunden gekennzeichnet. Das Dawn-Phänomen erschwert die Diabeteseinstellung, so das häufig eine Insulinpumpentherapie notwendig wird. Bezüglich der pathologisch veränderten zirkadianen Rhythmik des Glucosestoffwechsels beim Typ 2-Diabetes gibt es Befunde, die eindeutig darauf hindeuten, dass diese Störung auf einer Elevation nächtlicher Cortisolkonzentrationen beruht (Bolli et al., N. Engl. J. Med. 310 (1984) 746-750; Shapiro et al, J. Clin. Endocrinol. Metab. 72 (1991) 444-454; Schultes und Fehm, Der Internist 9 (2004) 983-993).

Weiterhin werden bei Diabetes mellitus erhöhte Cortisolwerte mit der Entstehung von Insulinresistenz und einer Beeinträchtigung der Glucosetoleranz in Verbindung gebracht (Phillips et al., J. Clin. Endocrinol. Metab. 83:757-760 (1998)). Die Leber spielt eine zentrale Rolle bei der Einstellung des Glucose-Gleichgewichts sowie bei der Entwicklung von Glucoseintoleranz und Typ 2-Diabetes mellitus. Unter physiologischen Bedingungen erfolgt die Glucose-Bereitstellung zu 25 % durch Gluconeogenese (Synthese von Glucose aus Lactat, Pyruvat, Glycerol und Aminosäuren) in der Leber, während in Diabetes mellitus Typ 2-Patienten 90 % der Glucose in der Leber durch Gluconeogenese generiert werden. Glucocorticoide antagonisieren die Insulinwirkung, regulieren die hepat ische Glucosefreisetzung und führen

zu einer Erhöhung der Blutglucosespiegel bei Diabetes mellitus. Ihre Wirkung besteht in der Kontrolle der Transkription mehrerer Gene, welche an der Regulation der hepatischen Gluconeogenese beteiligt sind (DeFronzo et al., Diabetes Rev. 5 (1997) 177-269).

Die gewebespezifische Antwort wird durch den Glucocorticoid-Rezeptor und die intrazelluläre Synthese von aktiven Glucocorticoiden durch 11beta-Hydroxysteroiddehydrogenase Typ 1 (11β-HSD-1) reguliert. 11β-HSD-1 katalysiert die Bildung von Cortisol aus Cortison in der Leber sowie in Adipocyten und den Beta-Zellen des Pankreas und steuert somit die Glucocorticoid-Wirkung in den jeweiligen Zielgeweben (Stewart und Krozowski, Vitam. Horm. 57:249-324 (1999)). Gegenwärtig wird die Anwendung von Inhibitoren der 11β-HSD-1 zur Regulation des Blutzuckerspiegels getestet. Alberts et al. beschreiben in diesem Zusammenhang, dass der selektive 11β-HSD-1 Inhibitor BVT.2733 in hyperglykämischen und hyperinsulinäischen Mäusen sowohl zur Reduktion der Blutglucosespiegel als auch der Insulinspiegel führte (Alberts et al., Diabetologica 45 (2002) 1528-1532). Hier könnten ebenso selektive CYP11 B1-Inhibitoren die erhöhte Cortisolfreisetzung, die zum Anstieg der Blutglucosekonzentration und zu einer Abnahme der Insulinsensitivität führt, reduzieren.

Die Anwendung von Inhibitoren der 11β-HSD-1 zur Regulation des Blutzuckerspiegels wird z.B. in EP 1461333 beschrieben. Die Indikationen für 11β-HSD-1 Inhibitoren gelten entsprechend für die Anwendung von CYP11 B1-Inhibitoren. Auch hier könnte die Inhibition der Glucocorticoid-Biosynthese durch direkte und selektive Hemmung des Schlüsselenzyms CYP11 B1 eine therapeutische Alternative darstellen.

Die Aldosteron-Sekretion wird von einer Vielzahl von Signalen reguliert: den Plasma-Konzentrationen von Natrium und Kalium und dem über mehrere Stufen verlaufenden Renin-Angiotensin-Aldosteron-System (RAAS). Bei diesem System wird als Antwort auf niedrigen Blutdruck von den Nieren Renin sekretiert, das aus einem Vorläuferpeptid Angiotensin I freisetzt. Angiotensin I wird wiederum zu Angiotensin II gespalten, das 8 Aminosäuren umfasst und ein potenter Vasokonstriktor ist. Außerdem wirkt es als Hormon zur Stimulierung der Freisetzung von Aldosteron (Weber, K.T. & Brilla, C.G., Circulation 83:1849-1865 (1991)).

Das Schlüsselenzym der Mineralcorticoid-Biosynthese, CYP1 1 B2 (Aldosteronsynthase), ein mitochondriales Cytochrom-P450-Enzym, katalysiert die Bildung des potentesten Mineralcorticoids Aldosteron aus seinem steroidalen Substrat 11-Deoxycorticosteron (Kawamoto, T. et al., Proc. Natl. Acad. Sci. USA 89:1458-1462 (1992)). Überhöhte Plasma-Aldosteron-Konzentrationen stehen im Zusammenhang mit Krankheitsbildern wie kongestivem Herzversagen und kongestiver Herzinsuffizienz, Myokardialfibrose, ventrikulärer Arrhythmie, Stimulierung kardialer Fibroblasten, kardialer Hypertrophie, renaler Minderperfusion und Hypertonie, und sie sind an der Progression dieser Erkrankungen beteiligt (Brilla, C. G., Herz 25:299-306 (2000)). Insbesondere bei Patienten mit chronischer Herzinsuffizienz bzw. renaler Minderperfusion oder Nierenarterienstenosen kommt es im Gegensatz zur physiologischen Wirkung des Renin-Angiotensin-Systems (RAAS) zu dessen pathophysiologischer Aktivierung (Young, M., Funder, J.W., Trends Endocrinol. Metab. 11:224-226 (2000)). Angiotensin-II-vermittelte Vasokonstriktion und die aufgrund der erhöhten Aldosteronspiegel auftretende Wasser- und Natriumrestriktion führen zu einer zusätzlichen Mehrbelastung des primär schon insuffizienten Myokards. Im Sinne eines "Circulus vitiosus" resultiert eine weitere Verminderung der renalen Perfusion und eine erhöhte Renin-Sekretion. Zusätzlich induzieren sowohl die erhöhten Plasma-Aldosteron- und Angiotensin-II-Spiegel als auch kardial lokal sezerniertes Aldosteron fibrotische Strukturveränderungen des Myokards, in deren Folge die Ausbildung einer Myokard-Fibrose zu einer weiteren Reduktion der Herzleistung führt (Brilla, C. G., Cardiovasc. Res. 47:1-3 (2000); Lijnen, P. & Petrov, V. J. Mol. Cell. Cardiol. 32:865-879 (2000)).

Fibrotische Strukturveränderungen sind gekennzeichnet durch die Entstehung von Gewebe, das durch eine abnormal hohe Menge von fibrotischem Material (v.a. Kollagensträngen) charakterisiert ist. Derartige Fibrosen sind in einigen Situationen, wie z.B. der Wundheilung, nützlich, können jedoch schädlich sein, u.a. wenn sie die Funktion innerer Organe beeinträchtigen. Bei myokardialer Fibrose ist der Herzmuskel von fibrotischen Strängen durchzogen, die den Muskel steif und unflexibel machen und dadurch seine Funktion beeinträchtigen.

Da selbst bei Patienten mit einer leichten Herzinsuffizienz die Mortalität 10-20 % beträgt, ist es dringend erforderlich, hier mit einer geeigneten medikamentösen Therapie einzugreifen. Trotz Langzeittherapie mit Digitalis-Glycosiden, Diuretika, ACE-Hemmern oder AT-II-Antagonisten bleiben die Plasma-Aldosteronspiegel bei den Patienten erhöht, und die Medikation hat keinen Effekt hinsichtlich der fibrotischen Strukturveränderungen.

Mineralcorticoid-Antagonisten, insbesondere Aldosteron-blockierende Wirkstoffe, sind bereits Gegenstand zahlreicher Patente. So blockiert der steroidale Mineralcorticoid-Antagonist Spironolacton (17-Hydroxy-7-alpha-mercapto-3-oxo-17-α-pregn-4-ene-21-carbonsäure-γ-lacton-acetat; Aldactone®) Aldosteron-Rezeptoren kompetitiv zu Aldosteron und verhindert so die Rezeptor-vermittelte Aldosteronwirkung. US 2002/0013303, US 6,150,347 und US 6,608,047 beschreiben die Dosierung von Spironolacton zur Therapie oder Vorbeugung von cardiovaskulären Erkrankungen und myokardialer Fibrose bei gleichzeitiger Erhaltung des normalen Elektrolyt- und Wasserhaushalts des Patienten.

Die "Randomized Aldactone Evaluation Study (RALES)" (Pitt, B. et al., New Engl. J. Med. 341:709-717 (1999)) zeigte eindrucksvoll, dass mit der Gabe des Aldostero n-rezeptor-Antagonisten Spironolacton (Aldactone®) zusätzlich zur Basistherapie mit ACE-Hemmern und Schleifendiuretika die Überlebensrate schwer herzinsuffizienter Patienten

signifikant verbessert werden konnte, da die Wirkung von Aldosteron in ausreichendem Maße inhibiert wurde (Kulbertus, H., Rev. Med. Liege 54:770-772 (1999)). Allerdings war die Anwendung von Spironolacton mit schwerwiegenden Nebenwirkungen wie Gynäkomastie, Dysmenorrhoe und Brustschmerzen verbunden, welche sich mit der steroidalen Struktur der Substanz und den sich daraus ergebenden Wechselwirkungen mit weiteren Steroidrezeptoren begründen (Pitt, B. et al., New Eng. J. Med. 341:709-717 (1999); MacFadyen, R. J. et al., Cardiovasc. Res. 35:30-34 (1997); Soberman, J.E. & Weber, K.T., Curr. Hypertens. Rep. 2:451-456 (2000)).

Mespirenon (15,16-methylene-17spirolactone) und seine Derivate galten als vielversprechende Alternativen zu Spironolacton, da sie nur einen niedrigen Prozentsatz der antiandrogenen Wirkung des Spironolaktons aufweisen (Losert, W. et al., Drug Res. 36:1583-1600 (1986); Nickisch, K. et al., J Med Chem 30(8):1403-1409 (1987); Nickisch, K. et al., J. Med. Chem. 34:2464-2468 (1991); Agarwal, M. K., Lazar, G., Renal Physiol. Biochem. 14:217-223 (1991)). Mespirenon blockiert die Aldosteron-Biosynthese als Teil einer vollständigen Mineralcorticoid-Biosynthese-Hemmung (Weindel, K. et al., Arzneimittelforschung 41 (9):946-949 (1991)). Mespirenone hemmt wie Spironolacton die Aldosteronbiosynthese allerdings nur in sehr hohen Konzentrationen.

WO 01/34132 beschreibt Methoden zur Behandlung, Vorbeugung oder Blockierung von pathogenen Veränderungen infolge von Gefäßverletzungen (Restenosen) in Säugetieren durch Gabe eines Aldosteron-Antagonisten, nämlich Eplerenone (ein Aldosteron-Rezeptor-Antagonist) oder verwandter Strukturen, die teilweise epoxysteroidal sind und sich sämtlich aus 20-Spiroxanen herleiten lassen.

WO 96/40255, US 2002/0123485, US 2003/0220312 und US 2003/0220310 beschreiben therapeutische Methoden zur Behandlung von Kardiovaskularerkrankungen, Myokardfibrose oder kardialer Hypertrophie durch Nutzung einer Kombinationstherapie aus einem Angiotensin- II-Antagonisten und einem epoxy-steroidalen Aldosteron-Rezeptor-Antagonisten wie Eplerone oder Epoxymexrenone.

Die kürzlich veröffentlichte Studie EPHESUS ("Eplerenone’s Heart Failure Efficacy and Survival Study", 2003) konnte die Ergebnisse von RALES untermauern. Ergänzend zur Basistherapie appliziert, reduziert der erste selektive, steroidale Mineralcorticoid-Rezeptor-Antagonist Eplerone (Inspra®) deutlich Morbidität und Mortalität bei Patienten mit akutem Myokardinfarkt sowie das Auftreten von Komplikationen, z.B. Abfall der linksventrikulären Auswurffraktion und Herzversagen (Pitt., B. et al., N. Eng. J. Med. 348:1390-1382 (2003)).

Durch RALES und EPHESUS wurde eindeutig belegt, dass Aldosteronantagonisten eine nicht zu unterschätzende Therapie-Option darstellen. Jedoch ergibt sich aus deren Nebenwirkungsprofil die Forderung nach Substanzen, welche sich in ihrer Struktur und ihrem Wirkungsmechanismus von Spironolacton unterscheiden. Eine viel versprechende Alternative stellen hier nichtsteroidale Inhibitoren der Mineralcorticoid-Biosynthese dar; denn es ist besser, die pathologisch erhöhte Aldosteronkonzentration zu reduzieren, als nur die Rezeptoren zu blockieren. CYP1 1 B2 als Schlüsselenzym bietet sich in diesem Zusammenhang als Angriffspunkt für spezifische Hemmstoffe an und wurde bereits in früheren Untersuchungen als Target für spezifische Inhibitoren vorgeschlagen (Hartmann, R. et al., Eur. J. Med. Chem. 38:363-366 (2003); Ehmer, P. et al., J. Steroid Biochem. Mol. Biol. 81:173-179 (2002)). So kann die überhöhte generalisierte Aldosteronfreisetzung und im besonderen die kardiale Aldosteronproduktion durch die gezielte I nhibition der Biosynthese vermindert werden, was wiederum strukturelle Veränderungen des Myokards reduziert.

Selektive Aldosteronsynthase-Inhibitoren könnten auch eine viel versprechende Stoffklasse darstellen, die nach einem Myokard-Infarkt die Abheilung des beeinträchtigten Myokard-Gewebes mit verringerter Narbenbildung fördert und damit das Auftreten schwerer Komplikationen reduziert.

WO 01/76574 beschreibt ein Arzneimittel, welches einen Hemmstoff der Aldosteronbildung oder eines seiner pharmazeutisch akzeptablen Salze, optional in Kombination mit anderen Wirksubstanzen umfasst. WO 01/76574 bezieht sich dabei auf die Verwendung von zum damaligen Zeitpunkt kommerziell erhältlichen nichtsteroidalen Hemmstoffe der Aldosteronbildung, insbesondere auf das (+)-Enantiomer von Fadrozol, ein 4-(5,6,7,8-tetrahydroimidazo(1,5-a)pyridin-5-yl)benzonitril, und auf dessen synergistische Wirkung mit Angiotensin II-Rezeptor-Antagonisten. Anastrozole (Arimidex®) und Exemestane (Coromasin®) sind weitere non-steroidale Aromatase-Inhibitoren. Ihr Einsatzgebiet ist die Behandlung von Brustkrebs durch Hemmung der Aromatase, die Androstendion und Testosteron in Östrogen umwandelt.

Die humane Steroid-11β-Hydroxylase CYP11B1 zeigt eine Homologie von über 93 % zu humaner CYP11B2 (Kawamoto, T. et al., Proc. Natl. Acad. Sci. USA 89:1458-1462 (1992); Taymans, S. E. et al., J. Clin. Endocrinol. Metab. 83:1033-1036 (1998)). Trotz der hohen strukturellen und funktionellen Ähnlichkeit dieser beiden Enzyme dürfen starke Hemmstoffe der Aldosteronsynthase die Steroid-11 β-Hydroxylase nicht beeinflussen und müssen daher auf ihre Selektivität geprüft werden. Zudem sollten nonsteroidale Inhibitoren der Aldosteronsynthase bevorzugt als Therapeutika einsetzbar sein, da weniger Nebenwirkungen auf das endokrine System zu erwarten sind. Darauf wurde schon in früheren Untersuchungen hingewiesen, ebenso darauf, dass die Entwicklung selektiver CYP11B2-Inhibitoren, die CYP11B1 nicht beeinflussen, durch die hohe Ähnlichkeit der beiden Enzyme erschwert wird (Ehmer, P. et al., J. Steroid Biochem. Mol. Biol. 81:173-179 (2002); Hartmann, R. et al., Eur. J. Med. Chem. 38:363-366 (2003)).

Die Inhibitoren sollten auch andere P450 (CYP)-Enzyme möglichst wenig beeinträchtigen. Der einzige heute bekannte Wirkstoff, der die Corticoidsynthese im Menschen beeinflusst, ist der Aromatase(Östrogensynthase, CYP19)-Inhibitor Fadrozol, der in der Brustkrebstherapie eingesetzt wird. Er kann auch Aldosteron- und Cortison-Pegel

beeinflussen, allerdings erst bei Gabe der zehnfachen therapeutischen Dosis (Demers, L.M. et al., J. Clin. Endocrinol. Metabol. 70:1162-1166 (1990)).

Für Inhibitoren der humanen Aldosteronsynthase CYP11 B2 wurde bereits ein Testsystem zur Durchmusterung von chemischen Verbindungen mit *Schizosaccharomyces pombe-Zellen,* welche humane CYP1 1 B2 stabil exprimieren, und zur anschließenden Prüfung der Selektivität mit V79MZ-Zellen, welche entweder CYP1 1 B2 oder CYP11 B1 stabil exprimieren, entwickelt (Ehmer, P. et al., J. Steroid Biochem. Mol. Biol. 81:173-179 (2002)). Mit Hilfe des S. *pombe-Systems* wurden exemplarisch 10 Substanzen geprüft, von denen eine mit Hilfe des V79MZ-Systems als potenter und selektiver non-steroidaler Inhibitor der humanen CYP11 B2 (und starker Aromatasehemmstoff) und vier weitere als nicht selektive, jedoch gegenüber CYP11 B1 stärkere Inhibitoren identifiziert wurden (A: CYP11B2-Inhibitor; B-D: nicht selektive CYP11B1 - Inhibitoren) :

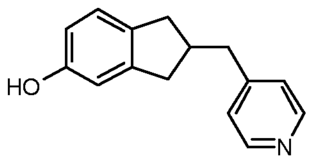

A

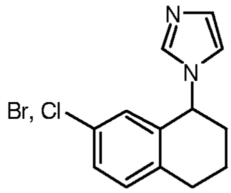

B

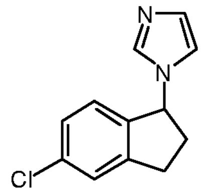

C

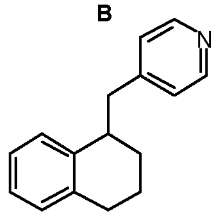

D

Diese Veröffentlichung hat sich jedoch auf die Bereitstellung eines effektiven Testsystems zur Suche nach selektiven CYP11 B2-Inhibitoren konzentriert und gibt außer dem sehr allgemeinen Verweis auf das aromatische N-Atom und die drei oben gezeigten Strukturen nur wenige Hinweise darauf, welche Substanzklassen letztendlich besonders wirksam sein könnten. Des Weiteren sei darauf hingewiesen, dass die meisten in dieser Veröffentlichung vorgestellten Strukturen starke CYP11B1 - Inhibitoren waren und daher nicht zum unmittelbaren Einsatz als selektive CYP11 B2-Inhibitoren in Betracht kommen sollten.

Die Durchmusterung einer P450-Inhibitor-Bibliothek von über 100 Substanzen nach Inhibitoren boviner Aldosteronsynthase (CYP18, CYP11B) (z. T. veröffentlicht in Hartmann, R. W. et al., Arch. Pharm. Pharm. Med. 339, 251-61 (1996)) mit Hilfe des von Ehmer et al. (Ehmer, P. et al., J. Steroid Biochem. Mol. Biol. 81:173-179 (2002)) vorgestellten Testsystems ergab eine hohe Zahl von Verbindungen, die inhibitorisch auf CYP11B2 wirkten (Hartmann, R. et al., Eur. J. Med. Chem. 38:363-366 (2003)). Diese Stoffe wurden im Rahmen der zitierten Untersuchung auch auf ihre orale Verfügbarkeit und des Weiteren auf *in vitro* Inhibition von stabil in Hefe und, falls diese Tests starke Inhibition von CYP11B2 zeigten, in V79MZ-Zellen exprimierter humaner CYP11 B2 geprüft. Es wurden hierbei auch Vergleiche mit der Inhibition anderer CYP, u.a. CYP11B1, exprimiert in V79MZ-Zellen, durchgeführt, um die Selektivität der Testsubstanzen festzustellen. Durch Strukturvariation wurden schließlich CYP11 B2-Inhibitoren gefunden, die $IC_{50}$-Werte im niedrigen nanomolaren Bereich zeigten, nämlich Cyclopropatetrahydronaphthalin-Abkömmlinge und Arylmethyl-substituierte Indane. Es wurde festgestellt, dass die CYP11B-Inhibition durch den Substituenten am Benzolring und durch den Heteroaryl-Rest stark beeinflusst wird. Als vielversprechende Leitstrukturen wurden die Verbindungen E und F gefunden:

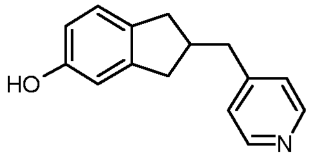

E

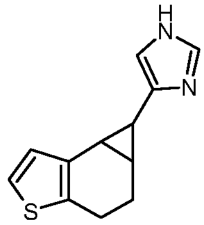

F

Die vorgenannten wissenschaftlichen Veröffentlichungen weisen darauf hin, dass das Vorhandensein eines aromatischen Stickstoff-Atoms wesentlich für die Komplexierung des Eisen-Atoms im Target-Enzym sei (Ehmer, P. et al., J. Steroid Biochem. Mol. Biol. 81:173-179 (2002); Hartmann, R. et al., Eur. J. Med. Chem. 38:363-366 (2003)). Zudem müsse dieses N-Atom unsubstituiert und sterisch zugänglich sein (Ehmer, P. et al., J. Steroid Biochem. Mol. Biol. 81 :173-179 (2002)).

Einige wenige Heteroaryl-substituierte Dihydronaphthaline wurden bereits im Vorfeld der hier vorgestellten Erfindung auf ihre Wirkung als Inhibitoren des unspezifischen bovinen CYP11 B geprüft (Hartmann, R.W. et al., Arch. Pharm. Pharm. Med. Chem. 329:251-261 (1996)) :

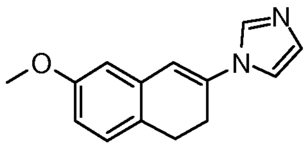

36% Inhibition

**G**

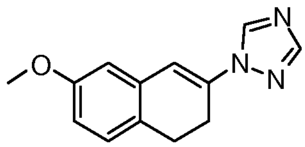

19% Inhibition

**H**

Ihre Wirkung auf CYP17 und CYP19 wird ebenfalls in dieser Veröffentlichung beschrieben. Sie erwiesen sich jedoch als zu unspezifisch, um as Therapeutika zur gezielten Hemmung von CYP11 B2 in Frage zu kommen. Zudem ist das bovine Enzym nicht optimal zur Evaluierung der therapeutischen Eignung von Verbindungen zur Hemmung humaner CYP11 B-Enzyme, da die Homologie zwischen diesem bovinen und den humanen Enzymen nicht hoch ist (75%) (Mornet, E. et. al., J. Biol. Chem. 264:20961 -20967 (1989)).

Des weiteren ist die Wirkung der folgenden Verbindung auf CYP1 7, CYP19 und TxA2 (Thromboxan A2-Synthase) beschrieben, eine inhibitorische Wirkung auf CYP11B wird jedoch nicht erwähnt (Jacobs, C. et al., J. Med. Chem. 43:1841-1851 (2000)):

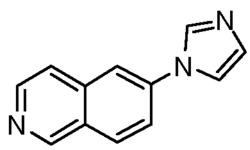

**I**

Weitere 1-Imidazolyl- und 4-Pyridyl-substituierte Naphthaline, Dihydronaphthaline, Chinoline und deren Oxa-Analoga der Formel

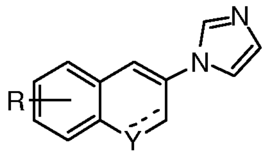

**J** Y = C, O

werden als TxA2-Inhibitoren beschrieben (Cozzi, P. et al., Eur. J. Med. Chem. 26:423-433 (1991)).

Auch

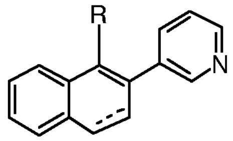

**K** R = H, Me

ist bereits als Inhibitor von CYP17 genannt worden (Bencze, W.L. und Barsky, L.I., J. Med. Pharm. Chem. 5:1298-1306 (1962) und US 3,165,525).

Des weiteren wurde

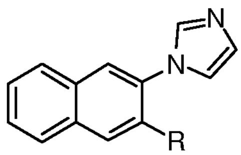

**L** R = H, $NO_2$

als Werkzeug zur Diagnose von primärem und sekundärem Aldosteronismus und Diabetes mellitus in Alternative zu Metyrapon vorgeschlagen (Johnson, A.L. et al., J. Med. Chem. 12(5):1024-1028 (1969)).

Einigen Pyridin-substituierten Chinolinen wird spasmolytische Wirkung zugeschrieben (Hey, D.H. und Williams, J.M., J. Chem. Soc. 1678-83 (1950)).

US 3,098,077 offenbart 2-Pyridylindene und deren Verwendung zur Hemmung einer Überfunktion der Nebennierenrinde.

Alle bislang bekannten Hemmstoffe der Aldosteron- bzw. Glucocorticoidbildung haben erhebliche Nachteile: Etomidat und Metyrapon hemmen die Glucocorticoidbildung stärker als die Aldosteronbildung. Etomidat ist ein starkes Narkotikum und Metyrapon ein relativ unselektiver CYP-Hemmstoff, der deshalb nur als Diagnostikum eingesetzt wird. Bei Fadrozol ist beschrieben, dass es die Aldosteronbildung stärker hemmt als die Glucocorticoidbildung (Bhatnagar, A.S. et al., J. Steroid Biochem. Mol. Biol. 37:1021-1027 (1990); Hausler, A. et al., J. Steroid Biochem. 34:567-570 (1989); Dowsett, M. et al., Clin. Endocrinol. (Oxf.) 32:623-634 (1990); Santen, R.J. et al., J. Clin. Endocrinol. Metabol. 73:99-106 (1991); Demers, L.M. et al., J. Clin. Endocrinol. Metabol. 70:1162-1166 (1990)). Auch diese Substanz kommt für eine Anwendung als Hemmstoff der Aldosteron- oder Glucocorticoidbildung nicht in Frage, da sie ein sehr starker Aromatasehemmstoff ist und daher hochpotent in die Geschlechtshormonbildung eingreift. Im Lichte des vorstehenden Standes der Technik bestand ein Bedürfnis nach potenten und selektiven Inhibitoren der 11ß-Hydrolase CYP11 B1 und der Aldosteronsynthase CYP11 B2.

3-Pyridyl-substituierte Chinoline und Chinoxaline wurden bereits durch eine Fe(salen)Cl-katalysierte Crosscoupling-Reaktion des entsprechenden chlorierten Heteroaryls mit einer Pyridyl-Grignard-Verbindung hergestellt (Fürstner, A. et al., JACS 124:13856-13863 (2002)).

Die 3-Pyridyl-Grignard-Verbindung reagiert auch mit Ethyl-2-chinolinylsulfoxid zu 2-pyridin-3-ylchinolin 20 (Furukawa, N. et al., Tet. Lett. 28(47):5845-8 (1987)). Eine weitere Synthesemethode für diese Verbindung ist die Behandlung von o-Aminobenzaldehyd mit 3-Pyridylmethylketon (Hey, D.H. und Williams, J.M., J.Chem. Soc. 1678-83 (1950)).

3-Pyridin-3-ylchinolin 19 kann durch eine Palladium-katalyisierte Crosscoupling-Reaktion von Tri(chinolinyl)magnesat mit 6-Brompyridin hergestellt werden (Dumouchel, S. et al., Tetrahedron 59:8629-8640 (2003)). 2-Pyridin-3-ylchinoxalin 21 ist durch Umsatz von o-Phenylendiamin mit bromiertem 3-Pyridylmethylketone erhältlich (Sarodnick

G. und Kempter G., Pharmazie 40(6):384-7 (1985)).

Alle genannten Cross-coupling-Reaktionen mit Eisen- oder Palladiumkomplexen haben Nachteile: Zur Herstellung des Eisenkomplexes wird ein zusätzlicher Syntheseschritt notwendig, und die Kopplung von Arylmagnesaten erfordert den teuren Liganden dppf (1,1'-Bis(diphenylphosphino)ferrocen). Die sichere Handhabung der Reagenzien ist zudem schwierig, eine trockene Atmosphäre und niedrige Temperaturen sind unentbehrlich.

Die genannte Reaktion von 3-Pyridylmethylketon mit o-Aminobenzaldehyd oder o-Phenylendiamin resultiert in niedrigen Ausbeuten (maximal 20%). Die Synthese von 4-(6-Methoxy-2-naphthyl)pyridin 31 ist zwar beschrieben (Kelley, C.J., J.Het.Chem. 38(1):11-23 (2001)), involviert jedoch sehr viele Einzelschritte.

Es bestand daher ebenfalls ein Bedarf an einem einfachen Syntheseverfahren für Heteroaryl-substituierte Naphthaline, 3,4-Dihydroxynaphthaline und Indane, das auf ein breites Spektrum von Heteroarylen anwendbar ist.

Zusammenfassung der Erfindung

Es wurde gefunden, dass bestimmte aromatische Verbindungen zur selektiven Hemmung der 11ß-Hydroxylase CYP11 B1 und/oder der Aldosteronsynthase CYP1 1 B2 geeignet sind. Deren biologische Aktivität bezüglich der Hemmung von humaner CYP1 1 B2 und CYP1 1 B1 , sowie zur Feststellung der Selektivität von hum a-ner CYP17 (17α-Hydroxylase-C17,20-lyase, Schlüsselenzym der Androgenbiosynthese) und CYP19 wurde untersucht. Im Vergleich zu bereits beschriebenen CYP11B2-Inhibitoren (Hartmann, R.W. et al., Eur. J. Med. Chem. 38:363-366 (2003)) und zu bekannten Inhibitoren der Corticoidbiosynthese (Fadrozol) bzw. Steroidbiosynthese (Ketoconazol) sind die im folgenden vorgestellten Verbindungen potenter und selektiver.

Weiterhin wurde ein geeignetes Syntheseverfahren für diese aromatischen Verbindungen, deren Hauptvertreter 3-Pyridyl-substituierte Naphthaline, 3,4-Dihydronaphthaline und Indane sind, entwickelt.

Gegenstand der Erfindung sind somit

(1) Verwendung einer Verbindung mit der Struktur der Formel (Ia)

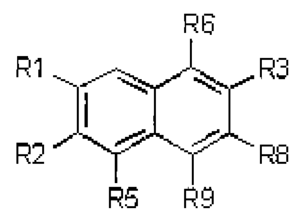

(Ia)

worin

$R^1$ oder $R^2$ Wasserstoff ist und der andere der Substituenten $R^1$ oder $R^2$ ausgewählt ist aus H, Fluor, Chlor, Brom, CN, $COOR^{11}$, Hydroxy, $C_{1-3}$-Alkyl und $C_{1-3}$-Alkoxy;
$R^3$ ausgewählt ist aus Oxazolyl-, Pyridyl-, Imidazolyl- und Pyrimidylresten;
$R^5$ und $R^6$ unabhängig voneinander ausgewählt sind aus H, Fluor, Chlor, Brom;
$R^3$ und $R^9$ H sind;
$R^{11}$ ausgewählt ist aus H und $C_{1-3}$-Alkyl, das geradkettig, verzweigt oder cyclisch, gesättigt oder ungesättigt sein kann;
oder eines pharmazeutisch geeigneten Salzes derselben zur Herstellung eines Medikaments zur Behandlung von Hyperaldosteronismus, Herzinsuffizienz und Myocardfibrose;

(2) eine pharmazeutische Zusammensetzung, enthaltend eine Verbindung der Formel (ia), worin $R^1$, $R^2$, $R^3$, $R^5$, $R^6$, $R^8$, $R^9$ und $R^{11}$ die in (1) angegebene Bedeutung aufweisen, oder ein pharmazeutisch geeignetes Salz derselben, vorausgesetzt dass

(a) $R^3$ nicht 1-imidazolyl ist;
(b) wenn $R^1$, $R^2$, $R^5$ und $R^6$ H-Atome sind, dann $R^3$ nicht 3- oder 4-Pyridyl ist;
(c) wenn $R^2$, $R^5$, und $R^6$ H-Atome sind und $R^1$ COOH ist, dann $R^3$ nicht 4-Pyridyl ist; und
(d) wenn $R^1$, $R^2$, $R^5$ und $R^6$ H-Atome sind, dann $R^3$ kein 2-Hydroxy-3-carboxypyridin-6-yl ist;

(3) eine Verbindung der Formel (Ia), worin $R^1$, $R^2$, $R^3$, $R^5$, $R^6$, $R^8$, $R^9$, $R^{11}$ die in (1) angegebene Bedeutung aufweisen,

oder ein pharmazeutisch geeignetes Salz derselben, vorausgesetzt dass

(a) $R^3$ nicht 1-Imidazolyl ist;
(b) wenn $R^1$, $R^5$ und $R^6$ H-Atome sind, und $R^2$ H oder Methoxy ist, dann $R^3$ nicht Pyridyl, Imidazolyl oder Oxazolyl ist;
(c) wenn $R^2$, $R^5$ und $R^6$ H-Atome sind und $R^1$ COOH ist, dann $R^3$ nicht 4-Pyridyl ist;
(d) wenn $R^1$, $R^2$, $R^4$, $R^5$ und $R^6$ H-Atome sind, dann $R^3$ nicht 3- oder 4-Pyridyl ist; und
(e) die Verbindung nicht 5-(Naphtalen-2-yl)oxazol, 4-Naphtalen-2-yl-pyrimidin, 4(5)-(2-Naphtyl)-1H-imidazol oder 2-(2-Naphtyl)-1H-imidazol ist;

(4) ein Verfahren zur Synthese der Verbindungen gemäß (3), umfassend (i) die Suzuki-Kupplung der Verbindung (Ia) worin $R^3$ ein Halogenatom oder Pseudohalogenid, bevorzugt Br oder OTf ist, mit der Verbindung (II)

$$R3\text{-}B(OH)_2 \qquad \textbf{II}$$

Dehydrierung und eine daran anschliessende Kupplung wobei $R^3$ die in (3) angegebene Bedeutung hat, und funktionelle Gruppen in $R^1$-$R^9$ optional mit geeigneten Schutzgruppen versehen sein können.

## Kurzbeschreibung der Figuren

<u>Fig. 1:</u> Steroldsekretion von H295R-Zellen bei Behandlung mit Inhibitoren der Steroidbiosynthese in Abhängigkeit von der Inhibitorkonzentration nach 6 h Inkubation (Bsp. 9); 1A: Fadrozol, 1B: Ketoconazol, 1C: Verbindung **2.**

## Detaillierte Beschreibung der Erfindung

In den Verbindungen der Formel (Ia) der Erfindung haben die Variablen und die zu ihrer Charakterisierung verwendeten Termini die folgende Bedeutung:

"Alkylreste" und "Alkoxyreste" im Sinne der Erfindung können geradkettig, verzweigt oder cyclisch sein und gesättigt oder (partiell) ungesättigt sein. Bevorzugte Alkylreste und Alkoxyreste sind gesättigt oder weisen eine oder mehrere Doppel- und/oder Dreifachbindungen auf. Dies sind geradkettige oder verzweigte Alkylreste mit 1 bis 6 C-Atomen, solche mit 1 bis 3 C-Atomen sind bevorzugt. Die cyclischen Alkyreste sind mono- oder bicyclische Alkylreste mit 3 bis 15 C-Atomen, insbesondere monocyclische Alkylreste mit 3 bis 8 C-Atomen. "Niederalkylreste" und "Niederalkoxyreste" im Sinne der Erfindung sind geradkettige, verzweigte oder cyclische gesättigte Niederalkylreste und Niederalkoxyreste oder solche mit einer Doppel- oder Dreifachbindung. Bei den geradkettigen sind solche mit 1 bis 6 C-Atomen, insbesondere mit 1 bis 3 C-Atomen besonders bevorzugt. Bei den cyclischen sind solche mit 3 bis 8 C-Atomen besonders bevorzugt.
"Aryle" im Sinne der vorliegenden Erfindung umfassen mono-, bi- und tricyclische Arylreste mit 3 bis 18 Ringatomen, die optional mit einem oder mehreren gesättigten Ringen anelliert sein können. Besonders bevorzugt sind Anthracenyl, Dihydronaphthyl, Fluorenyl, Hydrindanyl, Indanyl, Indenyl, Naphthyl, Naphthenyl, Phenanthrenyl, Phenyl und Tetralinyl.

$R^3$ ist bevorzugt ausgewählt aus Oxazolyl, Imidazolyl, Pyridyl und Pyrimidyl. $R^3$ ist am bevorzugtesten 3-Pyridyl.
"Pharmazeutisch geeignete Salze" im Sinne der vorliegenden Erfindung umfassen dabei Salze der Verbindungen mit organischen Säuren (wie Milchsäure, Essigsäure, Aminosäure, und Oxalsäure), anorganischen Säuren (wie HCl, HBr, und Phosphorsäure) und, falls die Verbindungen Säuresubstituenten aufweisen, auch mit organischen oder anorganischen Basen. Bevorzugt sind Salze mit HCl. Bevorzugt sind Verbindungen der Formel (Ia), wie vorstehend unter (1), (2) und (3) definiert, worin
die Alkylreste und Alkoxyreste gesättigt sind oder eine oder mehrere Doppel- und/oder Dreifachbindungen aufweisen, die geradkettigen oder verzweigten Alkylreste bevorzugt 1 bis 3 C-Atome aufweisen, und die cyclischen Alkylreste mono- oder bicyclische Alkylreste mit 3 bis 15 C-Atomen, bevorzugt monocyclische Alkylreste mit 3 bis 8 C-Atomen sind.
Die Verbindungen der Formel (Ha) können Chiralitätszentren aufweisen. Hier sind sowohl die Isomerengemische als auch die isolierten Einzelverbindungen von der Erfindung eingeschlossen. Bevorzugte Verbindungen der Ausführungsformen (1), (2) und (3) der Erfindung sind solche Verbindungen mit der Formel (Ia)

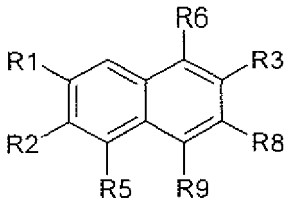

(Ia)

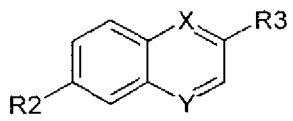

(Ib)

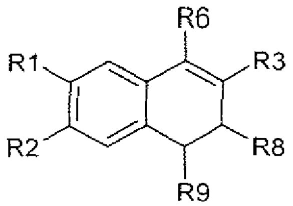

(Ic)

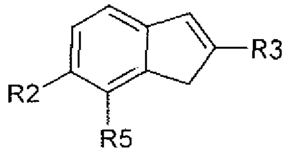

(Id)

in denen $R^3$ ausgewählt ist aus 3- und 4-Pyridyl, 1-Imidazolyl, 4-Imidazolyl und 5-Pyrimidyl, und deren pharmazeutisch geeignete Salze.

Bevorzugte Ausführungsformen der Verbindung (Ia) sind dabei die Verbindungen der folgenden Formeln (Ie) bis (Ii):

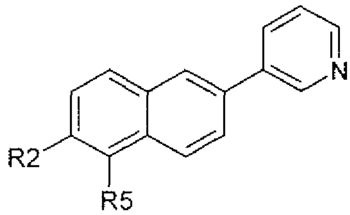

(Ie)

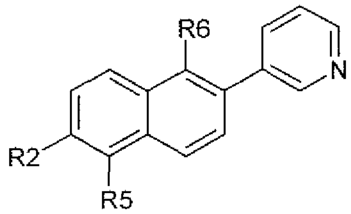

(If)

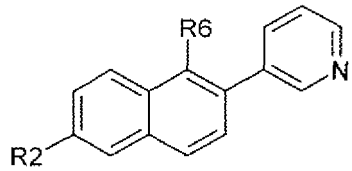

(Ig)

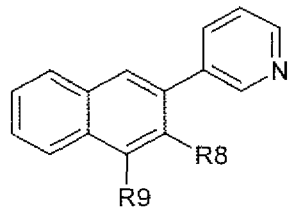

(Ih)

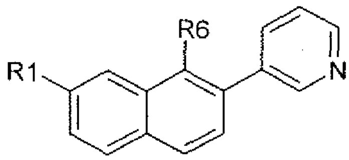

(Ii)

worin $R^1$ und $R^2$ bevorzugt unabhängig voneinander ausgewählt sind aus der Gruppe bestehend aus H, Halogen, CN, Hydroxy, O-$C_{1-3}$alkyl, O-$C_{1-3}$alkenyl,
O-$C_{1-3}$alkinyl, $C_{1-3}$alkyl, $C_{1-3}$alkenyl, $C_{1-3}$alkinyl, -$COOR^{11}$, -$CON(R^{11})_2$ und Arylalkyloxyresten, und besonders bevorzugt H, O-$C_{1-3}$alkyl und Arylalkoxy sind;

$R^5$ ausgewählt ist aus der Gruppe bestehend aus H, Fluor, Chloruns Brom;
$R^6$ ausgewählt ist aus H, Fluor, Chlor und Brom;
$R^8$ und $R^9$ sind;
und deren pharmazeutisch geeignete Salze.

Ganz besonders bevorzugt ist $R^3$ in allen Ausführungsformen der Erfindung 3-Pyridyl.

Bevorzugte Verbindungen der Formel (Ia) für Ausführungsform (1), (2) und (3) sind insbesondere die folgenden Verbindungen: 3-(2-naphthyl)pyridin; 3-(1 ,5-Dichlor-6-methoxy-2--naphthyl)pyridin; 3-(6-Methoxy-2-naphthyl)pyridin; 3-(6-Ethoxy-2-naphthyl)pyridin; 3-(6-Brom-2-naphthyl)pyridin; Methyl-6-pyridin-3-yl-2-naphthoat; 6-Pyridin-3-yl-2-naphthonitril.

Die erfindungsgemäßen chemischen Verbindungen, insbesondere die Verbindungen der Ausführungsform (3) können in einem Aspekt des Verfahrens gemäß Ausführungsform (4) durch eine Suzuki-Kupplung der Verbindung (III) mit Verbindung (II) synthetisiert werden (vgl. Bsp. 1 und 2). Das Verfahren erfolgt bevorzugt gemäß dem folgenden allgemeinen Syntheseschema:

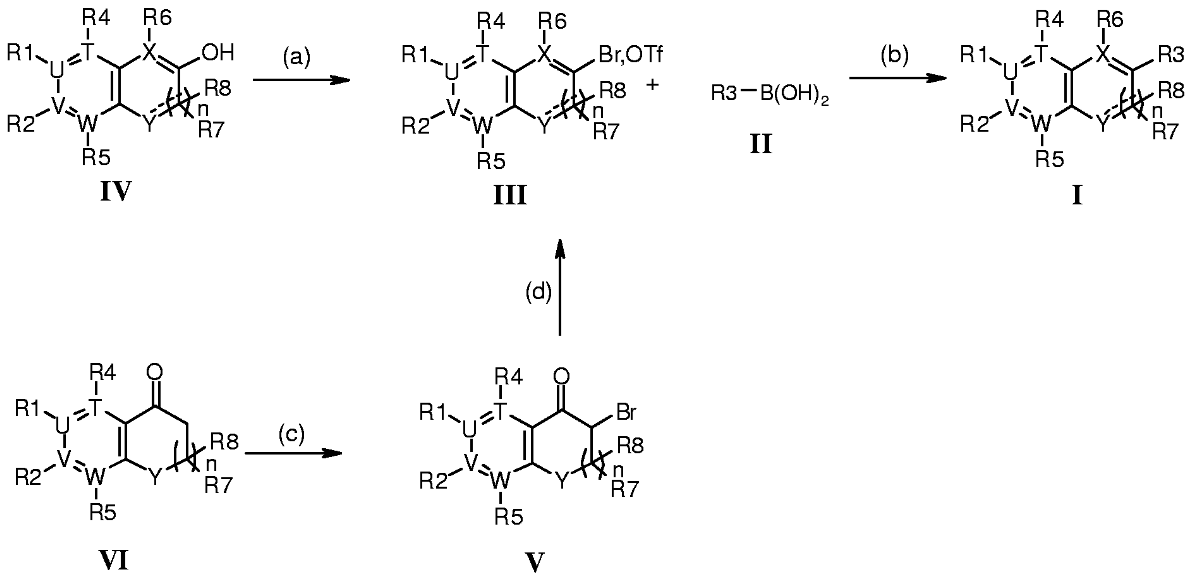

IV III II I VI V

Reaktionsbedingungen: (a) $(CF_3SO_2)_2O$, Pyridin, 30 min bei 0 °C und über Nacht bei RT (b) $PdP(Ph_3)_4$, $Na_2CO_3$, Toluol oder DME, 80 °C (c) $TBABr_3$, $CH_2Cl_2$, RT; (d) 1) $NaBH_4$, MeOH, 0 °C 2) pTSA, Toluol, Reflux.

Schlüsselschritt dieser Synthese ist die Suzuki-Kupplung verschiedener heterozyklischer Boronsäuren mit Brom- oder Triflat-substituierten bizyklischen Verbindungen. Die Triflate (III) werden in einem ersten Schritt ausgehend von den entsprechenden Alkoholen (IV) durch Reaktion mit Trifluormethansulfonsäureanhydrid und Pyridin synthetisiert. Im zweiten Schritt werden die Brom- oder Triflatverbindungen (III) und die herterozyklischen Boronsäuren (II) durch eine Suzukireaktion mit $PdP(Ph_3)_4$ als Katalysator, $Na_2CO_3$ als Base und Toluol oder DME als Lösemittel gekoppelt. Nach Aufarbeitung werden die Produkte durch Säulechromatgraphie gereinigt und mit NMR charakterisiert.

Die zur Synthese der erfindungsgemäßen chemischen Verbindungen benötigten Verbindungen (III) können in einem weiteren Aspekt des Verfahrens gemäß Ausführungsform (4) hergestellt werden durch $TBABr_3$-Behandlung der Ketone (VI), welche diese in die entsprechenden $\alpha$-Bromketone (V) überführt (Bsp. 2). Nach Reduktion mit $NaBH_4$ werden die resultierende Alkohole in Toluol unter Zusatz einer katalytischen Menge pTSA rückflussgekocht, wodurch die dehydratisierten Bromverbindungen (III) entstehen. Der letzte Schritt ist die oben geschilderte Suzukikopplung der Bromverbindungen (III) mit den Verbindungen (II).

Die Reaktionsprodukte können in ihre stabilen Salze überführt werden, bevorzugt in HCl-Salze oder pharmazeutisch akzeptable Salze.

Die erfindungsgemäßen Synthesen können zur Herstellung der erfindungsgemäßen Verbindungen und ähnlicher Verbindungen verwendet werden. Die Ausbeuten werden durch die erfindungsgemäße Synthese gegenüber bereits bekannten Verfahren in einigen Fällen deutlich erhöht (um bis zu 40%). So beträgt die Ausbeute für 4-(6-Methoxy-3,4-dihydronaphthalin-2-yl)pyridin 43 in der Literatur 21% (Kelley, C.J. et al., J. Het. Chem. 38(1):11-23 (2001)), während sie bei Anwendung der erfindungsgemäßen Synthese 61% beträgt.

Für die Synthese (4) zur Herstellung von Verbindungen mit deprotonierbaren Substituenten bzw. funktionellen Gruppen der heterozyklischen Verbindungen ist es erforderlich, diese mit geeigneten Schutzgruppen zu versehen. Geeignete Schutzgruppen und deren Entfernung sind dem Fachmann z.B. aus T.W. Green, Protective Groups in Organic Synthesis, Harvard University, John Wiley & Sons (1981) zugänglich. Die bedeutet selbstverständlich, dass bei Verwendung solcher Schutzgruppen in dem erfindungsgemäßen Verfahren (4) ein nachgeschalteter Entschützungsschritt notwendig ist.

Die Prüfung der erfindungsgemäßen Verbindungen auf Verwendung nach Ausführungsform (1) erfolgt an *in vitro*-Testsystemen, bevorzugt an mehr als einem *in vitro*-Testsystem. Die erste Stufe umfasst die Prüfung mit humanen CYP11B-Enzymen, bevorzugt humanen CYP11 B1 und CYP1 1 B2. Diese humanen Enzyme können entweder rekombinant exprimiert werden, insbesondere in *Schizosaccharomyces pombe* oder V79-Zellen, oder in einer getesteten Human-Zellinie, insbesondere der adrenocorticalen Tumorzelllinie NCI-H295R enthalten sein (vgl. Bsp. 4 und 8). Besonders bevorzugt werden Substanzen zur erfindungsgemäßen Verwendung nach (1) eingesetzt, welche eine Wirkung auf humane CYP11 B-Enzyme zeigen. Zur Identifizierung neuer therapeutisch wirksamer Verbindungen gemäß Ausführungsform (1) für den Menschen sind insbesondere Spalthefe und V79MZh-Zellen, welche CYP11 B1 und CYP1 1 B2 rekombinant exprimieren, und NCI-H295R-Zellen geeignet.

Zur erfindungsgemäßen Hemmung von humanem CYP1 1 B2 sind besonders diejenigen Verbindungen geeignet, deren Selektivitätsfaktor ($IC_{50}$ CYP11B1/$IC_{50}$ CYP1 1 B2) höher als 50 ist, ganz besonders diejeningen, deren $IC_{50}$ CYP11B2 kleiner als 20 nM ist.

Insbesondere die 3-Pyridyl-substituierten Naphthalinderivate und 3,4-Dihydronaphthaline gemäß Ausführungsform (1) sind zur Verwendung nach (1) geeignet. Zur selektiven Inhibition von CYP11 B2 sind dies besonders die Napthalinderivate 2, 4, 5 und 10 sowie die Dihydronaphthalinderivate 24, 33, 35 und 38 (vgl. Bsp. 6-7) der vorliegenden Erfindung. Ganz besonders bevorzugt sind 3-(6-Methoxy-2-naphthyl)pyridin 2 und 3-(6-Methoxy-3,4-dihydronaphthalin-2-yl)pyridin 35 (Bsp. 6); letzteres ist ein hochpotenter CYP11B2-Inhibitor ($IC_{50}$: 2 nM), der eine hundertfache Selektivität im Vergleich mit CYP11 B1 ($IC_{50}$: 213 nM) aufweist. Diese Verbindung stellt darüberhinaus eine vielversprechende Leitstruktur für weitere therapeutische Agentien dar.

Zur Bestimmung der Inhibition von humaner CYP11 B2 durch die Testverbindungen kann ein Screening-Test in rekombinanter S. *pombe,* insbesondere CYP11B2-exprimierender S. *pombe* P1, verwendet werden (Bsp. 4A). Für eine weitere Untersuchung auf den Einsatz gemäß Verwendung (5) werden danach besonders solche Verbindungen ausgewählt, die eine höhere inhibitorische Wirkung als die Referenz Fadrozol zeigen.

In der zweiten Stufe können Verbindungen auf ihre Verwendung in V79 MZh-Zellen (Hamsterlungen-Fibroblasten), die entweder CYP11B1 oder CYP11B2 exprimieren, auf ihre Aktivität und Selektivität getestet werden (Bsp. 4B). Es werden unterschiedliche Inhibitionsprofile gefunden: Inhibitoren, die entweder selektiv für CYP11B1 oder für CYP11B2 sind, und Inhibitoren die beide CYP11B- Enzyme hemmen können.

Die Inhibition von CYP19 durch die Testverbindungen kann *in vitro* unter Verwendung von humanen placentalen Mikrosomen und [1$\beta$, 2$\beta$-$^3$H]Testosteron als Substrat durchgeführt werden (modifiziert nach: Thompson, E.A. Jr. & Siterii, P.K., J. Biol. Chem. 249:5364-5372 (1974)) (Bsp. 3).

Die Inhibition von CYP 17 durch die Testsubstanzen kann *in vitro* mit einer CYP17-haltigen Membranfraktion aus *E.coli,* das CYP17 rekombinant exprimiert, und Progesteron als Substrat bestimmt werden (Bsp. 3).

Die NCI-H295R-Zellinie ist kommerziell erhältlich und wird häufig als Modell für den humanen adrenalen Kortex verwendet. Die Zellen wurden 1980 erstmals isoliert (Gazdar, A.F. et al., Cancer Res. 50:5488-5496 (1990)) und enthalten 5 steroidogene CYP450-Enzyme, darunter 17-alpha-Hydroxylase, CYP11 B1 und CYP1 1 B2. Da in dieser Zelllinie sämtliche steroidogenen CYP-Enzyme, die im adrenalen Cortex vorkommen, exprimiert werden, stellt sie ein wichtiges Instrument bei der Abschätzung der Selektivität von Hemmstoffen *in vitro* dar. Wesentlicher Unterschied zu den V79-Zellen ist folglich nicht nur, dass es sich bei NCI-H295R um humane Zellen handelt, sondern auch, dass in V79MZh11B1 bzw. V79MZh11B2 jeweils nur ein Targetenzym rekombinant exprimiert in einem ansonsten völlig CYP-Enzym-freien System vorliegt, während NCI-H295R ein wesentlich komplexeres Modell darstellt. Mit Hilfe dieses neuen Modells kann die Voraussage von Wirkungen und Nebenwirkungen von Verbindungen auf die komplexen Enzyme der Nebennierenrinde deutlich präziser werden.

Die Beeinflussung von humanem CYP11B1 und CYP11B2 in NCI-H295R-Zellen durch die in vorliegender Erfindung gefundenen Substanzen wurde erstmals anhand ein i-ger weniger Verbindungen exemplarisch getestet (Bsp. 8).

Unter Verwendung der NCI-H295R-Zelllinie wurde ein weiteres Testsystem zur Evaluierung der erfindungsgemäßen Substanzen etabliert (Bsp. 9). Hintergrund dieses Testsystems ist, dass H295R alle steroidogenen CYP-Enzyme, die zur Synthese der adrenalen Steroide notwendig sind, innerhalb der Zelle exprimiert. In der intakten humanen Nebennierenrinde hingegen erfolgt die Bildung der Mineralkortikoide in der Zona glomerolosa, die der Glukokortikoide in der Zona fasciculata und die der adrenalen Androgene in der Zona reticularis. In H295R liegt diese Zonierung nicht vor, dennoch lassen sich die Konzentrationen an Steroiden, die durch H295R sezerniert werden, qualitativ und quantitativ mit den von der intakten humanen Nebennierenrinde freigesetzten Konzentrationen vergleichen.

In diesem Modell wird die Cortisolbildung stärker gehemmt als die Aldosteronbildung, wenn Verbindung 2 als Testsubstanz eingesetzt wird (Bsp. 9, Tab. 5). Dieser Umstand kann dazu genutzt werden, auch indirekte Inhibitoren von CYP11 B1 nach Ausführungsform (5) zur Herstellung von Arzneimitteln gemäß Ausführungsform (2) zur Therapie von Hypercortisolismus und Diabetes mellitus zu verwenden. Grund für die indirekte Inhibition durch Verbindung 2, welche im V79MZ-System sehr selektiv für CYP1 1 B2 ist, ist möglicherweise die Affinität dieser Verbindung zu CYP17, das in H295R hochaktiv ist. Möglicherweise führt die Inhibition von CYP17 zu einer Kumulation seiner Substrate Progesteron und Pregnenolon. Infolgedessen kann der Abbau dieser Steroide über die CYP21- und CYP11B2-Stoffwechselwege führen, was wiederum zu erhöhten Konzentrationen des CYP11B2-Substrats DOC führt. CYP11B2-Inhibitoren konkurrieren dann mit diesen erhöhten Konzentrationen von DOC um die Bindungsstellen am aktiven Zentrum des

Proteins, was in höheren $IC_{50}$-Werten resultiert. Außerdem führt eine Inhibition von CYP17 zusätzlich zu einer Erniedrigung des CYP11B1-Substrats RSS und zu verminderten $IC_{50}$-Werten dieses Enzyms. Diese Kaskade kann folglich eine selektive CYP11B2-Inhibition in diesem Testsystem maskieren. Systemisch betrachtet können die erfindungsgemäßen Verbindungen also die Steroidogenese über die direkte Wirkung auf einzelne Enzyme hinaus erheblich beeinflussen.

Die *in vivo*-Aktivität der hier vorgestellten Verbindungen konnte in ersten Versuchen am Rattenmodell gezeigt werden. Fadrozol senkt die Aldosteron- und Corticosteronkonzentrationen in ACTH-stimulierten Ratten ab (Häusler et al., J. Steroid Biochem. 34:567-570 (1989)). Einige der hier vorgestellten Verbindungen zeigten *in vivo* ein ähnliches Verhalten wie Fadrozol.

Die zur Verwendung nach Ausführungsform (1) geeigneten Substanzen der Formel (I) können zur Entwicklung eines Arzneistoffs enthaltend eine pharmazeutische Zubereitung gemäß Ausführungsform (2) dienen, der die Lebensqualität von Patienten mit Herzinsuffizienz bzw. myokardialer Fibrose verbessert und die Mortalität entscheidend reduzieren kann. Die Ergebnisse der vorliegenden Erfindung zeigen eindeutig, dass es möglich ist, für das Targetenzym CYP11B2 Inhibitoren zu entwickeln, die hochaktiv sind, die allerdings CYP11B1, welches eine große strukturelle und funktionelle Homologie zu CYP11B2 aufweist, nur wenig beeinflussen, und umgekehrt.

Die pharmazeutische Zusammensetzung gemäß Ausführungsform (2) enthält vorzugsweise eine derjenigen Verbindungen der Formel (Ia), die bevorzugt für die Verwendung nach Ausführungsform (1) verwendet werden. Sie ist zur Therapie von Hyperaldosteronismus, Herzinsuffizienz oder myokardialer Fibrose und Hypercortisolismus bei Säugetieren und insbesondere Menschen geeignet.

Die erfindungsgemäßen Verbindungen sind als Einzelverbindungen und in Kombination mit anderen Wirkstoffen und Hilfsstoffen z. B. zur Hemmung von humanen und Säugetier-P450-Oxygenasen, besonders zur Hemmung der humanen oder Säugetier-Aldosteronsynthase, ganz besonders zur Hemmung der humanen Aldosteronsynthase CYP11B2 bei gleichzeitiger geringer Beeinträchtigung der humanen CYP11B1 *in vitro* und *in vivo* geeignet. Die für CYP11B2 selektiven Verbindungen können zur Herstellung von Arzneimitteln zur Therapie von Herzinsuffizienz, (myo)kardialer Fibrose, (kongestivem) Herzversagen, Hypertonie und primärem Hyperaldosteronismus beim Menschen und bei Säugetieren eingesetzt werden. Diese Arzneimittel bzw. die pharmazeutischen Zusammensetzungen gemäß Ausführungsform (2) der Erfindung können neben den erfindungsgemäßen Verbindungen noch weitere Wirkstoffe sowie geeignete Hilfs- und Trägerstoffe enthalten. Geeignete Hilfs- und Trägerstoffe werden vom Fachmann in Abhängigkeit von Anwendungsgebiet und Applikationsform bestimmt.

Die Erfindung umfasst darüber hinaus die Verwendung der erfindungsgemäßen Verbindung zur Vorbeugung, Verlangsamung des Verlaufs oder Therapie des Hyperaldosteronismus, der Myokardfibrose, des kongestiven Herzversagens oder der kongestiven Herzinsuffizienz und umfasst die Gabe einer wirksamen Dosis eines erfindungsgemäßen Aldosteronsynthase-Inhibitors oder eines pharmazeutisch akzeptablen Salzes davon an den betroffenen Menschen oder das betroffene Säugetier.

Die Verbindungen der vorliegenden Erfindung können in jeder dem Fachmann geläufigen Applikationsform verabreicht werden, wobei jedoch die orale Applikation die bevorzugte Applikationsform ist.

Die Menge verabreichten Wirksoff, d. h. die eingesetzte Dosis richtet sich dabei nach der Art und Schwere der zu behandelnden Krankheit, der Applikations- und Therapieform, dem Alter und der konstitutionellen Beschaffenheit des Patienten und wird von dem behandelnden Arzt individuell im Rahmen seines allgemeinen Fachwissens an die gegebene Situation angepasst.

Die Erfindung wird anhand der nachfolgenden Beispiele näher erläutert. Verbindungen, die nicht unter den Schutzumfang der Ansprüche fallen, dienen zur Erläuterung der Erfindung.

**Beispiele**

Material und Analysenmethoden: IR-Spektren wurden auf einem Bruker Vector 33 FT-Infrarotspektrometer aufgenommen. $^1$H-NMR-Spektren wurden auf einem Bruker DRX-500 (500 MHz)-Gerät aufgenommen. Chemische Verschiebungen werden in parts per million (ppm) angegeben. Alle Kopplungskonstanten (J) sind in Hz angegeben. Reagentien und Lösungsmittel stammten aus kommerziellen Quellen und wurden ohne weitere Reinigung verwendet. Säulenchromatographie (CC) wurde über Silicagel (70-200 $\mu$m) durchgeführt, der Reaktionsverlauf wurde mit Hilfe von Dünnschichtchromatographie über ALUGRAM SIL G/$UV_{254}$-Platten (Macherey-Nagel, Düren) nachgewiesen.

Beispiel 1: Synthese der Verbindungen **1** bis **31**

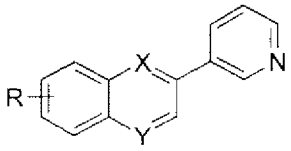

1-21

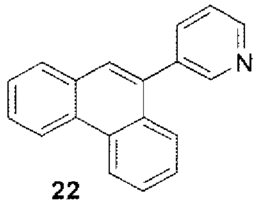

22

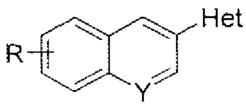

23-31

| Nr. | R | X/Y | Nr. | R | | X/Y | Nr. | R | Y | Het |
|---|---|---|---|---|---|---|---|---|---|---|
| **1** | H | CH | **12** | 1,5-Cl, 6-OMe | | CH | **23** | H | CH | 1-Imidazolyl |
| 2 | 6-OMe | CH | 13 | 7-OMe | | CH | 24 | 3-OMe | CH | 1-imidazolyl |
| 3 | 6-OH | CH | 14 | 1-Cl-7-OMe | | CH | 25 | 6-OMe | CH | 1-imidazolyl |
| 4 | 6-Br | CH | 15 | 3-OMe | | CH | 26 | H | N | 1-imidazolyl |
| 5 | 6-OEt | CH | 16 | 6- COOMe | | CH | 27 | H | CH | 4(5)-imidazolyl |
| 6 | 6-OPr | CH | 17 | 6-$CONH_2$ | | CH | 28 | H | CH | 5-(N-methyl) imidazolyl |
| 7 | 6-OBn | CH | 18 | 6-CONHMe | | CH | 29 | H | CH | 5-oxazolyl |
| 8 | 6-CN | CH | no | R | X | Y | 30 | 6-OMe | CH | 5-pyrimidyl |
| 9 | 5-Cl-6-OMe | CH | 19 | H | CH | N | 31 | 6-OMe | CH | 4-pyridyl |
| 10 | 5-Br-6-OMe | CH | 20 | H | N | CH | | | | |
| 11 | 5-Pyr-6-OMe | CH | 21 | H | N | N | | | | |

Die Synthese erfolgte gemäß dem allgemeinen Syntheseschema:

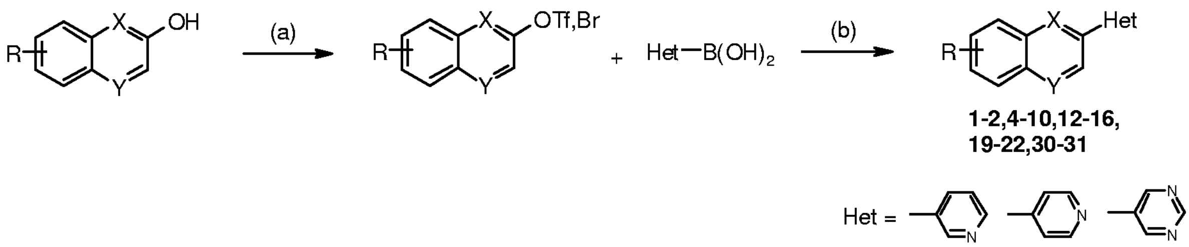


1-2,4-10,12-16, 19-22,30-31

Reaktionsbedingungen: (a) $(CF_3SO)_2O$, Pyridin, 30 min bei 0 °C, über Nacht bei RT (b) $PdP(Ph_3)_4$, $Na_2CO_3$, Toluol oder DME, 80 °C

Schlüsselschritt der Synthese war die Suzuki-Kupplung von verschiedenen heterozyklischen Boronsäuren mit Brom- oder Triflatverbindungen. Die Triflate (III) wurden in einem ersten Schritt ausgehend von den entsprechenden Alkoholen (IV) durch Reaktion mit Trifluormethansulfonsäureanhydrid und Pyridin synthetisiert. Die Bromverbindungen ware kommerziell erhältlich oder wurden wie unter (A) beschrieben hergestellt. Im zweiten Schritt wurden die Brom- oder Triflatverbindungen (III) und die heterozyklischen Boronsäuren (II) durch eine Suzukireaktion mit $PdP(Ph_3)_4$ als Katalysator, $Na_2CO_3$ als Base und Toluol oder DME als Lösemittel gekoppelt. Das nach der Reaktion erhaltene Gemisch wurde durch Säulenchromatographie gereinigt. Die Produkte wurden durch NMR charakterisiert.

A) Synthese der nicht kommerziell erhältlichen Vorstufen: Die folgenden Verbindungen wurden nach neuen oder bekannten Synthesemethoden hergestellt:

2-Brom-6-ethoxynaphthalin 5i, 2-Brom-6-propoxynaphthalin 6i und 2-Brom-6-phenoxynaphthalin 7i wurden analog zu Huisgen et al. (leicht modifiziert) hergestellt (Huisgen, R. und Sorge, G., Liebigs Ann. Chem. 566:162-184 (1950)):

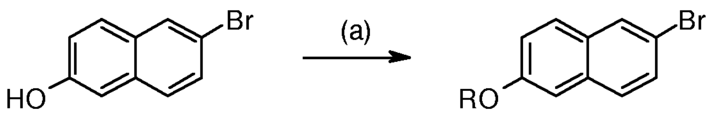


**5i** R=Et
**6i** R=Pr
**7i** R=Bn

Reaktionsbedingungen: (a) RBr, $K_2CO_3$, DMF, 3 h, Reflux

6-Brom-1-chlor-2-methoxynaphthalin 9i und 1,6-Dibrom-2-methoxynaphthalin 10i (neue Synthesemethode):

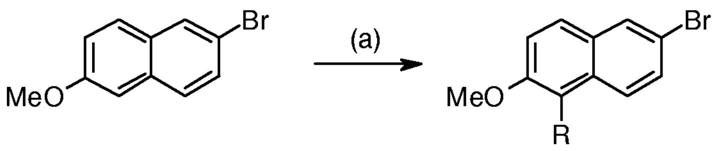


**9i** R=Cl
**10i** R=Br

Reaktionsbedingungen: (a) NBS oder NCS, THF, 3 h Reflux, über Nacht bei RT

1,5-Dichlor-6-methoxy-2-naphthol 12ii wurde anolog zu WO 03/051805 hergestellt:

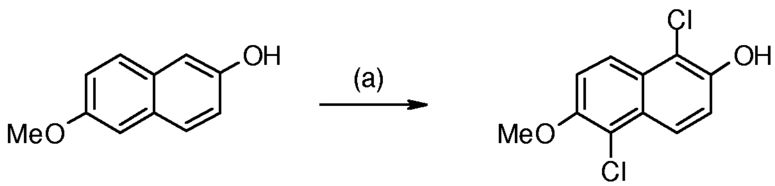


**12iii** **12ii**

Reaktionsbedingungen: (a) NCS, ACN, über Nacht bei RT

1-Chlor-7-methoxy-2-naphthol 14ii wurde in einem Schritt synthetisiert (neue Synthesemethode) :

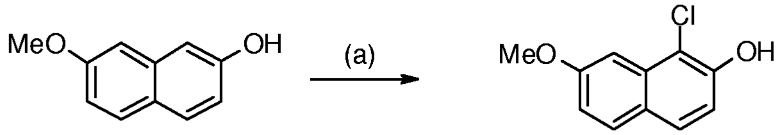


**14iii** **14ii**

Reaktionsbedingungen: (a) NCS, ACN, über Nacht bei RT

6-Cyano-2-naphthyltrifluormethansulfonat 8i, 1,5-Dichlor-6-methoxy-2-naphthyl-- trifluormethansulfonat 12i (WO 03/051805), 7-Methoxy-2-naphthyl-trifluormethansulfonat 13i (WO 03/051805) und 1-Chlor-7-methoxy-2-naphthyl-trifluormethansulfonat 14i:

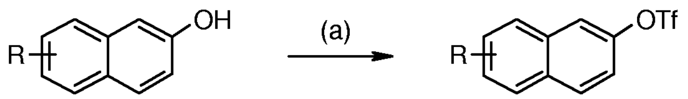


**8ii** R=6-CN
**12ii** R=1,5-Cl, 6-OMe
**13ii** R=7-OMe
**14ii** R=1-Cl, 7-OMe

**8i** R=6-CN
**12i** R=1,5-Cl, 6-OMe
**13i** R=7-OMe
**14i** R=1-Cl, 7-OMe

Reaktionsbedingungen: (a) Trifluormethansulfonsäureanhydrid, Pyridin, 30 min bei 0-5 °C, über Nacht bei RT

2-Bromchinolin 20i (Young, T.E. und Amstutz, E.D., JACS, 4773-5 (1951)) und 2-Bromchinoxalin 21i wurden durch Reaktion des entsprechenden Alkohols mit $POBr_3$ hergestellt:

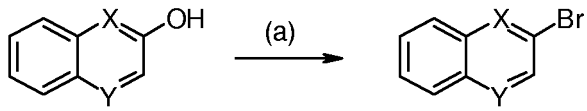


**20ii** X=N, Y=CH
**21ii** X=N, Y=N

**20i** X=N, Y=CH
**21i** X=N, Y=N

Reaktionsbedingungen: (a) $POBr_3$, 4h, 150 °C

*O*-Methoxynaphthylboronsäure 24i wurde nach einer bekannten Synthesemethode hergestellt (Chowdhury, S. et al., Tet. Lett. 40(43):7599-7603 (1999)):

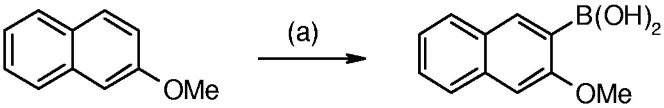


**24ii** **24i**

Reaktionsbedingungen: (a) 1) nBuLi, THF, -20°C 2) $B(OMe)_3$, THF, -78 °C

*N*-Benzylidenmethylamin 28i wurde wie beschrieben hergestellt (H.Dahn und P.Zoller, Helv.Chim.Acta 35:1348-1351 (1952)):

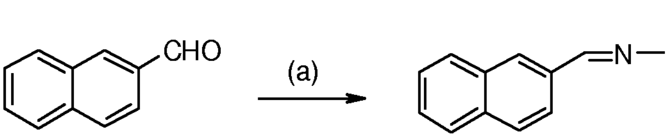


**29i** **28i**

Reaktionsbedingungen: (a) $MeNH_2$, EtOH, Reflux, 1 h.

B) Synthese der Verbindungen 1 -31 :

Allgemeine Synthese der Verbindungen 1-2.4-10,12-16,19-22,30-31 : Eine M-schung aus substituiertem 2-Bromnaphthalin oder 2-Trifluormethansulfonat (1 eq), heterozyklischer Boronsäure (1,3 eq), Natriumcarbonat (2,1 eq) und Tetrakis(triphenylphosphin)palladium (0,02 eq) in Ethylenglycoldimethylether oder Toluol wurde über Nacht bei 80 °C unter Stickstoffatmosphäre gerührt. Nach Abkühlung auf Raumtemperatur (RT) wurde Wasser zugegeben. Die Mischung wurde mit Ethylacetat extrahiert, über $MgSO_4$ getrocknet, gefiltert und das

Lösemittel *in vacuo* entfernt. Nach Reinigung über Säulenchromatographie betrugen die Ausbeuten bis zu 94%. Synthese von 6-Pyridin-3-yl-2-naphthol 3: Demethylierung von Verbindung 2 durch $BBr_3$ in Dichlormethan bei -78°C ergab die hydroxylierte Verbindung 3:

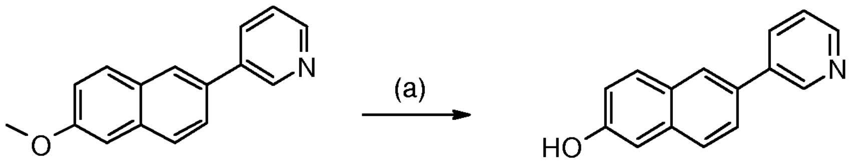


**2** **3**

Reaktionsbedingungen: (a) $BBr_3$, $CH_2Cl_2$, -78 °C

$BBr_3$ (0,85 ml, 0,85 mmol) wurde bei -78°C unter Stickstoffatmosphäre langsam zu Verbindung 2 (50 mg, 0,21 mmol) in 6 ml trockenem $CH_2Cl_2$ gegeben. Nach 30 min Rühren wurde die Kühlung beendet und die Mischung über Nacht bei RT gerührt. Die Reaktion wurde dann durch langsame Zugabe von Methanol beendet. Die Mischung wurde mit einer gesättigten Natriumbicarbonat-Lösung gewaschen, die organische Phase wurde über $MgSO_4$ getrocknet, abfiltriert und das Lösemittel *in vacuo* entfernt.

Synthese von 3-(5-Brom-6-methoxy-2-naphthyl)pyridin 1 0 und 3,3'-(2-Methoxy-naphthalin-1,6-diyl)dipyridin 1 1 : Die Suzuki-Kupplung von 10i mit zwei Äquivalenten (eq.) 3-Pyridylboronsäure (Katalysator: $PdP(Ph_3)_4$; Base: $Na_2CO_3$) ergab eine Mischung aus Mono(10)- und Di(11)pyridyl-substituiertem Naphthalin:

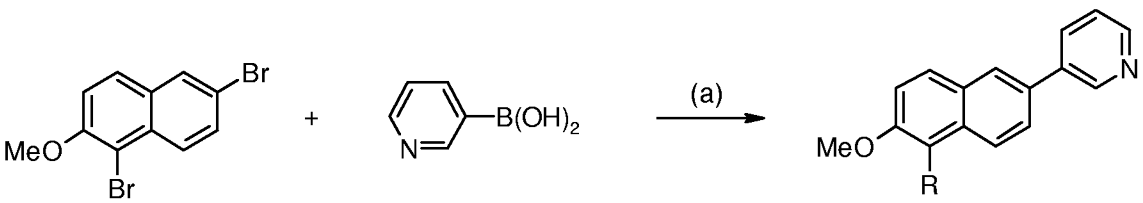


**10i**

**10** R=Br
**11** R=3-Pyr

Reaktionsbedingungen: (a) $PdP(Ph_3)_4$, $Na_2CO_3$, DME, 80 °C

Eine Mischung aus 1,6-Dibrom-2-methoxynaphthalin 10i (223 mg, 0,71 mmol), 3-pyridinboronsäure (260 mg, 2,12 mmol), Natriumcarbonat (299 mg, 2,82 mmol) und Tetrakis(triphenylphosphin)palladium (16 mg) in Ethylenglycoldimethylether wurde über Nacht bei 80 °C unter Stickstoffatmosphäre gerührt. Die Mischung wurde auf RT abgekühlt und Wasser zugegeben. Nach Extraktion mit Ethylacetat wurde die organische Phase über $MgSO_4$ getrocknet, filtriert und das Lösemittel *in vacuo* entfernt. Das Produkt wurde durch Säulenchromatographie gereinigt, als Eluent diente $CH_2Cl_2$/MeOH (98:2).

Synthese der Verbindungen 17, 18: Nach einer Literaturmethode (Jagdmann, G.E. et al., Synth.Comm. 20(8):1203-1208 (1990)) wurde der Carbonsäureester 16 mit den entsprechenden Formamiden und NaOMe als Base in wasserfreiem DMF in primäre oder sekundäre Amide umgewandelt:

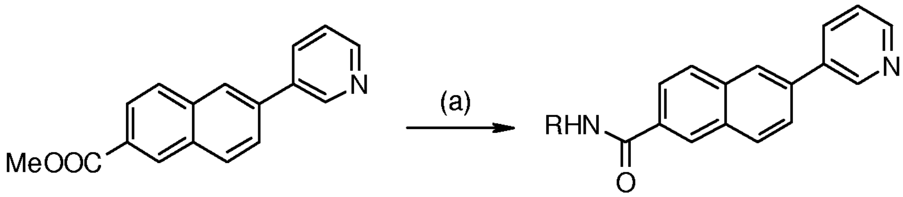


**16**

**17** R=H
**18** R=Me

Reaktionsbedingungen: (a) RNHCHO, NaOMe, DMF, 100 °C

Eine gerührte Mischung aus Methyl-6-brom-2-naphthoat 16 (1 eq) und Formamid (3,3 eq) unter Stickstoff wurde mit wasserfreiem DMF (2 ml) versetzt und auf 100°C erhitzt. Methanolisches Natriummethanolat (0,7 eq)wurde zugegeben und die Mischung weiter für 1 h gerührt. Nach Abkühlung und Wasserzugabe (2 ml) wurde die Mischung mit Ethylacetat extrahiert, die organische Phase über $MgSO_4$ getrocknet, filtriert und das Lösemittel *in vacuo* entfernt.

Synthese der Verbindungen 23-25: Die 1-Imidazolyl-substituierten Napthaline 23-25 wurden durch Kupplung der Naphthylboronsäure mit Imidazol in Anwesenheit einer katalytischen Menge eines Kupfersalzes hergestellt (Lam, P.Y.S. et al., Tet. Lett. 39:2941-4 (1998); Lan, J.-B. et al., Chem. Commun., 188-9 (2004)):

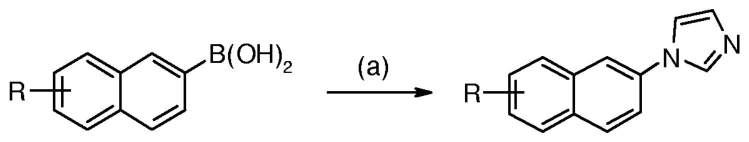


**23i-25i** **23-25**

Reaktionsbedingungen: (a) Imidazol, $Cu(OAc)_2$, Pyridin, $CH_2Cl_2$, RT; oder Imidazol, CuI, 2 h, Reflux

Eine Mischung aus Naphthalinboronsäure 23i oder 25i (2 eq.), Imidazol (1 eq.), Kupfer(II)acetat (1,5 eq.), Pyridin (2 eq.) und einem 4 Å-Molekularsieb in wasserfreiem Dichlormethan wurde zwei Tage bei RT gerührt. Die Mischung wurde abfiltriert und das Lösemittel *in vacuo* entfernt. Das Produkt wurde säulenchromatographisch gereinigt. Eine Mischung aus Naphthalinboronsäure 24i (1 eq.), Imidazol (1,2 eq.) und Kupferiodid (5 mol%) in wasserfreiem Methanol wurde unter Luftatmosphäre 2 h rückflussgekocht. Das Lösemittel wurde *in vacuo* entfernt. Das Produkt wurde säulenchromatographisch gereinigt.

Synthese der Verbindung 26: 3-(1*H*-Imidazol-1-yl)chinolin 26 wurde wie von Kauffmann et al. beschrieben hergestellt (Kauffmann, T. et al., Chem.Ber. 115:452-8 (1982)):

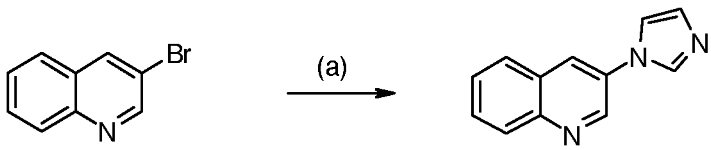


**19i** **26**

Reaktionsbedingungen: (a) Imidazol, CuO, $K_2CO_3$, Nitrobenzol, 24h, Reflux

Synthese der Verbindung 27: Behandlung des a-Bromketons 27i mit Formamid bei hoher Temperatur ergab 4(5)-(2-Naphthyl)-1*H*-imidazol 27 entsprechend einer Methode von Bredereck und Theilig (Bredereck, H. und Theilig, G., Chem.Ber. 86:88-96 (1953)):

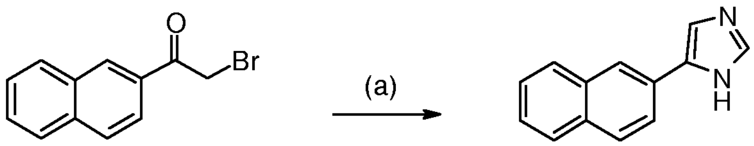


**27i** **27**

Reaktionsbedingungen: (a) $NH_2CHO$, 185 °C, 2 h

Synthese der Verbindungen 28, 29: Reaktion von *N*-Benzylidenmethylamin 28i oder 2-Naphthaldehyd 29i und Tosylmethylisocyanid (Tosmic) mit $K_2CO_3$ als Base ergab 1-Methyl-5-(2-naphthyl)-1*H*-imidazol 28 bzw. 5-(2-Naphthyl)-1,3-oxazol 29:

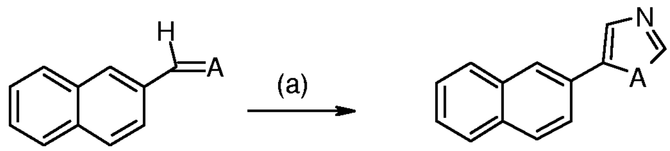


**28i** A=NMe
**29i** A=O

**28** A=NMe
**29** A=O

Reaktionsbedingungen: (a) Tosmic, $K_2CO_3$, RT

Eine Mischung aus *N*-Benzylidenmethylamin 28i oder 2-Naphthaldehyd 29i (1 eq.), Tosylmethylisocyanid (1,7 eq.) und Kaliumcarbonat (2 eq.) in absolutem Methanol wurde bei RT über Nacht gerührt. Methanol wurde *in vacuo* entfernt und Dichlo r-methan zum Rohprodukt zugegeben. Nach Waschen mit Wasser wurde die organische Phase über $MgSO_4$ getrocknet, filtriert und das Lösemittel *in vacuo* entfernt. Das Produkt wurde säulenchromatographisch gereinigt.

C) Reinigungsbedingungen, Ausbeute und Charakterisierung der Titelverbindungen:

3-(2-Naphthyl)pyridin (1). Reinigung: Säulenchromatographie (CC) ($CHCl_2$/MeOH, 97:3) Ausbeute 16% . $^1H$ NMR (500 MHz, $CDCl_3$) : $\delta$ 7,44 (dd, 1 H, $^3J$ = 8,2 Hz, $^4J$ = Hz, Pyr. H-5), 7,51 -7,56 (m, 2H, Ar H), 7,71 (dd, 1 H, $^3J$ = 8,5 Hz, $^4J$ = 1,6 Hz, Ar H), 7,88-7,90 (m, 2H, Ar H), 7,96 (d, 1 H, $^3J$ = 8,5 Hz, Ar H), 8,03-8,05 (m, 2H, Ar H, Pyr. H-4), 8,63 (dd, 1 H, $^3J$ = 4,7 Hz, $^4J$ = 1,6 Hz, Pyr. H-6), 8,99 (dd, 1 H, $^4J$ = 1,6 Hz, Pyr.H-2). IR $cm^{-1}$ : $v_{max}$ 3392, 3051, 3029, 1599, 1484. MS m/z 206 ($MH^+$), 178, 151, 77, 51.

3-(6-Methoxy-2-naphthyl)pyridin (2). Reinigung: CC ($CH_2Cl_2$/MeOH, 97:3) Ausbeute 77%. $^1H$ NMR (500 MHz, $CDCl_3$): $\delta$ 3,95 (s, 1 H, $OCH_3$), 7,17 (d, 1 H, $^4J$ = 2,5 Hz, Ar H), 7,22 (dd,1H, $^3J$ = 8,8 Hz, $^4J$ = 2,2 Hz, Ar H), 7,45-7,47 (m, 1 H, Pyr. H-5), 7,67 (dd, 1 H, $^3J$ = 8,5 Hz, $^4J$ = 1,8 Hz, Ar H), 7,81 (d, 1 H, $^3J$ = 8,5 Hz, Ar H), 7,85 (d, 1 H, $^3J$ = 8,5 Hz, Ar H), 7,98 (s, 1 H, Ar H), 8,04-8,06 (m, 1H, Pyr. H-4), 8,61 (dd, 1 H, $^3J$ = 4.7 Hz, $^4J$ = 1,3 Hz, Pyr. H-6), 8,97 (s, 1H, Pyr. H-2). IR $cm^{-1}$: $v_{max}$ 3058, 2938, 1605, 1489. MS *m/z* 236 ($MH^+$).

6-Pyridin-3-yl-2-naphthol (3). Reinigung: CC ($CH_2Cl_2$/MeOH, 98:2) Ausbeute 65%. $^1H$ NMR (500 MHz, $CDCl_3$): $\delta$ 7,20 (dd, 1 H, $^3J$ = 8,8 Hz, $^4J$ = 2,2 Hz, Ar H), 7,23 (d, 1 H, $^4J$ = 1,9 Hz, Ar H), 7,62 (m, 1 H, Pyr. H-5), 7,83 (dd, 1 H, $^3J$ = 8,5 Hz, $^4J$ = 1,6 Hz, Ar H), 7,87 (d, 1 H, $^3J$ = 8,8 Hz, Ar H), 7,92 (d, 1 H, $^3J$ = 8,8 Hz, Ar H), 8,24 (s, 1 H, Ar H), 8,29 (dt, 1H, $^3J$ = 7,9 Hz, $^4J$ = 1 ,9 Hz, Pyr. H-4), 8,65 (d, 1 H, $^3J$ = 4,7 Hz, Pyr. H-6), 9,08 (d, 1H, $^4J$ = 1,6 Hz, Pyr. H-2), 9,95 (s, 1 H, OH). IR $cm^{-1}$ : $v_{max}$ 3634, 3021, 1594, 1509, 1489, 793. MS *m/z* 222 ($MH^+$).

3-(6-Bromo-2-naphthyl)pyridin (4). Reinigung: CC ($CH_2Cl_2$/MeOH, 98:2) Ausbeute 21%. $^1H$ NMR (500 MHz, $CDCl_3$): $\delta$ 7,53 (m, 1 H, Pyr. H-5), 7,62 (dd, 1 H, $^3J$ = 8,5 Hz, $^4J$ = 1,6 Hz, Ar H), 7,73 (dd, 1 H, $^3J$ = 8,5 Hz, $^4J$ = 1,6 Hz, Ar H), 7,79 (d, 1 H, $^3J$ = 8,5 Hz, Ar H), 7,89 (d, 1 H, $^3J$ = 8,5 Hz, Ar H), 8,02 (d, 1 H, $^4J$ = 1,5 Hz, Ar H), 8,06 (d, 1 H, $^4J$ = 1,5 Hz, Ar H), 8,11 (dt, 1 H, $^3J$ = 7,9 Hz, $^4J$ = 1,6 Hz, Pyr. H-4), 8,65 (dd, 1H, $^3J$ = 5,0 Hz, $^4J$ = 1,6 Hz, Pyr. H-6), 8,99 (d, 1 H, $^4J$ = 1,6 Hz, Pyr. H-2). IR $cm^{-1}$ : $v_{max}$ 3032, 2963, 1482. MS *m/z* 286 ($M+2H^+$) 284 ($MH^+$).

3-(6-Ethoxy-2-naphthyl)pyridin (5). Reinigung: CC ($CH_2Cl_2$/MeOH, 98:2) Ausbeute 4% . $^1H$ NMR (500 MHz, $CDCl_3$) : $\delta$ 1,51 (t, 3H, $^3J$ = 6,9 Hz, $CH_3$), 4,18 (q, 2H, $^3J$ = 6,9 Hz, $CH_2$), 7,17 (d, 1 H, $^4J$ = 2,5 Hz, Ar H), 7,21 (dd, 1 H, $^3J$ = 8,8 Hz, $^4J$ = 2,5 Hz, Ar H), 7,55 (m, 1 H, Pyr. H-5), 7,66 (dd, 1 H, $^3J$ = 8,5 Hz, $^4J$ = 1,9 Hz, Ar H), 7,82 (d, 1H, $^3J$ = 8,8 Hz, Ar H), 7,85 (d, 1H, $^3J$ = 8,8 Hz, Ar H), 7,98 (d, 1H, $^4J$ = 1,9 Hz, Ar H), 8,16 (dt, 1 H, $^3J$ = 7,9 Hz, $^4J$ = 1,6 Hz, Pyr. H-4), 8,61 (dd, 1H, $^3J$ = 5,0 Hz, $^4J$ = 1,6 Hz, Pyr. H-6), 8,99 (d, 1H, $^4J$ = 2,5 Hz, Pyr. H-2). IR $cm^{-1}$ : $v_{max}$ 3021, 2995, 1601, 1489, 1252, 821. MS m/z 250 ($MH^+$), 221.

3-(6-Propoxy-2-naphthyl)pyridin (6). Reinigung: CC ($CH_2Cl_2$/MeOH, 98:2) Ausbeute 84% . $^1H$ NMR (500 MHz, $CDCl_3$): $\delta$ 1,10 (t, 3H, $^3J$ = 7,2 Hz, $CH_3$), 1,89 (q, 2H, $^3J$ = 6,6 Hz, $CH_2$), 4,07 (t, 2H, $^3J$ = 6,6 Hz, $CH_2$), 7,17 (d, 1H, $^4J$ = 2,5 Hz, Ar H), 7,21 (dd, 1 H, $^3J$ = 8,8 Hz, $^4J$ = 2,5 Hz, Ar H), 7,39 (m, 1 H, Pyr. H-5), 7,67 (dd, 1 H, $^3J$ = 8,5 Hz, $^4J$ = 1,9 Hz, Ar H), 7,82 (d, 1 H, $^3J$ = 8,5 Hz, Ar H), 7,83 (d, 1 H, $^3J$ = 8,8 Hz, Ar H), 7,97 (d, 1 H, $^4J$ = 1,9 Hz, Ar H), 7,98 (dt, 1H, $^3J$ = 7,9 Hz, $^4J$ = 1,6 Hz, Pyr. H-4), 8,60 (dd, 1 H, $^3J$ = 4,7 Hz, $^4J$ = 1,6 Hz, Pyr. H-6), 8,96 (d, 1 H, $^4J$ = 2,5 Hz, Pyr. H-2). IR $cm^{-1}$ : $v_{max}$ 2967, 2934, 2878, 1629, 1604, 1491 , 1389, 1254. MS *m/z* 264 ($MH^+$).

3-(6-Benzyloxy-2-naphthyl)pyridin (7). Reinigung: CC ($CH_2Cl_2$/MeOH, 97:3) Ausbeute 76%. $^1H$ NMR (500 MHz, $CDCl_3$): $\delta$ 5,22 (s, 2H, $CH_2$), 7,26 (d, 1 H, $^4J$ = 2,5 Hz, Ar H), 7,30 (dd, 1H, $^3J$ = 8. Hz, $^4J$ = 2,5 Hz, Ar H), 7,36 (t, 1H, $^3J$ = 7,2 Hz, Ar H), 7,42 (t, 2H, $^3J$ = 7,6 Hz, Ar H), 7,48-7,51 (m, 1 H, Pyr. H-5, Ar H), 7,67 (dd, 1H, $^3J$ = 8,5 Hz, $^4J$ = 1,9 Hz, Ar H), 7,84 (d, 1H, $^3J$ = 8,2 Hz, Ar H), 7,85 (d, 1H, $^3J$ = 8,5 Hz, Ar H), 7,99 (s, 1 H, Ar H),

8,10 (dt, 1H, $^3J$ = 7,8 Hz, $^4J$ = 1,6 Hz, Pyr. H-4), 8,62 (dd, 1 H, $^3J$ = 5,0 Hz, $^4J$ = 1,3 Hz, Pyr. H-6), 8,98 (d, 1 H, $^4J$ = 2,2 Hz, Pyr. H-2). IR cm$^{-1}$: $\nu_{max}$3040, 1604, 1490. MS *m/z* 312 (MH$^+$), 221.

6-Pyridin-3-yl-2-naphthonitril (8). Reaktionsdauer nur 2,5 h. Reinigung: CC ($CH_2Cl_2$/MeOH, 98:2) Ausbeute 71%. $^1$H NMR (500 MHz, $CDCl_3$): $\delta$ 7,55 (m, 1H, Pyr. H-5), 7,68 (dd, 1 H, $^3J$ = 8,5 Hz, $^4J$ = 1,5 Hz, Ar H), 7,84 (dd, 1 H, $^3J$ = 8,5 Hz, $^4J$ = 1,8 Hz, Ar H), 8,01 (d, 1 H, $^3J$ = 8,5 Hz, Ar H), 8,04 (d, 1 H, $^3J$ = 8,5 Hz, Ar H), 8,10 (d, 1 H, $^4J$ = 1,2 Hz, Ar H), 8,12 (dt, 1 H, $^3J$ = 7,9 Hz, $^4J$ = 1,5 Hz, Pyr. H-4), 8,28 (s, 1 H, Ar H), 8,70 (dd, 1 H, $^3J$ = 4,9 Hz, $^4J$ = 1,5 Hz, Pyr. H-6), 9,01 (d, 1 H, $^4J$ = 2,4 Hz, Pyr. H-2). IR cm$^{-1}$ : $\nu_{max}$ 3032, 2224 (CN), 1629, 1469, 1424. MS *m/z* 231 (MH$^+$).

3-(5-Chlor-6-methoxy-2-naphthyl)pyridin (9). Reinigung: CC ($CH_2Cl_2$/MeOH, 97:3) Ausbeute 93%. $^1$H NMR (500 MHz, $CDCl_3$): $\delta$ 4,07 (s, 3H, $OCH_3$), 7,39 (d, 1H, $^3J$= 9,1 Hz, Ar H), 7,64 (m, 1 H, Pyr. H-5), 7,79 (dd, 1 H, $^3J$ = 9,1 Hz, $^4J$ = 2,2 Hz, Ar H), 7,89 (d, 1 H, $^3J$ = 8,8 Hz, Ar H), 8,04 (d, 1 H, $^4J$ = 1,9 Hz, Ar H), 8,26 (dt, 1 H, $^3J$ = 8,1 Hz, $^4J$ = 1,6 Hz, Pyr. H-4), 8,36 (d, 1 H, $^3J$ = 8,8 Hz, Ar H), 8,65 (dd, 1 H, $^3J$ = 5,0 Hz, $^4J$ = 1,6 Hz, Pyr. H-6), 9,02 (d, 1H, $^4J$ = 2,2 Hz, Pyr. H-2). IR cm$^{-1}$ : $\nu_{max}$ 3033, 2955, 2844, 1602, 1490, 1275. MS *m/z* 272 (M+2H$^+$), 270 (MH$^+$), 255, 227, 192.

3-(5-Brom-6-methoxy-2-naphthyl)pyridin (10). Reinigung: CC ($CH_2Cl_2$/MeOH, 97:3) Ausbeute 66% . $^1$H NMR (500 MHz, $CDCl_3$) : $\delta$ 4,07 (s, 3H, $OCH_3$), 7,36 (d, 1 H, $^3J$ = 8,8 Hz, Ar H), 7,61 (m, 1 H, Pyr. H-5), 7,79 (dd, 1 H, $^3J$ = 9,1 Hz, $^4J$ = 2,2 Hz, Ar H), 7,92 (d, 1 H, $^3J$ = 8,8 Hz, Ar H), 8,02 (d, 1 H, $^4J$ = 1,9 Hz, Ar H), 8,22 (dt, 1 H, $^3J$ = 7,9 Hz, $^4J$ = 2,2 Hz, Pyr. H-4), 8,36 (d, 1 H, $^3J$ = 8,8 Hz, Ar H), 8,65 (dd, 1 H, $^3J$ = 5,0 Hz, $^4J$ = 1,6 Hz, Pyr. H-6), 9,01 (d, 1H, $^4J$ = 2,2 Hz, Pyr. H-2). IR cm$^{-1}$ : $\nu_{max}$ 3045, 2955, 2832, 1601, 1488, 1272. MS *m/z* 317, 316, 315, 314, 270, 227, 191, 163.

3,3'-(2-Methoxynaphthalin-1,6-diyl)dipyridin (11). Reinigung: CC ($CH_2Cl_2$/MeOH, 97:3) Ausbeute 5% . $^1$H NMR (500 MHz, $CDCl_3$) : $\delta$ 3,89 (s, 3H, $OCH_3$), 7,43-7,46 (m, 2H, Ar H, Pyr. H-5), 7,54-7,56 (m, 2H, Ar H, Pyr. H-5), 7,62 (dd, 1H, $^3J$ = 8,8 Hz, $^4J$ = 1,9 Hz, Ar H), 7,86 (dt, 1 H, $^3J$ = 7,9 Hz, $^4J$ = 1,9 Hz, Pyr. H-4), 8,01 -8,04 (m, 2H, Ar H, Pyr.H-4), 8,07 (d, 1 H, $^4J$ = 1,6 Hz, Ar H), 8,62 (d, 1 H, $^3J$ = 5,0 Hz, Pyr. H-6), 8,68 (s, 1 H, Pyr. H-2), 8,70 (d, 1 H, $^3J$ = 5,0 Hz, Pyr. H-6), 8,97 (s, 1 H, Pyr. H-2). IR cm$^{-1}$ : $\nu_{max}$ 3031, 2950, 2842, 1599, 1488, 1258. MS *m/z* 313 (MH$^+$),

3-(1,5-Dichlor-6-methoxy-2-naphthyl)pyridin (12). Reinigung: CC ($CH_2Cl_2$/MeOH, 98:2) Ausbeute 41% . $^1$H NMR (500 MHz, $CDCl_3$) : $\delta$ 4,10 (s, 3H, $OCH_3$), 7,48 (d, 1 H, $^3J$ = 9,1 Hz, Ar H), 7,50 (d, 1H, $^3J$ = 8,8 Hz, Ar H), 7,70 (m, 1H, Pyr. H-5), 8,18 (dt, 1 H, $^3J$ = 7,9 Hz, $^4J$ = 1,6 Hz, Pyr. H-4), 8,30 (d, 1 H, $^3J$ = 8,8 Hz, Ar H), 8,37 (d, 1 H, $^3J$ = 9,1 Hz, Ar H), 8,73 (dd, 1 H, $^3J$ = 5,3 Hz, $^4J$ = 1,6 Hz, Pyr. H-6), 8,85 (d, 1 H, $^4J$ = 1,6 Hz, Pyr. H-2). IR cm$^{-1}$ : $\nu_{max}$ 3060, 2940, 2830, 1617, 1487. MS *m/z* 307, 306, 305, 304, 270, 227, 191 , 163.

3-(7-Methoxy-2-naphthyl)pyridin (13). Reaktionszeit: 8 h. Reinigung: CC ($CH_2Cl_2$/MeOH, 97:3) Ausbeute 31%. $^1$H NMR (500 MHz, $CDCl_3$): $\delta$ 3,95 (s, 3H, $OCH_3$), 7,18-7,21 (m, 2H, Ar H), 7,48 (m, 1H, Pyr. H-5), 7,54 (dd, 1H, $^3J$ = 8,2 Hz, $^4J$ = 1,6 Hz, Ar H), 7,78 (d, 1 H, $^3J$ = 8,5 Hz, Ar H), 7,88 (d, 1 H, $^3J$ = 8,2 Hz, Ar H), 7,95 (d, 1 H, $^4J$ = 1,8 Hz, Ar H), 8,08 (dt, 1 H, $^3J$ = 7,8 Hz, $^4J$ = 1,6 Hz, Pyr. H-4), 8,63 (dd, 1 H, $^3J$ = 5,0 Hz, $^4J$ = 1,6 Hz, Pyr. H-6), 8,99 (d, 1 H, $^4J$ = 2,5 Hz, Pyr. H-2). IR cm$^{-1}$ : $\nu_{max}$ 3030, 2973, 2835, 1626. MS *m/z* 236 (MH$^+$), 221.

3-(1-Chlor-7-methoxy-2-naphthyl)pyridin (14). Reinigung: CC (Hexan/EtOAc, 8:2) Ausbeute 18%. $^1$H NMR (500 MHz, $CDCl_3$): $\delta$ 4,00 (s, 3H, $OCH_3$), 7,26-7,29 (m, 2H, Ar H), 7,59 (m, 1 H, Pyr. H-5), 7,64 (d, 1 H, $^4J$ = 2,5 Hz, Ar H), 7,80 (d, 1 H, $^3J$ = 8,8 Hz, Ar H), 7,82 (d, 1 H, $^3J$ = 8,8 Hz, Ar H), 8,06 (dt, 1 H, $^3J$ = 7,9 Hz, $^4J$ = 1,6 Hz, Pyr. H-4), 8,70 (d, 1 H, $^3J$ = 5,0 Hz, Pyr. H-6), 8,82 (s, 1H, Pyr. H-2). IR cm$^{-1}$: $\nu_{max}$ 3020, 2932, 2878, 1677, 1506, 1225. MS *m/z* 272 (M+2H$^+$), 270 (MH$^+$), 255, 191. 3-(3-Methoxy-2-naphthyl)pyridin (1 5). Reinigung:CC ($CH_2Cl_2$/MeOH, 98:2) Ausbeute 31%. $^1$H NMR (500 MHz, $CDCl_3$): $\delta$ 3,94 (s, 3H, $OCH_3$), 7,25 (s, 1 H, Ar H), 7,35-7,40 (m, 2H, Ar H, Pyr. H-5), 7,48 (td, 1 H, $^3J$ = 8,2 Hz, $^4J$ = 1,3 Hz, Ar H), 7,77 (s, 1 H, Ar H), 7,78 (d, 1 H, $^3J$ = 8,2 Hz, Ar H), 7,81 (d, 1 H, $^3J$ = 8,2 Hz, Ar H), 7,93 (dt, 1 H, $^3J$ = 7,9 Hz, $^4J$ = 1,9 Hz, Pyr. H-4), 8,60 (dd, 1 H, $^3J$ = 4,9 Hz, $^4J$ = 1,8 Hz, Pyr. H-6), 8,85 (s, 1 H, Pyr. H-2). IR cm$^{-1}$: $\nu_{max}$. 3054, 2962, 2832, 1631, 1599, 1504, 1466, 1410, 1254. MS *m/z* 236 (MH$^+$).

Methyl-6-pyridin-3-yl-2-naphthoat (16). Reinigung: CC ($CH_2Cl_2$/MeOH, 98:2) Ausbeute 10%. $^1$H NMR (500 MHz, $CDCl_3$): $\delta$ 3,93 (s, 3H, $OCH_3$), 7,35-7,37 (m, 1 H, Pyr. H-5), 7,71 (dd, 1 H, $^3J$ = 8,5 Hz, $^4J$ = 1,9 Hz, Ar H), 7,89 (d, 1 H, $^3J$ = 8,5 Hz, Ar H), 7,94 (dt, 1 H, $^3J$ = 7,9 Hz, $^4J$ = 1,6 Hz, Pyr. H-4), 8,00 (d, 1 H, $^3J$ = 8,5 Hz, Ar H), 8,01 (s, 1 H, Ar H), 8,05 (dd, 1 H, $^3J$ = 8,5 Hz, $^4J$ = 1,6 Hz, Ar H), 8,58 (s, 1 H, Ar H), 8,59 (dd, 1 H, $^3J$ = 5,0 Hz, $^4J$ = 1,6 Hz, Pyr. H-6), 8,92 (d, 1 H, $^4J$ = 2,2 Hz, Pyr. H-2). IR cm$^{-1}$: $\nu_{max}$. 3052, 2952, 1718 (CO), 1598, 1499, 1292. MS *m/z* 264 (MH$^+$).

6-Pyridin-3-yl-2-naphthamid (17). Reinigung: CC (Hexan/Ethylacetat, 84:15) Ausbeute 61%. $^1$H NMR (500 MHz, $CDCl_3$): $\delta$ 7,42-7,45 (m, 1H, Pyr. H-5), 7,75 (dd, 1H, $^3J$ = 8,5 Hz, $^4J$ = 1,9 Hz, Ar H), 7,90 (dd, 1H, $^3J$ = 8,5 Hz, $^4J$ = 1,9 Hz, Ar H), 7,96 (d, 1H, $^3J$ = 8,8 Hz, Ar H), 8,01-8,04 (m, 2H, Ar H, Pyr. H-4), 8,06 (d, 1 H, $^4J$ = 1,3 Hz, Ar H), 8,38 (d, 1 H, $^4J$ = 1,3 Hz, Ar H), 8,59 (dd, 1 H, $^3J$ = 4,7 Hz, $^4J$ = 1,6 Hz, Pyr. H-6), 8,92 (d, 1H, $^4J$= 1,9 Hz, Pyr. H-2). IR cm$^{-1}$: $\nu_{max}$. MS *m/z* 249 (MH$^+$). *N*-Methyl-6-pyridin-3-yl-2-naphthamide (1 8). N-Methylformamid wurde verwendet. Reinigung: CC ($CH_2Cl_2$/MeOH, 95:5) Ausbeute 83%. $^1$H NMR (500 MHz, $CDCl_3$): $\delta$ 3,10 (s,

3H, $OCH_3$), 6,33 (s, 1 H, NH), 7,42-7,44 (m, 1H, Pyr. H-5), 7,77 (dd, 1 H, $^3J$ = 8,5 Hz, $^4J$ = 1,8 Hz, Ar H), 7,87 (dd, 1 H, $^3J$ = 8,5 Hz, $^4J$ = 1,5 Hz, Ar H), 7,96-8,07 (m, 4H, 3xAr H, Pyr. H-4), 8,33 (s, 1 H, Ar H), 8,65 (dd, 1 H, $^3J$ = 4,6 Hz, $^4J$ = 1,5 Hz, Pyr. H-6), 8,98 (d, 1 H, $^4J$ = 1,5 Hz, Pyr. H-2). IR $cm^{-1}$ : $\nu_{max}$ 3316, 3059, 2929, 1644, 1549, 1313. MS *m*/*z* 263 ($MH^+$).

3-Pyridin-3-ylchinolin (19). Reinigung: CC ($CH_2Cl_2$/MeOH, 97:3) Ausbeute 3%. $^1H$ NMR (500 MHz, $CDCl_3$): $\delta$ 7,46 (m, 1 H, Pyr. H-5), 7,62 (td, 1 H, $^3J$ = 8,2 Hz, $^4J$ = 1,3 Hz, Ar H), 7,77 (td, 1 H, $^3J$ = 8,5 Hz, $^4J$ = 1,6 Hz, Ar H), 7,91 (d, 1 H, $^3J$ = 7,6 Hz, Ar H), 8,02 (dt, 1H, $^3J$ = 7,9 Hz, $^4J$ = 2,2 Hz, Pyr. H-4), 8,16 (d, 1H, $^3J$ = 8,2 Hz, Ar H), 8,33 (d, 1H, $^4J$ = 2,2 Hz, Ar H), 8,69 (dd, 1H, $^3J$ = 4,7 Hz, $^4J$ = 1,3 Hz, Pyr. H-6), 8,98 (d, 1H, $^4J$ = 2,5 Hz, Pyr. H-2), 9,16 (d, 1H, $^4J$ = 2,2 Hz, Ar H). IR $cm^{-1}$ : $\nu_{max}$. 3055, 2930, 1494. MS *m*/*z* 207 ($MH^+$).

2-Pyridin-3-ylchinolin (20). Reinigung: CC ($CH_2Cl_2$/MeOH, 99:1). Das Produkt wurde in 4 ml Diethylether aufgenommen und mit einem Äquivalent HCl inDdiethylether (1 M) versetzt. Das Präzipitat wurde abfitriert und gründlich mit Diethylether gewaschen. Ausbeute 43%. $^1H$ NMR (500 MHz, $CDCl_3$): $\delta$ 7,68 (td, 1 H, $^3J$ = 7,9 Hz, $^4J$ = 0,9 Hz, Ar H), 7,86 (td, 1H, $^3J$ = 8,5 Hz, $^4J$ = 1,6 Hz, Ar H), 7,95 (dd, 1 H, $^3J$ = 8,2 Hz, $^4J$ = 0,9 Hz, Ar H), 8,12 (m, 1 H, Pyr. H-5), 8,17 (d, 1 H, $^3J$ = 8,5 Hz, Ar H), 8,34 (d, 1H, $^3J$ = 8,5 Hz, Ar H), 8,57 (d, 1H, $^3J$ = 8,5 Hz, Ar H), 8,87 (d, 1 H, $^3J$ = 5,4 Hz, Pyr. H-4), 9,34 (d, 1H, $^3J$ = 8,2 Hz, Pyr. H-6), 9,67 (s, 1H, Pyr. H-2). IR $cm^{-1}$ : $\nu_{max}$ 3053, 2931, 1596, 1548, 1506. MS *m*/*z* 207 ($MH^+$).

2-Pyridin-3-ylchinoxalin (21). Reinigung: CC ($CH_2Cl_2$/MeOH, 99:1). Das Produkt wurde in 4 ml Diethylether aufgenommen und mit einem Äquivalent HCl inddiethylether (1 M) versetzt. Das Präzipitat wurde abfitriert und gründlich mit Diethylether gewaschen. Ausbeute 44%. $^1H$ NMR (500 MHz, $CDCl_3$): $\delta$ 7,79-7,82 (m, 2H, Ar H), 8,07-8,12 (m, 2H, Ar H), 8,14-8,17 (m,1H, Pyr. H-5), 8,92 (d, 1 H, $^3J$ = 5,0 Hz, Pyr. H-4), 9,33 (d, 1H, $^3J$ = 7,9 Hz, Pyr. H-6), 9,46 (s, 1 H, Pyr. H-2), 9,71 (s, 1 H, Ar H). IR $cm^{-1}$ : $\nu_{max}$ 3066, 1604, 1547, 1499, 1313. MS *m*/*z* 208 ($MH^+$), 181, 102, 75, 51 . 3-(9-Phenanthryl)pyridin (22). Reinigung: CC ($CH_2Cl_2$/MeOH, 98:2) Ausbeute 94%. $^1H$ NMR (500 MHz, $CDCl_3$): $\delta$ 7,56-7,61 (m, 2H, Ar H, Pyr. H-5), 7,66 (td, 1H, $^3J$ = 8,2 Hz, $^4J$ = 1,3 Hz, Ar H), 7,70-7,74 (m, 3H, Ar H), 7,77 (dd, 1 H, $^3J$ = 8,2 Hz, $^4J$ = 0,9 Hz, Ar H), 7,92 (dd, 1 H, $^3J$ = 7,9 Hz, $^4J$ = 1,6 Hz, Ar H), 8,03 (dt, 1H, $^3J$ = 7,9 Hz, $^4J$ = 1,9 Hz, Pyr. H-4), 8,73-8,76 (m, 2H, Ar H, Pyr. H-6), 8,81 (d, 1H, $^3J$ = 8,5 Hz, Ar H), 8,85 (d, 1 H, $^4J$ = 2,2 Hz, Pyr. H-2). IR $cm^{-1}$: $\nu_{max}$ 3055, 1659, 1535, 1456, 1247. MS *m*/*z* 256 ($MH^+$).

1-(2-Naphthyl)-1*H*-imidazol (23). Reinigung: CC ($CH_2Cl_2$/MeOH, 95:5) Ausbeute 34%. $^1H$ NMR (500 MHz, $CDCl_3$) : $\delta$ 7,26 (s, 1H, Im. H-4), 7,40 (s, 1 H, Im. H-5), 7,51-7,58 (m, 3H, Ar H), 7,82 (d, 1H$^4J$ = 1,5 Hz, Ar H), 7,88 (t, 2H, $^3J$ = 7,6 Hz, Ar H), 7,96 (d, 1 H, $^3J$ = 7,6 Hz, Ar H), 8,05 (s, 1H, Im. H-2). IR $cm^{-1}$: $\nu_{max}$ 3116, 3058, 1688, 1602, 1493. MS *m*/*z* 195 ($MH^+$), 167, 139, 115, 77, 51.

1-(3-Methoxy-2-naphthyl)-1*H*-imidazol (24). Reinigung: CC ($CH_2Cl_2$/MeOH, 97:3) Ausbeute 13%. $^1H$ NMR (500 MHz, $CDCl_3$): $\delta$ 3,97 (s, 3H, $OCH_3$), 7,21 (s, 1 H, Im. H-4), 7,30 (s, 2H, Ar H, Im. H-5), 7,24 (td, 1H, $^3J$ = 8,2 Hz, $^4J$ = 1,3 Hz, Ar H), 7,51 (td, 1 H, $^3J$ = 8,2 Hz, $^4J$ = 1,3 Hz, Ar H), 7,74 (s, 1 H, Ar H), 7,79 (d, 2H, $^3J$ = 8,5 Hz, Ar H), 7,87 (s, 1 H, Im. H-2). IR $cm^{-1}$: $\nu_{max}$ 3059, 2940, 2839, 1506. MS *m*/*z* 225 ($MH^+$).

1-(6-Methoxy-2-naphthyl)-1*H*-imidazol (25). Reinigung: CC ($CH_2Cl_2$/MeOH, 97:3) Ausbeute 13%. $^1H$ NMR(500 MHz, $CDCl_3$): $\delta$ 3,95 (s, 3H, $OCH_3$), 7,18 (d, 1H, $^4J$= 2,5 Hz, Ar H), 7,24 (dd, 1 H, $^3J$ = 8,5 Hz, $^4J$ = 2,5 Hz, Ar H), 7,28 (s, 1 H, Im. H-4), 7,38 (s, 1 H, Im. H-5), 7,48 (dd, 1 H, $^3J$ = 8,5 Hz, $^4J$ = 2,5 Hz, Ar H), 7,76 (s, 1 H, Ar H), 7,77 (d, 1 H, $^3J$ = 8,5 Hz, Ar H), 7,85 (d, 1 H, $^3J$ = 8,5 Hz, Ar H), 8,07 (s, 1 H, Im. H-2). IR $cm^{-1}$: $\nu_{max}$ 3113, 3003, 2962, 2842, 1607. MS *m*/*z* 225 ($MH^+$), 210, 126.

3-(1*H*-Imidazol-1-yl)chinolin (26). Reinigung: CC ($CH_2Cl_2$/MeOH, 97:3) Ausbeute 18% . $^1H$ NMR (500 MHz, $CDCl_3$) : $\delta$ 7,34 (t, 1H, $^4J$ = 1,3 Hz, Im. H-4), 7,44 (t, 1 H, $^4J$ = 1,5 Hz, Im. H-5), 7,66 (td, 1 H, $^3J$ = 8,2 Hz, $^4J$ = 1,3 Hz, Ar H), 7,79 (td, 1 H, $^3J$ = 8,2 Hz, $^4J$ = 1,5 Hz, Ar H), 7,90 (dd, 1H, $^3J$ = 8,2 Hz, $^4J$ = 1,6 Hz, Ar H), 8,17 (s, 1H, Im. H-2), 8,18-8,19 (m, 2H, Ar H), 9,04 (d, 1H, $^4J$ = 1,8 Hz, Ar H). IR $cm^{-1}$: $\nu_{max}$ 3102, 2962, 1608, 1497. MS *m*/*z* 196 ($MH^+$),169, 77, 51.

4(5)-(2-Naphthyl)-1 *H*-imidazol (27). Reinigung: CC ($CH_2Cl_2$) Ausbeute 9%. $^1H$ NMR (500 MHz, $CDCl_3$): $\delta$ 7,38 (s, 1 H, Im. H-4), 7,39-7,45 (m, 2H, Ar H), 7,73 (dd, 1H, $^3J$ = 8,5 Hz, $^4J$ = 1,9 Hz, Ar H), 7,74-7,81 (m, 4H, Ar H + Im. H-2), 8,13 (s, 1H, Ar H). IR $cm^{-1}$: $\nu_{max}$ 3125, 3044, 2852. MS *m*/*z* 195 ($MH^+$), 168, 141. 1-Methyl-5-(2-naphthyl)-1*H*-imidazol (28). Reinigung: CC ($CH_2Cl_2$/MeOH, 97:3) Ausbeute 18%. $^1H$ NMR (500 MHz, $CDCl_3$): $\delta$ 3,75 (s, 3H, $CH_3$), 7,22 (s, 1H, Im. H-4), 7,49-7,53 (m, 3H, Ar H), 7,68 (s, 1H, Im. H-2), 7,85-7,87 (m, 3H, Ar H), 7,91 (d, 1H, $^3J$ = 8,5 Hz, Ar H). IR $cm^{-1}$: $\nu_{max}$ 3083, 3053, 2952, 1600, 1490. MS *m*/*z* 209 ($MH^+$), 167, 139, 115.

5-(2-Naphthyl)-1,3-oxazol (29). Reinigung: CC ($CH_2Cl_2$/MeOH, 97:3). Ausbeute 28% . $^1H$ NMR (500 MHz, $CDCl_3$): $\delta$ 7,47 (s, 1 H, Im. H-4), 7,49-7,54 (m, 2H, Ar H), 7,73 (dd, 1 H, $^3J$ = 8,5 Hz, $^4J$ = 1,6 Hz, Ar H), 7,83-7,90 (m, 3H, Ar H), 7,97 (s, 1 H, Im. H-2), 8,14 (s, 1H, Ar H). IR $cm^{-1}$: $\nu_{max}$ 3128, 3055, 2952, 1630, 1497. MS *m*/*z* 196 ($MH^+$), 167, 139, 115.

5-(6-Methoxy-2-naphthyl)pyrimidin (30). Reinigung: CC ($CH_2Cl_2$/MeOH, 97:3) Ausbeute 24%. $^1H$ NMR (500 MHz, $CDCl_3$) : $\delta$ 3,96 (s, 3H, $OCH_3$), 7,19 (d, 1H, $^4J$ = 2,5 Hz, Ar H), 7,24 (dd, 1 H, $^3J$ = 8,8 Hz, $^4J$ = 2,5 Hz, Ar H), 7,67 (dd, 1 H, $^3J$ = 8,5 Hz, $^4J$ = 1,9 Hz, Ar H), 7,84 (d, 1 H, $^3J$ = 8,5 Hz, Ar H), 7,90 (d, 1 H, $^3J$ = 8,5 Hz, Ar

H), 8,02 (d, 1 H, $^4J$ = 1,9 Hz, Ar H), 9,15 (s, 2H, Pyr. H-4, Pyr. H-6), 9,26 (s, 1H, Pyr. H-2). IR $cm^{-1}$ : $\nu_{max}$ 3034, 2940, 1694, 1626, 1606, 1487, 1210. MS *m/z* 237 ($MH^+$). 4-(6-Methoxy-2-naphthyl)pyridin (31). Reinigung: CC ($CH_2Cl_2$/MeOH, 97:3) Ausbeute 60%. $^1H$ NMR (500 MHz, $CDCl_3$) : $\delta$ 3,97 (s, 3H, $OCH_3$), 7,19 (d, 1H, $^4J$ = 2,5 Hz, Ar H), 7,25 (dd, 1 H, $^3J$ = 8,8 Hz, $^4J$ = 2,5 Hz, Ar H), 7,76 (dd, 1 H, $^3J$ = 8,5 Hz, $^4J$ = 1,9 Hz, Ar H), 7,86 (d, 1 H, $^3J$ = 8,5 Hz, Ar H), 7,90 (d, 1 H, $^3J$ = 8,5 Hz, Ar H), 7,94 (dd, 1 H, $^3J$ = 6,6 Hz, $^4J$ = 1,6 Hz, Pyr. H-3, Pyr. H-5), 8,15 (d, 1 H, $^4J$ = 1,9 Hz, Ar H), 8,75 (dd, 1H, $^3J$ = 6,3 Hz, $^4J$ = 1,3 Hz, Pyr. H-2, Pyr. H-6). IR $cm^{-1}$: $\nu_{max}$ 3040, 2938, 2840, 1699, 1621, 1488, 1210. MS *m/z* 236 ($MH^+$).

Beispiel 2: Synthese von Indanen und 3,4-Dihydronaphthalinen 32 bis 46.

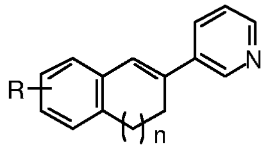

**32-37**

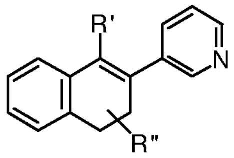

**38-42**

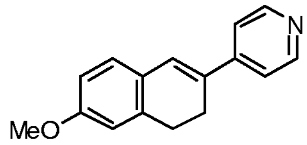

**43**

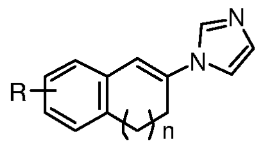

**44-46**

| Nr. | n | R | Nr. | R' | R" |
|---|---|---|---|---|---|
| 32 | 0 | H | 39 | Et | H |
| 33 | 1 | H | 40 | H | 3- Me |
| 34 | 0 | 6-OMe | 41 | H | 4- Me |
| 35 | 1 | 6-OMe | 42 | H | 4- Et |
| 36 | 0 | 7-OMe | Nr. | n | R |
| 37 | 1 | 7- OMe | 44 | 0 | H |
| Nr. | R' | R" | 45 | 1 | H |
| 38 | Me | H | 46 | 1 | 6-OMe |

A) Synthese der Verbindungen 32 bis 46:

Allgemeine Synthese der Verbindungen 32-33, 35, 37, 43: Das generelle Vorgehen zur Synthese der Pyridyl-substituierten Verbindungen 32-33, 35, 37, 43 war wie in folgendem Schema dargestellt:

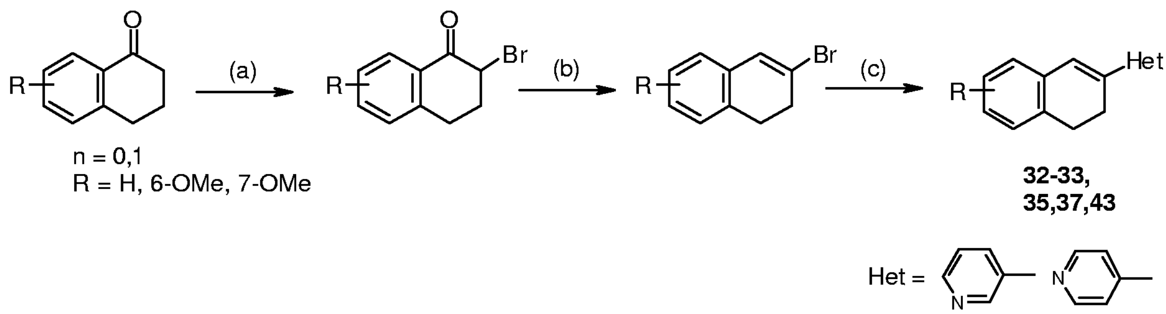


n = 0,1
R = H, 6-OMe, 7-OMe

**32-33, 35,37,43**

Reaktionsbedingungen: (a) $TBABr_3$, $CH_2Cl_2$, RT; (b) 1) $NaBH_4$, MeOH, 0°C 2) pTSA, Toluol, Reflux; (c) 3- oder 4-Pyridylboronsäure, $PdP(Ph_3)_4$, $Na_2CO_3$, DME, 80°C.

Zu einer Lösung des substituierten Ketons (1 eq) in Dichlormethan/methanol (2,5:1) wurde $TBABr_3$ (1,1 eq) bei RT zugegeben. Die Mischung wurde gerührt, bis sich die orange Lösung entfärbte. Das Lösemittel wurde *in vacuo* entfernt und das Präzipitat mit Diethylether extrahiert. Die Etherphase wurde über $MgSO_4$ getrocknet, gefiltert und das Lösemittel *in vacuo* entfernt.

Eine Lösung des α-Bromketons (1 eq) in wasserfreiem Methanol/Tetrahydrofuran (1:1) wurde unter Stickstoffatmosphäre in einem Eisbad gerührt. $NaBH_4$ (0,7 eq) wurde portionsweise zugegeben. Nach 15 min Rühren bei 0°C und 30 min Rühren bei RT wurde die Mischung in Wasser geschüttet und mit Diethylether extrahiert. Die organische Phase wurde über $MgSO_4$ getrocknet, gefiltert und das Lösemittel *in vacuo* entfernt.

Eine Mischung des resultierenden Alkohols (1 eq) und von p-Toluolsulfonsäure (0,1 eq) in Toluol wurde 2 h rückflussgekocht (mit einer Dean-Stark-Falle) um sämtliches Wasser zu entfernen. Die Mischung wurde mit gesättigter Natriumbicarbonatlösung und Wasser gewaschen, Die organische Phase wurde über $MgSO_4$ getrocknet, gefiltert und das Lösemittel *in vacuo* entfernt.

Nach Reinigung wurde eine Mischung aus Bromverbindung (1 eq), 3- oder 4-Pyridylboronsäure (1,3 eq), Natriumcarbonat (2,1 eq) und Tetrakis(triphenylphosphin)palladium (0,02 eq) in Ethylenglycoldimethylether über Nacht bei 80°C gehalten. Nach Abkühlen auf RT und Zugabe von Wasser wurde die Mischung mit Dichlormethan extrahiert, die organische Phase wurde über $MgSO_4$ getrocknet, gefiltert und das Lösemittel *in vacuo* entfernt. Das Endprodukt wurde durch Säulenchromatographie gereinigt und charakterisiert.

Allgemeine Synthese der Verbindungen 34, 36, 40-42: Zur Synthese der 3-Pyridylsubstitierten Verbindungen 34, 36, 40-42 wurde wie folgt vorgegangen:

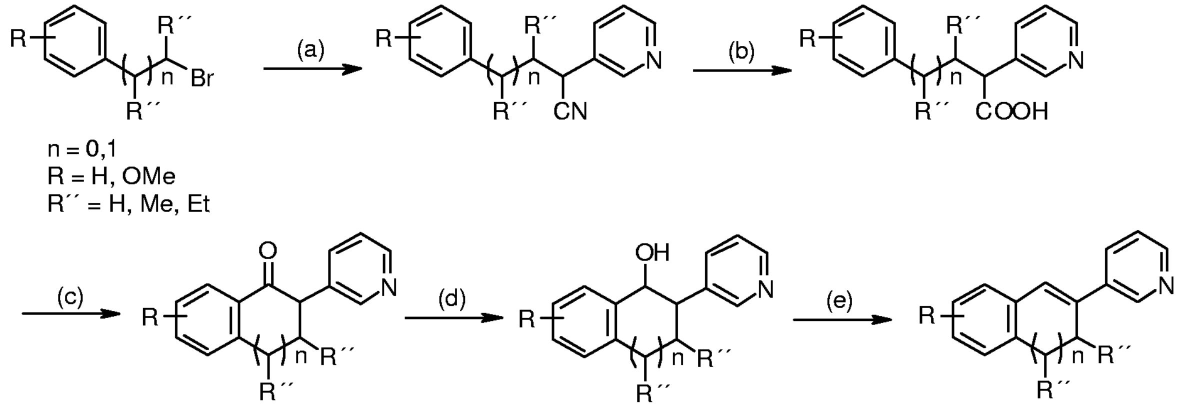


**34,36,40-42**

Reaktionsbedingungen: (a) 3-Pyridylacetonitril, $NaNH_2$, DMF; (b) NaOH, EtOH, Reflux; (c) PPA, 110 °C; (d) $NaBH_4$, MeOH, 0 °C; (e) $CH_3COOH/H_2SO_4$, 100 °C.

Diese Methode wurde bereits von Bencze und Barsky für die Synthese von 3-(3,4-dihydronaphthalin-2-yl)pyridin 33 beschrieben (Bencze, W.L. und Barsky, L.I., J. Med. Pharm. Chem. 5:1298-1306 (1962)), nicht jedoch für die der neuen Verbindungen 34, 36, 40-42.

Zu einer $NaNH_2$ (1,2 eq)-Suspension in 20 ml wasserfreiem DMF (Dreihalskolben, Rückflusskühler, Gaseinlass und Tropftrichter mit Septum) unter Stickstoffatmosphäre wurde unter Rühren und Eisbadkühlung tropfenweise 3-Pyridylacetonitril (1,07 eq) gegeben. Nach 1 h Rühren bei RT und 1h bei 80°C wurde die Mischung mit einem Eisbad gekühlt und die Bromverbindung (1 eq) tropfenweise zugegeben. Die Reaktionsmischung wurde dann 3 h bei RT und 1 h bei 80°C gerührt. Nach Abkühlung wurde ein Wasserüberschuss zugegeben und die Mischung mit Diethylether extrahiert. Die organische Phase wurde mit Wasser gewaschen, über $MgSO_4$ getrocknet, gefiltert und das Lösemittel *in vacuo* entfernt. Das Produkt wurde durch Säulenchromatographie gereinigt (Elution mit $CH_2Cl_2$/MeOH (99:1)).

Zu einer Lösung des resultierenden Nitrils (1 eq) in wenigen ml Ethanol wurde NaOH (11 eq) in Wasser zugegeben. Nach 24 h Reflux wurde Wasser zugegeben und mit 2 N HCl und wässriger Essigsäure auf pH 5 eingestellt. Die Mischung wurde mit Ethylacetat extrahiert, die organische Phase wurde über $MgSO_4$ getrocknet, gefiltert und das Lösemittel *in vacuo* entfernt. Die resultierende Säure wurde ohne weitere Reinigung im nächsten Reaktionsschritt eingesetzt.

Eine Mischung aus Polyphosphorsäure (2,2 g) und der so hergestellten Säure (0,53 g, 2,05 mmol) wurde 20 min bei 110 °C gerührt. Die Mischung wurde in Eiswasser gegossen und mit 6%iger NaOH neutralisiert. Mit Natriumbicarbonat wurde pH 8 eingestellt und die Lösung mit Diethylether extrahiert. Die organische Phase wurde über $MgSO_4$ getrocknet, gefiltert und das Lösemittel *in vacuo* entfernt. Das Produkt wurde durch Säulenchromatographie gereinigt.

Eine Lösung des Ketons (1 eq) in wasserfreiem Methanol wurde unter Stickstoffatmosphäre in einem Eisbad gerührt. $NaBH_4$ (2 eq) wurde portionsweise zugegeben. Nach 1 h Rühren bei RT wurde die Mischung in Wasser gegossen und mit Dichlormethan extrahiert. Die organische Phase wurde über $MgSO_4$ getrocknet, gefiltert und das Lösemittel *in vacuo* entfernt. Der resultierende Alkohol wurde ohen weitere Reinigung für den nächsten Reaktionsschritt verwendet.

Eine Mischung aus 1 ml Essigsäure, 0,14 ml konz. Schwefelsäure und dem Alkohol (0,30 mmol) wurde 1 h bei

100°C gerührt. Das Gemisch wurde in Eiswasser geschüttet und mit einer 6%igen NaOH basifiziert. Nach Extraktion mit Dichlormethan wurde die organische Phase wurde über $MgSO_4$ getrocknet, gefiltert und das Lösemittel *in vacuo* entfernt. Das Produkt wurde säulenchromatographisch gereinigt (Elution mit $CH_2Cl_2$/MeOH (98:2)).

Synthese von Verbindungen 38 und 39: Die Synthese von 3-(1-Methyl-3,4-dihydronaphthalin-2-yl)pyridin 38 wurde ebenfalls von Bencze und Barsky beschrieben (Bencze, W.L. und Barsky, L.I., J. Med. Pharm. Chem. 5:1298-1306 (1962)); 3-(1-Ethyl-3,4-dihydronaphthalin-2-yl)pyridin 39 ist bislang nicht beschrieben:

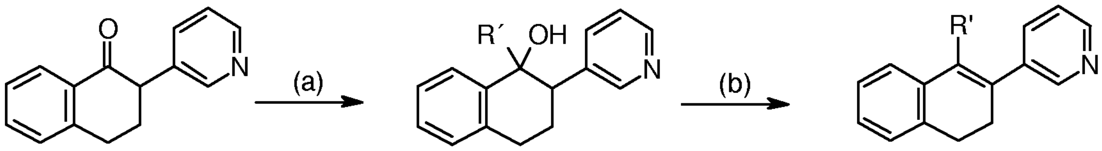


**38, 39**

**38** R' = Me
**39** R' = Et

Reaktionsbedingungen: (a) R'MgHal, Toluol, Reflux; (b) HCl, 100 °C, 2 h.

Allgemeine Synthese der Verbindungen 44-46: Das Vorgehen für die Synthese der 1-Imidazolylsubstituierten Verbindungen 44-46 war wie im folgenden Schema:

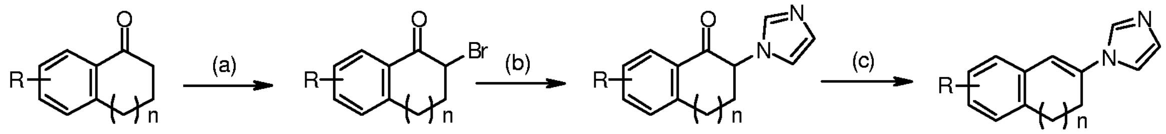


n = 0,1
R = H, 6-OMe

**44-46**

Reaktionsbedingungen: (a) $TBABr_3$, $CH_2Cl_2$, RT; (b) Imidazol, DMF, RT; (C) 1) $NaBH_4$, MeOH, 0 °C 2) $CH_3COOH/H_2SO_4$, 120 °C.

Diese Methode wurde bereits von Cozzi et al. für die Synthese von 1-(3,4-Dihydronaphthalin-2-yl)-1*H*-imidazol 45 und 1-(6-Methoxy-3,4-dihydronaphthalin-2-yl)-1*H*-imidazol 46 beschrieben (Cozzi, P. et al., Eur.J.Med.Chem. 26:423-433 (1991)), nicht jedoch für Verbindung 44.

Zu einer Lösung des substituierten Ketons (1 eq) in Dichlormethan/methanol (2,5:1) wurde bei RT $TBABr_3$ (1,1 eq) gegeben. Die Mischung wurde bis zur Entfärbung der orangen Lösung gerührt. Das Lösemittel wurde in vacuo entfernt und das Präzipitat mit Diethylether extrahiert. Die Etherphase wurde über $MgSO_4$ getrocknet, gefiltert und das Lösemittel *in vacuo* entfernt.

Eine Lösung von $\alpha$-Bromketon (1 eq) und Imidazol in DMF wurde über Nacht bei RT gerührt. Die Mischung wurde in Eiswasser geschüttet und mit Dichlormethan extrahiert. Die organische Phase wurde gründlich mit Wasser gewaschen, über $MgSO_4$ getrocknet, gefiltert und das Lösemittel *in vacuo* entfernt. Das Produkt wurde säulenchromatographisch gereinigt.

Eine Lösung des 1-Imidazolylsubstituierten Ketons (1 eq) in wasserfreiem MeOHwurde in einem Eisbad unter Stickstoffatmosphäre gerührt und portionsweise mit $NaBH_4$ (2 eq) versetzt. Nach 1 h Rühren bei RT wurde die Mischung in Wasser geschüttet und mit Dichlormethan extrahiert. Die organische Phase wurde über $MgSO_4$ getrocknet, gefiltert und das Lösemittel *in vacuo* entfernt. Der resultierende Alkohol wurde ohen weitere Reinigung für den nächsten Reaktionsschritt verwendet.

Der Alkohol wurde in einer Mischung aus Eisessig und konzentrierter Schwefelsäure gelöst und 4 h auf 100 °C erhitzt. Nach Abkühlung auf RT wurde die Mischung auf Eis geschüttet, mit NaOH neutralisiert und mit Dichlormethal extrahiert. Die organische Phase wurde über $MgSO_4$ getrocknet, gefiltert und das Lösemittel *in vacuo* entfernt. Das Endprodukt wurde säulenchromatographisch aufgereinigt.

B) Reinigungsbedingungen, Ausbeute und Charakterisierung der Titelverbindungen:

3-(1*H*-Inden-2-yl)pyridin (32). Reinigung: CC ($CH_2Cl_2$/MeOH, 99:1) Ausbeute 51%. $^1$H NMR (500 MHz, $CDCl_3$): $\delta$ = 3,81 (s, 2H, H-3), 7,23 (td, 1 H, $^3J$ = 7,2 Hz, $^4J$ = 0,9 Hz, Ar H), 7,29-7,32 (m, 3H, Ar H, Pyr. H-5), 7,44 (d, 1 H, $^3J$ =

7,2 Hz, Ar H), 7,50 (d, 1 H, $^3J$ = 7,2 Hz, Ar H), 7,89 (dt, 1 H, $^3J$ = 8,1 Hz, $^4J$ = 1,9 Hz, Pyr. H-4), 8,50 (s, 1 H, Pyr. H-6), 8,90 (s, 1H, Pyr. H-2). IR cm$^{-1}$: $\nu_{max}$ 3054, 2916, 1533. MS *m/z* 194 (MH$^+$).

3-(3,4-Dihydronaphthalin-2-yl)pyridin (33). Reinigung: CC ($CH_2Cl_2$/MeOH, 98:2) Ausbeute 62%. $^1$H NMR (500 MHz, $CDCl_3$) $\delta$ 2,73 (t, 2H, $^3J$ = 8,3 Hz, H-4), 2,97 (t, 2H, $^3J$ = 7,8 Hz, H-3), 6,89 (s, 1 H, H-1), 7,13-7,19 (m, 4H, Ar H), 7,32-7,34 (m, 1H, Pyr. H-5), 7,85 (td, 1H, $^3J$ = 8,1 Hz,$^4J$ = 1,5 Hz, Pyr. H-4), 8,49 (dd, 1H, $^3J$ = 4,9 Hz, $^4J$ = 1,5 Hz, Pyr. H-2), 8,79 (d, 1H, $^4J$ = 1,9 Hz, Pyr. H-6). IR cm$^{-1}$ : $\nu_{max}$ 3022, 2935, 2885, 2831, 1485, 1421. MS *m/z* 208 (MH$^+$), 179, 101, 79. 3-(6-Methoxy-1 *H*-inden-2-yl)pyridin (34). Reinigung: CC ($CH_2Cl_2$/MeOH, 98:2) Ausbeute 45%. $^1$H NMR (500 MHz, $CDCl_3$) $\delta$ 3,77 (s, 2H, H-3), 3,85 (s, 3H, $OCH_3$), 6,86 (dd, 1H, $^3J$ = 8,2 Hz, $^4J$ = 2,2 Hz, Ar H), 7,09 (d, 1 H, $^4J$ = 1,5 Hz, Ar H), 7,30 (s, 1 H, H-1), 7,34 (d, 1 H, $^3J$ = 8,2 Hz, Ar H), 7,36-7,39 (m, 1H, Pyr. H-5), 7,94 (dt, 1 H, $^3J$ = 7,9 Hz, $^4J$ = 1,5 Hz, Pyr. H-4), 8,46 (dd, 1 H, $^3J$ = 4,9 Hz, $^4J$ = 1,5 Hz, Pyr. H-6), 8,85 (d, 1H, $^4J$ = 1,8 Hz, Pyr. H-2). IR cm$^{-1}$: $\nu_{max}$ 3059, 2905, 2836, 1685, 1607, 1493, 1259. MS *m/z* 224 (MH$^+$).

3-(6-Methoxy-3,4-dihydronaphthalin-2-yl)pyridin (35). Reinigung: CC ($CH_2Cl_2$/MeOH, 98:2) Ausbeute 89%. $^1$H NMR (500 MHz, $CDCl_3$): $\delta$ 2,73 (t, 2H, $^3J$ = 8,5 Hz, H-3), 2,96 (t, 2H, $^3J$ = 8,5 Hz, H-4), 3,82 (s, 3H, $OCH_3$), 6,73-6,75 (m, 2H, Ar H), 6,88 (s, 1H, H-1), 7,10 (d, 1 H, $^3J$ = 8,2 Hz, Ar H), 7,32-7,35 (m, 1H, Pyr. H-5), 7,86 (dt, 1 H, $^3J$ = 8,2 Hz, $^4J$ = 1,6 Hz, Pyr. H-4), 8,48 (dd, 1 H, $^3J$ = 4.7 Hz, $^4J$ = 1,6 Hz, Pyr. H-6), 8,79 (d, 1 H, $^4J$ = 2,5 Hz, Pyr. H-2). IR cm$^{-1}$: $\nu_{max}$ 3021 , 2936, 2834, 1607, 1570, 1499, 1250. MS *m/z* 238 (MH$^+$) , 223, 194,165. 3-(7-Methoxy-1*H*-inden-2-yl)pyridin (36). Reinigung: CC ($CH_2Cl_2$/MeOH, 99:1) Ausbeute 34%. $^1$H NMR (500 MHz, $CDCl_3$): $\delta$ 2,73 (t, 2H, $^3J$ = 8,5 Hz, H-3), 2,96 (t, 2H, $^3J$ = 8,5 Hz, H-4), 3,82 (s, 3H, $OCH_3$), 6,73-6,75 (m, 2H, Ar H), 6,88 (s, 1 H, Ar H), 7,10 (d, 1 H, $^3J$ = 8,2 Hz, Ar H), 7,32-7,35 (m, 1H, Pyr. H-5), 7,86 (dt, 1 H, $^3J$ = 8,2 Hz, $^4J$ = 1,6 Hz, Pyr. H-4), 8,48 (dd, 1 H, $^3J$ = 4,7 Hz, $^4J$ = 1,6 Hz, Pyr. H-6), 8,79 (d, 1 H, $^4J$ = 2,5 Hz, Pyr. H-2). IR cm$^{-1}$ : $\nu_{max}$ 3034, 2938, 2836, 1595, 1483, 1263, 1088. MS *m/z* 224 (MH$^+$).

3-(7-Methoxy-3,4-dihydronaphthalin-2-yl)pyridin (37). Reinigung: CC ($CH_2Cl_2$/MeOH, 98:2) Ausbeute 79%. $^1$H NMR (500 MHz, $CDCl_3$): $\delta$ 2,74 (t, 2H, $^3J$= 8,2 Hz, H-3), 2,92 (t, 2H, $^3J$ = 8,2 Hz, H-4), 3,81 (s, 3H, $OCH_3$), 6,73-6,75 (m, 2H, Ar H), 6,87 (s, 1 H, H-1), 7,09 (d, 1 H, $^3J$ = 8,2 Hz, Ar H), 7,35-7,38 (m, 1H, Pyr. H-5), 7,88 (dt, 1 H, $^3J$ = 7,8 Hz, $^4J$ = 1,9 Hz, Pyr. H-4), 8,51 (dd, 1H, $^3J$ = 5,0 Hz, $^4J$ = 1,6 Hz, Pyr. H-6), 8,80 (d, 1 H, $^4J$ = 1,6 Hz, Pyr. H-2). IR cm$^{-1}$: $\nu_{max}$ 3025, 2934, 2833, 1604, 1571, 1497, 1255, 1040. MS *m/z* 238 (MH$^+$).

3-(1-Methyl-3,4-dihydronaphthalin-2-yl)pyridin (38). Reinigung: CC ($CH_2Cl_2$/MeOH, 97:3) Ausbeute 63%. $^1$H NMR (500 MHz, $CDCl_3$): $\delta$ 2,05 (s, 3H, $CH_3$), 2,57 (td, 1 H, $^3J$ = 8,2 Hz, $^4J$ = 1,6 Hz, H-3), 2,92 (t, 1 H, $^3J$ = 8,2 Hz, H-4), 7,19-7,23 (m, 2H, Ar H), 7,27 (t, 1 H, $^3J$ = 7,6 Hz, Ar H), 7,38 (d, 1 H, $^3J$ = 7,6 Hz, Ar H), 7,41-7,45 (m, 1 H, Pyr. H-5), 7,72 (td, 1 H, $^3J$ = 7,92 Hz, $^4J$ = 1,6 Hz, Pyr. H-4), 8,54 (dd, 1 H, $^3J$ = 5,0 Hz, $^4J$ = 1,6 Hz, Pyr. H-6), 8,57 (s, 1H, Pyr. H-2). IR cm$^{-1}$ : $\nu_{max}$ 3024, 2937, 2831, 1602, 1487, 1408, 1250. MS *m/z* 222 (MH$^+$).

3-(1-Ethyl-3,4-dihydronaphthalin-2-yl)pyridin (39). Reinigung: CC ($CH_2Cl_2$/MeOH, 99:1) Ausbeute %. $^1$H NMR (500 MHz, $CDCl_3$) : $\delta$ 1,05 (t, 3H, $^3J$ = 7,6 Hz, $CH_3$), 2,47-2,54 (m, 4H, $CH_2$, H-3), 2,87 (t, 2H, $^3J$ = 7,6 Hz, H-4), 7,18-7,20 (m, 2H, Ar H), 7,24-7,27 (m, 1 H, Ar H), 7,30-7,33 (m, 1H, Pyr. H-5), 7,38 (d, 1H, $^3J$ = 7,6 Hz, Ar H), 7,56 (dt, 1H, $^3J$ = 7,6 Hz, $^4J$ = 1,8 Hz, Pyr. H-4), 8,51-8,53 (m, 2H, Pyr. H-6, Pyr. H-2). IR cm$^{-1}$ : $\nu_{max}$. MS *m/z* (MH$^+$).

3-(3-Methyl-3,4-dihydronaphthalin-2-yl)pyridin (40). Reinigung: CC ($CH_2Cl_2$/MeOH, 98:2) Ausbeute 63%. $^1$H NMR (500 MHz, $CDCl_3$) $\delta$ 1,03 (d, 3H, $^3J$ = 6,9 Hz, $CH_3$), 2,76 (dd, 1 H, $^2J$ = 15,4 Hz, $^3J$ = 2,2 Hz, H-4), 3,00-3,06 (m, 1 H, H-3), 3,23 (dd, 1 H, $^2J$ = 15,4 Hz, $^3J$ = 6,9 Hz, H-4'), 6,86 (s, 1 H, H-1), 7,1 6-7,22 (m, 2H, Ar H), 7,36 (m, 1 H, Pyr. H-5), 7,92 (dt, 1 H, $^3J$ = 7,9 Hz, $^4J$ = 1,6 Hz, Pyr. H-4), 8,52 (dd, 1 H, $^3J$ = 4,7 Hz, $^4J$ = 1,9 Hz, Pyr. H-6), 8,85 (d, 1H, $^4J$ = 1,9 Hz, Pyr. H-2). IR cm$^{-1}$ : $\nu_{max}$ 3020, 2962, 2924, 1564, 1453, 1216. MS *m/z* 222 (MH$^+$).

3-(4-Methyl-3,4-dihydronaphthalin-2-yl)pyridin (41). Reinigung: CC ($CH_2Cl_2$/MeOH, 99:1) Ausbeute 51% $^1$H NMR (500 MHz, $CDCl_3$) $\delta$ 1,32 (d, 3H, $^3J$ = 6,9 Hz, $CH_3$), 2,54 (ddd, 1 H, $^2J$ = 16,1 Hz, $^3J$ = 7,6 Hz, $^4J$ = 0,9 Hz, H-3), 2,87 (ddd, 1H, $^2J$ = 16,1 Hz, $^3J$ = 6,6 Hz, $^4J$ = 1,6 Hz, H-3') 3,11-3,17 (m, 1H, H-4), 6,89 (s, 1H, H-1), 7,16-7,24 (m, 4H, Ar H), 7,33-7,35 (m, 1 H, Pyr. H-5), 7,86-7,85 (td, 1 H, $^3J$ = 8,1 Hz, $^4J$ = 1,5 Hz, Pyr. H-4), 8,52 (dd, 1H, $^3J$ = 4,7 Hz, $^4J$ = 1,6 Hz, Pyr. H-6), 8,82 (d, 1 H, $^4J$ = 2,0 Hz, Pyr. H-2). IR cm$^{-1}$: $\nu_{max}$. MS *m/z* 222 (MH$^+$), 203, 178, 77.

3-(4-Ethyl-3,4-dihydronaphthalin-2-yl)pyridin (42) Reinigung: Ausbeute 55%. $^1$H NMR ($CDCl_3$) $\delta$ 0,92 (t, 3H, $^3J$ = 7,6 Hz, $CH_3$), 1,56-1,69 (m, 2H, $CH_2$), 2,68 (dd, 1 H, $^2J$ = 16,4 Hz, $^3J$ = 3,8 Hz, H-3), 2,83-2,86 (m, 1H, H-4), 2,93 (dd, 1H, $^2J$ = 16,4 Hz, $^3J$ = 2,5 Hz, H-3'), 6,86 (d, 1H, $^4J$ = 2,5 Hz, H-1), 7,16-7,22 (m, 4H, Ar H), 7,32-7,34 (m, 1 H, Pyr. H-5), 7,84 (dt, 1 H, $^3J$ = 7,9 Hz, $^4J$ = 1,6 Hz, Pyr. H-4), 8,52 (dd, 1 H, $^3J$ = 4,7 Hz, $^4J$ = 1,6 Hz, Pyr. H-6), 8,82 (d, 1 H, $^4J$ = 1,9 Hz, Pyr H-2). IR cm$^{-1}$: $\nu_{max}$ 3035, 2962, 2931, 1682, 1569, 1486, 1456, 1022. MS *m/z* 236 (MH$^+$).

4-(6-Methoxy-3,4-dihydronaphthalin-2-yl)pyridin (43). Reinigung: CC ($CH_2Cl_2$/MeOH, 97:3) Ausbeute 51%. $^1$H NMR (500 MHz, $CDCl_3$) : $\delta$ 2,66 (t, 2H, $^3J$ = 8,5 Hz, H-3), 2,91 (t, 2H, $^3J$ = 8,5 Hz, H-4), 3,77 (s, 3H, $OCH_3$), 6,69-6,71 (m, 2H, Ar H), 7,09-7,1 (m, 2H, H-1, Ar H), 7,53 (dd, 1 H, $^3J$ = 6,6 Hz, $^4J$ = 1,6 Hz, Pyr. H-3, Pyr. H-5), 8,49 (dd, 1 H, $^3J$ = 6,6 Hz, $^4J$ = 1,6 Hz, Pyr. H-2, Pyr. H-6). IR cm$^{-1}$ : $\nu_{max}$ 3040, 2939, 2835, 1599, 1504, 1209. MS *m/z* 238 (MH$^+$).

1-(1 *H*-Inden-2-yl)-1*H*-imidazol (44). Reinigung: CC ($CH_2Cl_2$/MeOH, 97:3) Ausbeute 70%. $^1$H NMR (500 MHz, $CDCl_3$): $\delta$ 3,87 (s, 2H, H-3), 6,78 (s, 1 H, H-1), 7,22-7,24 (m, 2H, Im. H-4, Ar H), 7,30-7,33 (m, 2H, Ar H, Im. H-5), 7,38 (d, 1H, $^3J$ = 7,6 Hz, Ar H), 7,45 (d, 1 H, $^3J$ = 7,6 Hz, Ar H), 8,09 (s, 1H, Im. H-2). IR cm$^{-1}$: $\nu_{max}$ 2940, 1707, 1616, 1498, 1264, 1238. MS *m/z* 183 (MH$^+$).

1-(3,4-Dihydronaphthalin-2-yl)-1*H*-imidazol (45). Reinigung: CC ($CH_2Cl_2$/MeOH, 98:2) Ausbeute 96%. $^1H$ NMR (500 MHz, $CDCl_3$): $\delta$ 2,83 (t, 2H, $^3J$ = 8,2 Hz, H-4), 3,08 (t, 1 H, $^3J$ = 8,2 Hz, H-3), 6,57 (s, 1 H, H-1), 7,12 (d, 1 H, $^3J$ = 7,8 Hz, Ar H), 7,18-7,22 (m, 4H, Im. H-4, Ar H), 7,28 (s, 1H, Im. H-5), 7,95 (s, 1H, Im. H-2). IR $cm^{-1}$: $\nu_{max}$ 3006, 3990, 1650, 1484, 1295, 1045. MS *m/z* 197 ($MH^+$).

1-(6-Methoxy-3,4-dihydronaphthalin-2-yl)-1*H*-imidazol (46). Reinigung: CC ($CH_2Cl_2$/MeOH, 98:2) Ausbeute 54%. $^1H$ NMR (500 MHz, $CDCl_3$): $\delta$ 2,79 (t, 2H, $^3J$ = 8,2 Hz, H-4), 2,96 (t, 2H, $^3J$ = 8,2 Hz, H-3), 3,75 (s, 3H, $OCH_3$), 6,76-6,77 (m, 2H, Ar H), 6,82 (s, 1 H, H-1), 7,04 (s, 1 H, Im. H-4), 7,07 (d, 1H, $^3J$ = 8,2 Hz, Ar H), 7,64 (s, 1 H, Im. H-5), 8,10 (s, 1H, Im. H-2). IR $cm^{-1}$: $\nu_{max}$ 2924, 1729, 1646, 1604, 1495, 1459, 1298, 1253. MS *m/z* 227 ($MH^+$).

Beispiel 3: Enzym-Testsysteme zum Test von Verbindungen auf Inhibition von CYP-Enzymen *in vitro*

Es wurden folgende CYP-Enzyme nach beschriebenen Methoden hergestellt und getestet: humane CYP17 (rekombinant exprimiert in *E. coli*) (Hutschenreuter, T.U. et al., J. Enzyme Inhib. Med. Chem. 19:17-32 (2004)) und humane placentale CYP19 (Hartmann, R. W. & Batzl, C., J. Med. Chem. 29:1362-1369 (1986)).

A) Isolation der CYP 17-haltigen Membranfraktion aus *E. coli* pJL17/OR: Der rekombinant veränderte *E. coli*-Stamm pJL17/OR, in dem das humane CYP17 und die Ratten NADPH-P450-Reduktase coexprimiert wurden, wurde nach der Methode von Ehmer et al. angezogen und aufbewahrt (Ehmer, P. B. et al., J. Steroid Biochem. Mol. Biol. 75:57-63 (2000)). Für die Isolation der Membranfraktion wurden 5 ml der Bakterienzellsuspension mit einer $OD_{578}$ von 50 mit Phosphatpuffer (0,05 M; pH 7,4; 1 mM $MgCl_2$; 0,1 mM EDTA und 0,1 mM DTT) gewaschen. Die Bakterien wurden abzentrifugiert und in 10 ml eiskaltem TES-Puffer (0,1 M Tris-Acetat; pH 7,8; 0,5 mM EDTA; 0,5 M Saccharose) resuspendiert. Es wurden 4 mg Lysozym in 10 ml eiskaltem Wasser zugegeben, so dass eine Endkonzentration von 0,2 mg/ml erhalten wurde. Es folgte eine dreißigminütige Inkubation unter andauerndem Schütteln auf Eis. Die Spheroplasten wurden durch einen erneuten Zentrifugationsschritt bei 12.000 g für 10 min gewonnen und erneut in 3 ml eiskaltem Phosphatpuffer resuspendiert (Zusammensetzung s.o., zusätzlich 0,5 mM PMSF).

Nach Einfrieren und Auftauen wurden die Zellen auf Eis mit einem Ultraschallstab aufgeschlossen. Die ganzen Zellen und die Zelltrümmer wurden bei 3.000 g für 7 min abzentrifugiert. Der Überstand wurde nochmals bei 50.000 g für 20 min bei 4 °C zentrifugiert. Dabei setzte sich das Membranpellet ab, das in 2ml Phosphatpuffer (Zusammensetzung s.o.) mit 20 % Glycerol mit Hilfe eines Ultra-Turrax-Stabes resuspendiert wurde. Die Proteinkonzentration wurde nach der Methode von Lowry et al. bestimmt (Lowry, O. H. et al., J Biol Chem 193:265-275 (1951)). Aliquots mit einer ungefähren Proteinkonzentration von 5 mg/ml wurden bis zum Gebrauch bei -70 °C aufbewahrt.

B) Isolierung der CYP19 (Aromatase): Das Enzym wurde aus der Mikrosomenfraktion von frischer menschlicher Plazenta (St. Josephs Krankenhaus, Saarbrücken-Dudweiler, Deutschland) nach der Methode von Thompson und Siiteri gewonnen (Thompson, E. A. & Siiteri, P. K., J. Biol. Chem. 249:5364-5372 (1974)). Die isolierten Mikrosomen wurden in einem minimalen Volumen an Phosphatpuffer (0,05 M; pH 7,4; 20% Glycerol) suspendiert. Zusätzlich wurde DTT (10 mM) und EDTA (1 mM) hinzugegeben, um das Enzym vor Abbaureaktionen zu schützen. Die Proteinkonzentration wurde nach Lowry et al. bestimmt (Lowry, O. H. et al., J. Biol. Chem. 193:265-275 (1951)) und sollte nach der Aufarbeitung etwa 35 mg/ml betragen.

C) Bestimmung der prozentualen Hemmung von CYP17: Eine Lösung von 6,25 nmol Progesteron (in 5 $\mu$l MeOH) wurde in 140 $\mu$l Phosphatpuffer (0,05 M; pH 7,4; 1 mM $MgCl_2$; 0,1 mM EDTA und 0,1 mM DTT) gelöst und zusammen mit 50 $\mu$l NADPHregenerierendem System (Phosphatpuffer mit 10 mM $NADP^+$, 100 mM Glucose-6-phosphat und 2,5 Units Glucose-6-phosphat-dehydrogenase) und Inhibitor (in 5 $\mu$l DMSO) bei 37 °C für 5 min präinkubiert. Kontrollinkubationen wurden parallel mit 5 $\mu$l DMSO ohne Hemmstoff durchgeführt. Die Reaktion wurde durch Zugabe von 50 $\mu$l einer 1 zu 5 verdünnten Membransuspension in Phosphatpuffer (0,8 - 1 mg Protein pro ml) gestartet. Nach Durchmischen des Ansatzes wurde für 30 min bei 37 °C inkubiert. Die Reaktion wurde durch Zugabe von 50 $\mu$l 1 N HCl abgestoppt.

Die Steroide wurden mit 1 ml EtOAc extrahiert. Nach einem Zentrifugationsschritt (5 min bei 2.500 g) wurden 900 $\mu$l der organischen Phase in ein Eppendorfgefäß mit 250 $\mu$l des Inkubationspuffers und 50 $\mu$l 1 N HCl überführt und erneut geschüttelt. Nach der Zentrifugation wurden 800 $\mu$l der organischen Phase abgenommen, in ein neues Gefäß gegeben und zur Trockne eingedampft. Die Proben wurden in 50 $\mu$l einer Wasser-Methanol-Mischung (1:1) gelöst und per HPLC analysiert. Der Substratumsatz wurde aus dem Verhältnis der Flächen der Produktpeaks (17$\alpha$-Hydroxyprogesteron und 16$\alpha$-Hydroxyprogesteron) gegenüber der des Substratpeaks berechnet. Die Aktivität der Inhibitoren wurde aus dem verminderten Substratumsatz nach Zugabe von Hemmstoffen nach folgender Formel berechnet:

$$\%Hemmung = \left[\left[\frac{\sum Peakflächen(Hemmstoffinkubation)}{\sum Peakflächen(Kontrollinkubation)}\right]-1\right]\cdot(-100)$$

D) Bestimmung des $IC_{50}$-Wertes von CYP19: Der Assay wurde annähernd analog zu den von Foster et al. und Graves und Salahanick beschriebenen Testverfahren durchgeführt, eine detaillierte Beschreibung findet sich in Hartmann und Batzl 1986 (Foster, A. B. et al., J Med Chem 26:50-54 (1983); Graves, P. E. & Salhanick, H. A., Endocrinology 105:52-57 (1979); Hartmann, R. W. & Batzl, C., J. Med. Chem. 29:1362-1369 (1986)). Dabei wurde die Enzymaktivität durch Messung des bei der Aromatisierung aus [1β-$^3$H] Androstendion gebildeten $^3H_2O$ verfolgt. Jedes Reaktionsgefäß enthielt 15 nM radioaktiv markiertes [1β-$^3$H] Androstendion (entspricht 0,08 μCi) und 485 nM unmarkiertes Androstendion, 2mM NADP$^{\oplus}$, 20 mM Glucose-6-phosphat, 0,4 Units Glucose-6-phosphatdehydrogenase und Hemmstoff (0 - 100 μM) in Phosphatpuffer (0,05 M; pH 7,4). Die zu testenden Verbindungen wurden in DMSO gelöst und mit Puffer auf die gewünschte Konzentration verdünnt. Die endgültige DMSO-Konzentration der Kontroll- und der Hemmstoffinkubation betrug ca. 2 %. Jedes Gefäß wurde für 5 min in einem Wasserbad bei 30 °C präinkubiert. Durch die Zugabe des mikrosomalen Proteins (0,1 mg) wurde die Reaktion gestartet. Das Gesamtvolumen jedes Ansatzes betrug 200 μl. Durch Zugabe von 200 μl eiskalter 1 m M $HgCl_2$-Lösung wurde die Reaktion nach 14 min abgestoppt. Es wurden 200μl einer 2 %-igen wässrigen Suspension von mit Dextran überzogener Aktivkohle (dextran-coated charcoal, DCC) zur Absorbtion der Steroide zugegeben, und die Gefäße wurden 20 min geschüttelt. Danach wurde die Aktivkohle bei 1.500 g für 5 min abzentrifugiert. Das im Überstand befindliche radioaktive Wasser ($^3H_2O$) wurde durch Scintillationsmessung mittels eines LKB-Wallac β-Counters bestimmt. Die Berechnung der $IC_{50}$-Werte erfolgte durch eine halblogarithmische Auftragung der prozentualen Hemmung gegen die Inhibitorkonzentration. Hieraus wurde die molare Konzentration, bei der 50% Hemmung auftritt, abgelesen.

Beispiel 4: Biologische Testsysteme zum Test von Verbindungen auf selektive Inhibition von humaner CYP11B1 und CYP11B2 *in vitro*

A) Screening-Test in transgener Spalthefe: Eine Spalthefesuspension (*S. pombe* PE1) mit einer Zelldichte von $3\cdot10^7$ Zellen/ml wurde aus einer frisch gewachsenen Kutur hergestellt unter Verwendung von frischem EMMG (pH 7,4), modifiziert nach Ehmer et al. (Ehmer, P. B. et al., J. Steroid. Biochem. Mol. Biol. 81, 173-179 (2002)). 492,5 μl dieser Zellsuspension wurden mit 5 μl Inhibitorlösung (50 μM der zu testenden Verbindung in Ethanol oder DMSO) versetzt und 15 min bei 32 °C inkubiert. Kontrollen wurden mit 5 μl Ethanol versetzt. Die Enzymreaktion wurde durch Zugabe von 2,5 μl 11-Deoxycorticosteron (20 μM, enthaltend 1,25 nCi [4-$^{14}$C] 11-Deoxycorticosteron, in Ethanol) gestartet, dann wurde horizontal bei 32°C6 h geschüttelt. Der Test wurde durch Extraktion der Probe mit 500 μl EtOAc gestoppt. Nach Zentrifugation (10.000 g, 2 min), wurde die EtOAc-Phase abgenommen und bis zur Trockne eingedampft. Der Rückstand wurde in 10 μl Chloroform aufgenommen. Die Umsetzung des Substrats zu Corticosteron wurde durch HPTLC (siehe unten) analysiert.

Die Quantifizierung der Spots für das Substrat Deoxycorticosteron und der gebildeten Produkte Corticosteron (und, sofern nachweisbar, 18-Hydroxycorticosteron und Aldosteron) erfolgte mit dem zugehörigen Auswerteprogramm AIDA. Für die in S. *pombe* exprimierte humane Aldosteronsynthase wurde als Produkt nur Corticosteron sowie das Substrat Deoxycorticosteron erfasst. 18-Hydroxycorticosteron und Aldosteron wurden bei einer Inkubationszeit von 6 Stunden nicht in nachweisbaren Konzentrationen gebildet und gingen daher nicht in die Auswertung mit ein. Die Berechnung der *Konversion* erfolgte gemäß Gleichung 1.

Gleichung 1:

$$\%P = \frac{[PSL_B] - PSL_{HG}}{[PSL_{DOC} + PSL_B] - 2\times PSL_{HG}} \times 100$$

| | |
|---|---|
| $\%P$ | Konversion (prozentualer Anteil des Produktes an Gesamtsteroid) |
| $PSL$ | Phospho Stimulated Luminescence (Lumineszenzwert) |
| $PSL_B$ | PSL für Corticosteron (B) |
| $PSL_{DOC}$ | PSL für Deoxycorticosteron (DOC) |
| $PSL_{HG}$ | PSL des Hintergrundes |

Die *prozentuale Hemmung,* die durch einen Hemmstoff in der jeweils eingesetzten Konzentration verursacht wurde, errechnete sich nach Gleichung 2.

Gleichung 2:

$$\% H = \left[ 1 - \frac{\% P_H}{\% P_K} \right] \times 100$$

| | |
|---|---|
| % *H* | prozentuale Hemmung |
| % *P* | Prozentwert der Konversion des Substrates zu Produkten |
| % $P_H$ | Prozentuale Konversion in Anwesenheit eines Hemmstoffs |
| % $P_K$ | Prozentuale Konversion der Kontrolle |

B) Test auf selektive CYP11B1 - und CYP11B2-Inhibitoren: *Erhaltung der Zellen:* V79 MZh1 1 B1 und V79 MZh1 1 B2, welche die humane A-dosteronsynthase bzw. Steroid-11-β-Hydroxylase rekombinant exprimieren und nach Denner et al. hergestellt wurden (Denner, K. et al., Pharmacogenetics 5:89-96 (1995)), wurden in einem $CO_2$-Inkubator bei 37 °C und in wasserdampfgesättigter Atmosphäre mit 5% $CO_2$ in Zellkulturschalen mit 60 oder 90 mm Durchmesser kultiviert. Beide Zelllinien wurden in DMEM$^+$ kultiviert, welches 10 % FCS und zum Schutz vor bakterieller Kontamination die Antibiotika Penicillin und Streptomycin (1%) enthielt. Die Zellen wurden alle 2 - 3 Tage nach Behandlung mit Trypsin/EDTA passagiert, da die Verdopplungsdichte je nach Zellzahl 1 - 2 Tage betrug. Die Zellen wurden maximal 12 - 15 mal passagiert, um mögliche Zellveränderungen auszuschließen. Bei weiterem Bedarf wurden frisch aufgetaute Zellen eingesetzt.

| *DMEM$^+$ - Medium* | |
|---|---|
| DMEM-Pulvermedium | 13,4 g |
| $NaHCO_3$ | 3,7 g |
| L-Glutam in (200 m M) | 20,0 ml |
| Penicillin (100 Einheiten /ml)/Streptomycin (0,1 mg/ml) | 10,0 ml |
| Natrium pyruvat (100 mM) | 10,0 ml |
| Fetales Kälberserum (FCS) | 100 ml |
| $H_2O$ bidest. | ad 11 |

Der pH-Wert der Mediums wurde auf 7,2-7,3 eingestellt. FCS wurde erst nach der Sterilfiltration zugesetzt.

*Inhibitionstest:* V79 MZh 11B1- und V79 MZh 11B2-Zellen ($8 \cdot 10^5$ Zellen pro well) wurden auf 24-well Zellkulturplatten mit 1,9 $cm^2$ Kulturfläche pro well (Nunc, Roskilde, Dänemark) bis zur Konfluenz angezogen. Vor dem Test wurde das vorhandene DMEM Kulturmedium entfernt, und es wurden 450 μl frisches DMEM mit Inhibitor in mindestens drei verschiedenen Konzentrationen in jedes well zugegeben, um den $IC_{50}$-Wert zu bestimmen. Nach Präinkubation (60 min, 37°C) wurde die Reaktion durch Zugabe von 50 μl DMEM mit 2,5 μl Lösung des Substrats 11-Deoxycorticosteron (20 μM, enthaltend 1,25 nCi [4-$^{14}$C] 11-Deoxycorticosteron, in Ethanol) gestartet. Danach wurde die Platte bei 37°C und 5% $CO_2$ im $CO_2$-Inkubator aufbewahrt. Die V79 MZh 11 B1-Zellen wurden 120 m in inkubiert, die V79 MZh 11B2-Zellen 40 min. Kontrollen ohne Inhibitor wurden in derselben Weise behandelt. Die Enzymreaktionen wurden durch Extraktion des Überstands mit 500 μl EtOAc gestoppt. Die Proben wurden zentrifugiert (10000·g, 2 min), das Lösungsmittel wurde abgenommen und evaporiert. Der Rückstand wurde in 10 μl Chloroform aufgenommen und durch HPTLC (siehe unten) analysiert.
Die Konversion bei V79 MZh11B1 wurde analog Gleichung 1 (Bsp. 4A) berechnet, wobei:

| | |
|---|---|
| $PSL_B$ | PSL für Cortisol bzw. Corticosteron |
| $PSL_{DOC}$ | PSL für Deoxycortisol (RSS) bzw. Deoxycorticosteron |

Für V79 MZh11B2 ergab sich die Konversion entsprechend Gleichung 3:

Gleichung 3:

$$\%P = \frac{[PSL_B + PSL_{18OHB} + PSL_{Aldo}] - 3 \times PSL_{HG}}{[PSL_{DOC} + PSL_B + PSL_{18OHB} + PSL_{Aldo}] - 4 \times PSL_{HG}} \times 100$$

| | |
|---|---|
| %P | Konversion (Anteil des Produktes an Gesamtsteroid |
| *PSL* | Phospho Stimulated Lumineszence (Lumineszenzwert) |
| $PSL_B$ | PSL für Corticosteron (B) |
| $PSL_{18OHB}$ | PSL für 18-Hydroxycorticosteron (18OHB) |
| $PSL_{Aldo}$ | PSL für Aldosteron |
| $PSL_{DOC}$ | PSL für 11-Deoxycorticosteron (DOC) |
| $PSL_{HG}$ | PSL des Hintergrundes |

Die prozentuale Hemmung, die durch einen Hemmstoff in der jeweils eingesetzten Konzentration verursacht wurde, errechnete sich nach Gleichung 2 (Bsp. 4A).

*Bestimmung des $IC_{50}$- Wertes:* Der $IC_{50}$-Wert ist definiert als die Konzentration des Hemmstoffes, bei der das Enzym zu 50% gehemmt wird. Er wurde durch Bestimmung der prozentualen Hemmung bei mindestens 3 verschiedenen Hemmstoff-Konzentrationen, die alle im linearen Bereich der sigmoiden $IC_{50}$-Kurve (log C/% Hemmung) liegen müssen, berechnet. Die Kalkulation erfolgte durch lineare Regression. Die bestimmten Werte wurden nur verwendet, wenn sie mit einer Wahrscheinlichkeit von r < 0,95 eine Gerade bildeten.

C) HPTLC Analyse und Phospho-Imaging der radioaktiv markierten Steroide: Der resuspendierte Rückstand aus Beispiel 4A oder 4B - enthaltend die radioaktiv markierten Steroide - wurde auf eine HPTLC-Platte (20 x 10 cm, Silicagel $60F_{254}$) mit Konzentrationszone (Merck, Darmstadt, Deutschland) aufgetragen. Die Platte wurde zweimal mit dem Laufmittel Chloroform: Methanol: Wasser (300:20:1) entwickelt. Unmarkiertes 11-Deoxycorticosteron und Corticosteron wurden als Referenz für die CYP11B1-Reaktion aufgetragen. Für die CYP11 B2-Reaktion wurden 11-Deoxycorticosteron, Corticosteron, 18-Hydroxycorticosteron und Aldosteron als Referenz verwendet. Die Detektion der unmarkierten Referenzen erfolgte bei 260 nm. Anschliessend wurden Imaging-Platten (BAS MS2340, für $^{14}C$-Proben, Raytest, Straubenhardt, Deutschland) 48 h mit den HPTLC-Platten belichtet. Die Imaging-Platten wurden mit dem Phosphoimager-System Fuji FLA 3000 (Raytest, Straubenhardt, Deutschland) gescannt und die Steroide quantifiziert.

Beispiel 5: Inhibition adrenaler CYP11B-Enzyme *in vitro* durch heteroaryl-substituierte Naphthaline

Heteroaryl-substituierte Naphthaline wurden wie in Beispiel 3 und 4 beschrieben als Inhibitoren getestet. Die Ergebnisse der Tests sind in Tab. 1 zusammengefasst.

Tab.1 : Heteroaryl-substitutierte Naphthaline: Inhibition adrenaler CYP11B-Enzyme, CYP1 7 und CYP1 9 *in vitro.*

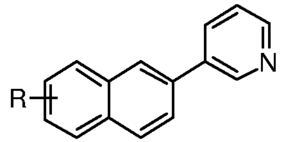

**1-5,8-10, 12-16**

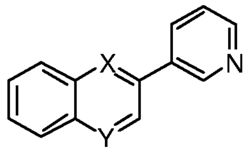

**19,20**

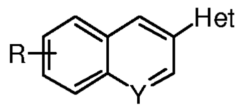

**23-24,27,29, 30**

| Verbindung | R | Het | % Inhib.[a] | $IC_{50}$-Wert (nM)[c] | | % Inhub. a [$IC_{50}$ (nM)[g]] | $IC_{50}$ (nM)[g] [% Inhib.[a]] |
|---|---|---|---|---|---|---|---|
| | | | Humane[b] hCYP11B2 | V79 11B1[d] hCYP11B1 | V79 11B2[e] hCYP11 B2 | humane[f] CYP17 | Humane[h] Typ19 |
| 1 | H | | 92 | 2395 | 28 | 40 | 5727 |
| 2 | 6-OMe | | 91 | 574 | 6 | [667] | 586 |
| 3 | 6- OH | | 88 | 715 | 23 | 65 | [61] |
| 4 | 6- Br | | 85 | 899 | 15 | 46 | [33] |
| 5 | 6-OEt | | 86 | 883 | 12 | 53 | 6639 |
| 8 | 6-CN | | 98 | 691 | 3 | [686] | [44] |
| 9 | 5-Cl-6-OMe | | 86 | 569 | 13 | 32 | 1805 |
| 10 | 5-Br-6-OMe | | 68 | 1531 | 33 | 38 | 9107 |
| 12 | 1,5-Cl-6-OMe | | 82 | 1545 | 28 | [233] | 970 |
| 13 | 7-OMe | | 46 | nd | nd | nd | nd |
| 14 | 1-Cl-7-OMe | | 85 | 771 | 29 | [27] | [65] |
| 15 | 3-OMe | | 38 | nd | nd | nd | nd |
| 16 | 6-COOMe | | 66 | 10505 | 73 | 12 | 1252 |
| 19 | X=CH, Y=N | | 40 | nd | nd | nd | nd |
| 20 | X=N, Y= CH | | 53 | nd | nd | nd | nd |
| 23 | H | 1-imidazolyl | 80 | 1052 | 38 | 13 | 2821 |
| 24 | 3-OMe | 1-imidazolyl | 86 | 5015 | 40 | 4 | 129 |
| 27 | H | 5-imidazolyl | 41 | 206 | nd | nd | nd |
| 29 | H | 5-oxazolyl | 16 | 805 | nd | nd | nd |
| 30 | 6-OMe | 5-pyrimidyl | 46 | nd | nd | nd | nd |
| Ketoconazol | - | | 36 | 224 | 81 | 40 | nd |

(fortgesetzt)

| Verbindung | R | Het | % Inhib.[a] | $IC_{50}$-Wert (nM)[c] | | % Inhub. a [$IC_{50}$ (nM)[g]] | $IC_{50}$ (nM)[g] [% Inhib.[a]] |
|---|---|---|---|---|---|---|---|
| | | | Humane[b] hCYP11B2 | V79 11B1[d] hCYP11B1 | V79 11B2[e] hCYP11 B2 | humane[f] CYP17 | Humane[h] Typ19 |
| Fadrozol | - | | 68 | 10 | 1 | 5 | 30 |

[a] Mittelwert aus 4 Bestimmungen, Standardabweichung < 10%. *S. pombe-Zellen,* die humane CYP11B2 exprimieren; Substrat Deoxycorticosteron, 100nM; Inhibitor, 500nM.
[c] Mittelwert aus 4 Bestimmungen, Standardabweichung < 20%.
[d] Hamsterfibroblasten, die humane CYP11B1 exprimieren; Substrat Deoxycorticosteron, 100nM.
[e] Hamsterfibroblasten, die humane CYP11B2 exprimieren; Substrat Deoxycorticosteron, 100nM.
[f] *E. coli,* die humane CYP17 exprimieren; 5 mg/ml Protein; Substrat Progesteron, 2,5 μM; Inhibitor 2,5 μM.
[g] Mittelwert aus 4 Bestimmungen, Standardabweichung < 5%.
[h] humane placentale CYP19, 1 mg/ml Protein; Substrat Androstenedion, 2,5 μM. (nd = nicht bestimmt)

Beispiel 6: Inhibition adrenaler CYP11B-Enzyme *in vitro* durch heteroaryl-substituierte 3,4-Dihydronaphthaline und Indane

Heteroaryl-substituierte 3,4-Dihydronaphthaline und Indane wurden wie in Beispiel 3 und 4 beschrieben als Inhibitoren getestet. Die Ergebnisse der Tests sind in Tab. 2 zusammengefasst.

Tab.2: Heteroaryl-substitutierte 3,4-Dihydronaphthaline und Indane: Inhibition adrenaler CYP11 B-Enzyme, CYP17 und CYP19 *in vitro.*

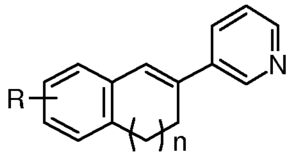

**32-35, 37-41, 45-46**

| | | | % Inhibition[a] | $IC_{50}$-Wert (nM)[c] | | % Inhib.[a] [$IC_{50}$ (nM)[g]] | $IC_{50}$ (nM)[g] [% Inhib.[a]] |
|---|---|---|---|---|---|---|---|
| Verbindung | n | R | Humane[b] hCYP11B2 | V79 11B1[d] hCYP11B1 | V79 11B2[e] hCYP11B2 | humane[f] CYP17 | Humane[h] CYP19 |
| 32 | 0 | H | 90 | 862 | 13,5 | 2 | [47] |
| 33 | 1 | H | 95 | 411 | 7 | 37 | 4,073 |
| 34 | 0 | 6-OMe | 66 | 5684 | 4 | 7 | [50] |
| 35 | 1 | 6-OMe | 97 | 213 | 2 | 62 | 814 |
| 37 | 1 | 7-OMe | 51 | nd | nd | nd | nd |
| 38 | 1 | 1-Me | 94 | 1268 | 7 | [1085] | 6045 |
| 39 | 1 | 1-Et | 81 | 2117 | 30 | 57 | 1507 |
| 40 | 1 | 3- Me | 84 | 503 | 5 | 27 | 1787 |
| 41 | 1 | 4- Me | 82 | 437 | 13 | 23 | 3551 |
| 45 | 1 | H | 34 | 638 | nd | nd | nd |
| 46 | 1 | H | 35 | 763 | nd | nd | nd |
| Ketoconazol | - | | 36 | 224 | 81 | 40 | nd |
| Fadrozol | - | | 68 | 10 | 1 | 5 | 30 |

[a] Mittelwert aus 4 Bestimmungen, Standardabweichung < 10%. S. pombe-Zellen, die humane CYP11B2 exprimieren; Substrat Deoxycorticosteron, 100nM; Inhibitor, 500nM.
[c] Mittelwert aus 4 Bestimmungen, Standardabweichung < 20%.
[d] Hamsterfibroblasten, die humane CYP11B1 exprimieren; Substrat Deoxycorticosteron, 100nM.
[e] Hamsterfibroblasten, die humane CYP11B2 exprimieren; Substrat Deoxycorticosteron, 100nM.
[f] *E.coli,* die humane CYP17 exprimieren; 5 mg/ml Protein; Substrat Progesteron, 2,5 $\mu$M; Inhibitor 2,5 $\mu$M.
[g] Mittelwert aus 4 Bestimmungen, Standardabweichung < 5%.
[h] humane placentale CYP19,
1 mg/ml Protein; Substrat Androstenedion, 2,5 $\mu$M. (nd = nicht bestimmt)

Beispiel 7: Inhibition von CYP-Enzymen *in vitro* durch die Referenzverbindungen Ketoconazol und Fadrozol

Ketocoanzol oder Fadrozol wurden wie in Beispiel 3 und 4 beschrieben als Inhibitoren getestet. Die Ergebnisse der Tests sind in Tab. 3 zusammengefasst.

Tab. 3: Ketoconazol und Fadrozol: Inhibition von adrenalen CYP11B-Enzymen, CYP17 und CYP19 *in vitro*

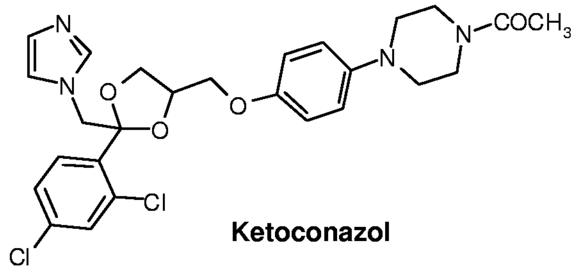

**Ketoconazol**

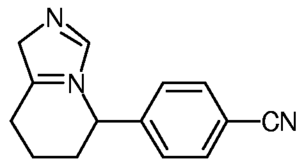

**Fadrozol**

| Verbindung | % Inhibition[a] | $IC_{50}$-Wert (nM)[c] | | % Inhibition $IC_{50}$-Wert (nM) | |
|---|---|---|---|---|---|
| | humane[b] hCYP11B2 | V79 11B1[d] hCYP11B1 | V79 11B2[e] hCYP11B2 | humane[f] CYP17 | humane[g] Typ19 |
| Ketoconazol | 36 | 224,4 | 80,5 | 40 | n.d. |
| Fadrozol | 68 | 9,7 | 1,0 | 7 | 29,5 |

[a] Mittelwert aus 4 Bestimmungen, Standardabweichung < 10 %;
[b] *S. pombe*-Zellen, die humane CYP11B2 exprimieren; Substrat Deoxycorticosteron, 100 nM; Inhibitor, 500 nM.
[c] Mittelwert aus 4 Bestimmungen, Standardabweichung < 20 %.
[d] Hamsterfibroblasten, die humane CYP11B1 exprimieren; Substrat Deoxycorticosteron, 100 nM.
[e] Hasterfibroblasten, die humane CYP11B2 exprimieren; Substrat Deoxycorticosteron, 100 nM.
[f] Mittelwert aus 4 Bestimmungen, Standardabweichung < 10 %; E.coli, de humane CYP17 exprimieren; 5 mg/ml Protein; Substrat Progesteron, 2,5 μM; Inhibitor, 2,5 μM.
[g] Mittelwert aus 4 Bestimmungen, Standardabweichung < 5 %; humane placentale CYP19, 1 mg/ml Protein; Substrat Testosteron, 2,5 μM; (n.d. = nicht bestimmt)

Beispiel 8: Test ausgewählter Verbindungen mit NCI-H295R-Zellen

Von den unter Bsp. 5 und 6 vorgestellten Verbindungen wurden einige am NCI-H295R-System untersucht. Zum Vergleich wurde Fadrozol als Referenz verwendet. Die erzielten exemplarischen Ergebnisse sind nicht direkt mit den $IC_{50}$-Werten und prozentualen Hemmwerten, die in V79-Zellen erzielt wurden, vergleichbar, da für die Hemmstoff-assays an NCI-H295R u.a. andere Testparameter sowie ein anderes Substrat verwendet wurden (Erläuterung siehe Tab. 4).
Im Vergleich mit Fadrozol konnte eine grobe Korrelation zwischen den beiden Testsystemen ermittelt werden, wobei die Verbindungen 1 und 2 die CYP11 B1 in deutlich geringerem Maß beeinflussen.

Tab. 4: Vergleich Hemmdaten NCI-H295R, V79 MZ

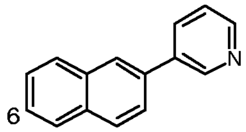

**1 = 6-H; 2 = 6-OMe**

| Verbindung | NCI-H295R | | | V7911B1 | V7911B2 |
|---|---|---|---|---|---|
| | CYP11B1 RSS *% Hemmung*[a] [$IC_{50}$ nM] | CYP11B2 B *% Hemmung*[b] [$IC_{50}$ nM] | CYP11B2 DOC *% Hemmung*[b] [$IC_{50}$nM] | Typ11B1 DOC [$IC_{50}$ nM][c] | CYP11B2 DOC [$IC_{50}$ nM][c] |
| Fadrozol | *89,8 ± 1,5* [28,3 ± 2,8] | *32,2 ± 6,1* [20870 ± 6268] | *89,6 ± 0,2* [18,7 ± 0,8] | [9] | [1] |
| 1 | *43,4 ± 4,7* [3449 ± 273] | *45,6 ± 2,2* [2111± 124] | *82,9 ± 0,9* [163,9 ± 21,5] | [2395] | [28,4] |

(fortgesetzt)

| Verbindung | NCI-H295R CYP11B1 RSS % Hemmung[a] [$IC_{50}$ nM] | NCI-H295R CYP11B2 B % Hemmung[b] [$IC_{50}$ nM] | NCI-H295R CYP11B2 DOC % Hemmung[b] [$IC_{50}$nM] | V7911B1 Typ11B1 DOC [$IC_{50}$ nM][c] | V7911B2 CYP11B2 DOC [$IC_{50}$ nM][c] |
|---|---|---|---|---|---|
| 2 | *48,5 ± 2,9* [2950 ± 54,3] | *49,5 ± 2,4* [558,5 ± 118,4] | *81,4 ± 6,6* [100,1 ± 7,6] | [574] | [6,2] |

[a]: Hemmung der CYP11B1 Aktivität in NCI-H295R, Inhibitorkonzentration 2,5 µM, [$^3$H]-Deoxycortisol (RSS, 500 nM); Quantifizierung der Produkte nach HPLC Trennung

[b]: Hemmung der CYP11B2 Aktivität in NCI-H295R, Inhibitorkonzentration 2,5 µM, [$^3$H]-Corticosteron (B, 500 nM) oder [$^{14}$C]-Deoxycorticosteron (DOC, 500 nM); Quantifizierung der Produkte nach HPTLC Trennung mittels Phosphoimager System

[c]: Bestimmung von $IC_{50}$ Werten für CYP11B1 und CYP11B2 in V79 MZh11B1 and V79 MZh11B2, [$^{14}$C]- Deoxycorticosteron (DOC, 100 nM); Quantifizierung der Produkte nach HPLC Trennung mittels Phosphoimager System. Zur Bestimmung von $IC_{50}$ Werten wurden die Verbindungen in mindestens 3 verschiedenen Konzentrationen getestet.

n. t.: nicht getestet, RSS = Deoxycortisol; B = Corticosteron; DOC = Deoxycorticosteron, dargestellt sind MW (± S. D.) von mindestens drei unabhängigen Tests.

Zur Untersuchung der Wirkung verschiedener Hemmstoffe auf NCI-H295R wurde ein Testverfahren im 24-well-Format entwickelt. Zur Hemmstofftestung wurde zunächst eine einstündige Prä-Inkubation durchgeführt, bevor die Enzymreaktionen durch Substratzugabe (500 nM) gestartet wurden.

A) Aussaat: Anzucht und Passagieren der Zelllinien erfolgte, bis ein konfluenter Zellrasen ausgebildet worden war. Durch Trypsinbehandlung wurde das Zellm aterial von mindestens zwei Kulturschalen gewonnen und die Zellzahl mit Hilfe eines CASY TT-Zellzählgerätes (150 µl-Kapillare) bestimmt. Durch Verdünnen der Zellsuspension mit DMEM:Ham's F12 wurde eine Zelldichte von 1 x $10^6$ Zellen/ml eingestellt. Von der so erhaltenen Zellsuspensionen wurde jeweils 1 ml auf ein well einer 24-well-Platte gegeben, so dass jedes well mit 1 x $10^6$ Zellen beschichtet war. Mit dem Zellmaterial von zwei konfluent bewachsenen Kulturschalen konnten zwei 24-wellPlatten beschichtet werden. Nach 24 Stunden waren die Zellen angewachsen und nach einer weiteren 24-stündigen Stimulationsphase mit Kaliumionenhaltiger Lösung (Endkonzentration: 20 mM KCl) zum Test einsetzbar.

B) Substratlösungen: Für die Testung des Einflusses der Hemmstoffe auf CYP11B1 wurde als Substrat Tritium-markiertes Deoxycortisol eingesetzt ([$^3$H]-RSS). Zur Herstellung der Substratstammlösung wurden 60 µl [$^3$H(G)] -Deoxycortisol (3 Ci/mmol, 1 mCi/ml) in Ethanol (Hartmann Analytik, Braunschweig, Deutschland) mit 140 µl Ethanol verdünnt. Von dieser Lösung wurden 2,5 µl pro Probe eingesetzt, was bei einem Testvolumen von 500 µl einer Endkonzentration von 500 nM im Test entsprach.

Bei den Substratlösungen für die Untersuchungen an CYP11B2 bestand die Corticosteron-Substratlösung (Endkonzentration im Test 500 nM) aus 38,4 µl [1,2-$^3$H (N)]-Corticosteron (1 mCi/ml, 76,5 Ci/mmol; NEN-Perkin-Elmer) in Ethanol, 39,0 µl unmarkierter Corticosteronlösung (0,5 mM in Ethanol) und 122,6 µl Ethanol. Deoxycorticosteron, welches ebenfalls in einer Endkonzentration von 500 nM eingesetzt wurde, setzte sich zusammen aus 18 µl [$^{14}$C] -markiertem Deoxycorticosteron (60,0 mCi/mmol; 0,5 nCi/µl) in Ethanol im Gemisch mit 54 µl unmarkierter Substanz (0,5 mM in Ethanol) und 228 µl Ethanol.

C) Hemmstofflösungen: Die für die Bestimmung der $IC_{50}$-Werte erforderlichen Konzentrationen wurden durch 1:40-Verdünnen der Stammlösung (10 mM) mit Ethanol eingestellt. Von dieser Lösung wurden den Proben jeweils 5 µl zugesetzt.

D) Durchführung des Tests:

*Prä-Inkubation:* Das vorhandene Medium wurde abgesaugt und durch 450 µl DMEM:Ham's F12 ersetzt, in dem der Hemmstoff in der entsprechenden Konzentration zugesetzt war (Endkonzentration des Hemmstoffes im Endvolumen (500 µl) des Tests: 2,5 µM), danach wurde 1 h prä-inkubiert.

*Teststart:* Die Reaktion wurde durch Zugabe von 50 µl DMEM: Ham's F12, 2,5 µl des jeweiligen Substratmixes (Endkonzentration des Substrats: 0,5 µM) enthaltend, eingeleitet.

Die 24-well-Platte wurde dann bei 37 °C und 5 % $CO_2$ im $CO_2$-Inkubator aufbewahrt. Die Inkubationszeit betrug 3 Stunden bei Verwendung von Deoxycorticosteron als Substrat, bei Corticosteron 24 Stunden und bei Deoxycortisol 48 Stunden. *Teststop:* Nach Ablauf der Inkubationszeiten wurde nach kurzem Schwenken der Inhalt der wells möglichst

quantitativ entnommen und durch Mischen mit 1000 µl Dichlormethan in einem 2ml-Eppendorfgefäß inaktiviert. Nach 10-minütigem Schütteln wurde zur Phasentrennung zentrifugiert und die obere organische Phase in ein 1,5ml-Eppendorf-Gefäß überführt.

Nach Abdampfen des Lösungsmittel über Nacht unter dem Abzug wurde der Rückstand in 10 µl Chloroform aufgenommen und in der Mitte der Aufkonzentrierungszone einer HPTLC-Platte aufgetragen. Die Steroide wurden durch zweifaches Entwickeln mit einem Fließmittel, das sich aus Chloroform, Methanol und Wasser in Verhältnis 300:20:1 zusammensetzte, aufgetrennt. Zur Detektion der Steroide auf der TLC wurde nach zwei Tagen der strahlenexponierte Film im Phosphoimager FLA 3000 gescannt.

Im Falle von Deoxycortisol als Substrat erfolgte die Auftrennung nach Rekonstitution in 50 µl Methanol, dem als interne Standards unmarkiertes Deoxycortisol, Cortisol und Cortison zugesetzt waren, per HPLC über eine RP18-Säule mit dem Fließmittel Methanol:Wasser 1:1 und einer Flussrate von 0,25 ml/min, die Detektion erfolgte mit Hilfe eines Berthold Radiomonitors 509.

Nach Gleichung 4 wurde die Konversion für das Substrat Deoxycortisol nach HPLC-Trennung berechnet:

Gleichung 4:

$$\%P = \frac{A_{Cortisol} + A_{Cortison}}{\left[A_{Cortison} + A_{Cortisol} + A_{RSS}\right]} \times 100$$

| | |
|---|---|
| %P | Konversion (Anteil des Produktes an Gesamtsteroid in %) |
| *A* | Area (Fläche) in [Units ? sec] |
| $A_{Cortisol}$ | Fläche für Cortisol |
| $A_{Cortison}$ | Fläche für Cortison |
| $A_{RSS}$ | Fläche für Deoxycortisol (RSS) |

Für das Substrat Deoxycorticosteron ergab sich die Konversion entsprechend Gleichung 3 (Bsp. 4B).

Für das Substrat Corticosteron galt Gleichung 5:

Gleichung 5:

$$\%P = \frac{\left[PSL_{18OHB} + PSL_{Aldo}\right] - 2 \times PSL_{HG}}{\left[PSL_{B} + PSL_{18OHB} + PSL_{Aldo}\right] - 3 \times PSL_{HG}} \times 100$$

| | |
|---|---|
| %*P* | Konversion (Anteil des Produktes an Gesamtsteroid in % |
| *PSL* | Phospho Stimulated Lumineszence (Lumineszenzwert) |
| $PSL_B$ | PSL für Corticosteron (B) |
| $PSL_{18OHB}$ | PSL für 18-Hydroxycorticosteron (180HB) |
| $PSL_{Aldo}$ | PSL für Aldosteron |
| $PSL_{HG}$ | PSL des Hintergrundes |

Die prozentuale Hemmung, die durch einen Hemmstoff in der jeweils eingesetzten Konzentration verursacht wurde, errechnete sich nach Gleichung 2 (Bsp. 4A).

Die Bestimmung des $IC_{50}$-Wertes erfolgte wie in Bsp. 4B beschrieben.

Beispiel 9: Quantifizierung von Steroiden im Überstand einer Zellkultur von NCl-H295R-Zellen

H295R-Zellen (s. Bsp. 8) wurden mit einer Zelldichte von 1 x $10^6$ Zellen/ml in einem Volumen von jeweils 1 ml/well in einer 24-well-Platte subkultiviert und 48 Stunden lang inkubiert. Das Medium wurde danach ersetzt durch 500 µl Ultroser SF-freies DMEM:Ham's F12, in dem der zu testende Hemmstoff in der entsprechenden Konzentration und 1% Etahnol enthalten waren. Kontrollinkubationen enthielten lediglich 1% Ethanol. Nach Inkubation bei 37°C und 5% $CO_2$ für 6 Stunden wurde der Überstand entfernt und bis zur weiteren Analyse eingefroren. Die Inhibitoraktivitäten wurden anhand der Differenz zwischen den Steroidkonzentrationen in An-oder Abwesenheit der verwendeten Testsubstanzen kalkuliert. Die Aldosteronkonzentration wurde entsprechend der Herstellervorschrift mit einem RIA Kit bestimmt (DRG, Marburg, Deutschland). Zur Bestimmung der Androgene (DHEA und Androstendion) sowie von Cortisol wurden

spezifische ELISA-Kits (ibl-Hamburg, Hamburg, Deutschland) bzw. der Cortisol-ELISA-Kit von Cayman Chemical (Ann Arbor, USA) entsprechend der Herstellerangaben verwendet.

Zur Messung des Gesamtproteingehalts wurden die Zellen mit PBS gewaschen, in einem Lysepuffer (8M Harnstoff, 4% (w/v) CHAPS) solubilisiert und bei -70°C eingefroren. Nach drei Gefrier-Tau-Zyklen waren die Zellen lysiert und ihr Proteingehalt wurde mit Hilfe der Methode von Bradford bestimmt. Die Resultate der Steroidbestimmungen wurden in pg/mg Zellproteine für Aldosteron und ng/mg Zellproteine für Androgene und Cortisol ausgedrückt.

Innnerhalb von 6 Stunden konnte eine deutliche konzentrationsabhängige Abnahme der Aldosteronsekretion bei Einsatz von Verbindung 2 und Ketoconazol festgestellt werden (Fig. 1B und 1C). Fadrozol reduzierte die Bildung von Aldosteron mit einem 9-fach niedrigeren $IC_{50}$ gegenüber der Cortisol-Bildung (39,4 nM versus 363,8 nM; Fig. 1A, Tab. 5), während die Bildung adrenaler Androgene weniger stark reduziert wurde ($IC_{50}$ >3900 nM bei Androstendion und 150 nM bei DHEA). Ketoconazol zeigte $IC_{50}$-Werte von 59,6 nM und 51,8 nM für die Inhibition der Androstendion- und DHEA-Produktion. Die Verbindung 2 stellte sich als potenter Supressor der Bildung von Cortisol und von adrenalen Androgenen heraus ($IC_{50}$-Werte <1 1 nM), während die Aldosteronbildung nur schwach gehemmt wurde (Fig. 1C, Tab. 5).

Tab. 5: Inhibition der Steroidproduktion in H295R-Zellen, ermittelt anhand der Steroidkonzentration im Überstand

| Verbindung | % Hemmung der Aldosteronbildung[a] [$IC_{50}$ nM] | % Hemmung der Cortisolbildung[a] [$IC_{50}$ nM] | % Hemmung der Androstendionbildung[a] [$IC_{50}$ nM] | % Hemmung der DHEA-Bildung[a] [$IC_{50}$ nM] |
|---|---|---|---|---|
| Fadrozol | 72,9 ± 1,3 [39,4] | 57,8 ± 2,3 [363,8] | 45,0 ± 0,9 [3976] | 66,5 ± 0,9 [150,1] |
| 2 | 56,4 ± 3,5 [962,7] | 78,6 ± 5,4 [< 1] | 84,6 ± 2,0 [< 1] | 84,5 ± 2,0 [< 1] |
| Ketoconazol | 33,3 ± 3,3 [13105] | 88,1 ± 1,0 [154,6] | 89,2 ± 1,2 [59,6] | 94,4 ± 0,6 [51,8] |

[a] Quantifizierung nach 6 h Inkubation mit einer Inhibitorkonzentration von 2,5 µM. Verwendet wurde der Überstand der Zellkulturen, die Bestimmung erfolgte durch spezifische Immunoassays. Mittelwerte (± SD) aus mindestens drei unabhängigen Experimenten.

## Patentansprüche

1. Verwendung einer Verbindung mit der Struktur der Formel (Ia)

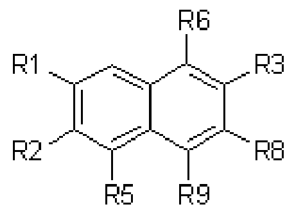

**(Ia)**

worin

$R^1$ oder $R^2$ Wasserstoff ist und der andere der Substituenten $R^1$ oder $R^2$ ausgewählt ist aus H, Fluor, Chlor, Brom, CN, $COOR^{11}$, Hydroxy, $C_{1-3}$-Alkyl und $C_{1-3}$-Alkoxy;
$R^3$ ausgewählt ist aus Oxazolyl-, Pyridyl-, Imidazolyl- und Pyrimidylresten;
$R^5$ und $R^6$ unabhängig voneinander ausgewählt sind aus H, Fluor, Chlor, Brom;
$R^8$ und $R^9$ H sind;
$R^{11}$ ausgewählt ist aus H und $C_{1-3}$-Alkyl, das geradkettig, verzweigt oder cyclisch, gesättigt oder ungesättigt sein kann;
oder eines pharmazeutisch geeigneten Salzes derselben
zur Herstellung eines Medikaments zur Behandlung von Hyperaldosteronismus, Herzinsuffizienz und Myocardfibrose.

2. Verwendung nach Anspruch 1, wobei $R^3$ ausgewählt ist aus 3- und 4-Pyridyl, 1-Imidazolyl, 4-Imidazolyl und 5-Pyrimidyl.

3. Verwendung nach Anspruch 2, wobei $R^3$ 3-Pyridyl ist.

4. Verwendung nach einem oder mehreren der Ansprüche 1 bis 3, wobei die Verbindung der Formel (Ia) ausgewählt ist aus 3-(2-Naphthyl)pyridin, 3-(6-Methoxy-2-naphthyl)pyridin, 3-(6-Brom-2-naphthyl)pyridin, 3-(6-Ethoxy-2-naphthyl)pyridin, 6-Pyridin-3-yl-2-naphthonitril, 3-(1,5-Dichlor-6-methoxy-2-naphthyl)pyridin und Methyl-6-pyridin-3-yl-2-naphthoat.

5. Pharmazeutische Zusammensetzung, enthaltend eine Verbindung mit der Struktur der Formel (Ia)

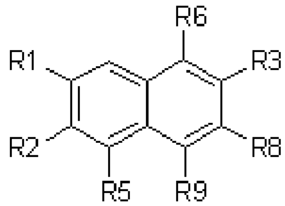

(Ia)

worin
$R^1$, $R^2$, $R^3$, $R^5$, $R^6$, $R^8$, $R^9$ und $R^{11}$ die in Anspruch 1 angegebene Bedeutung aufweisen, oder ein pharmazeutisch geeignetes Salz derselben, vorausgesetzt dass

(a) $R^3$ nicht 1-Imidazolyl ist;
(b) wenn $R^1$, $R^2$, $R^5$ und $R^6$ H-Atome sind, dann $R^3$ nicht 3- oder 4-Pyridyl ist; und
(c) wenn $R^2$, $R^5$, und $R^6$ H-Atome sind und $R^1$ COOH ist, dann $R^3$ nicht 4-Pyridyl ist.

6. Pharmazeutische Zusammensetzung gemäß Anspruch 5, wobei die Variable $R^3$ die in Anspruch 2 bis 3 angegebene Bedeutung hat.

7. Pharmazeutische Zusammensetzung nach Anspruch 5 oder 6, wobei die pharmazeutische Zusammensetzung vorzugsweise eine der in Anspruch 4 definierten Verbindungen enthält.

8. Pharmazeutische Zusammensetzung gemäß einem oder mehreren Ansprüche 5 bis 7, die zur Therapie von Hyperaldosteronismus, Herzinsuffizienz und myokardialer Fibrose bei Säugetieren und insbesondere Menschen geeignet ist.

9. Verbindung der Formel (Ia)

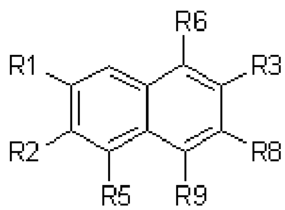

(Ia)

worin
$R^1$, $R^2$, $R^3$, $R^5$, $R^6$, $R^8$, $R^9$ und $R^{11}$ die in Anspruch 1 angegebene Bedeutung aufweisen, oder deren pharmazeutisch geeignete Salze, vorausgesetzt dass

(a) $R^3$ nicht 1-Imidazolyl ist;
(b) wenn $R^1$, $R^5$ und $R^6$ H-Atome sind, und $R^2$ H oder Methoxy ist, dann $R^3$ nicht Pyridyl, Imidazolyl oder Oxazolyl

ist;
(c) wenn $R^2$, $R^5$ und $R^6$ H-Atome sind und $R^1$ COOH ist, dann $R^3$ nicht 4-Pyridyl ist;
(d) wenn $R^1$, $R^2$, $R^4$, $R^5$ und $R^6$ H-Atome sind, dann $R^3$ nicht 3- oder 4-Pyridyl ist; und
(e) die Verbindung nicht 5-(Naphtalen-2-yl)oxazol, 4-Naphtalen-2-yl-pyrimidin, 4(5)-(2-Naphtyl)-1H-imidazol oder 2-(2-Naphtyl)-1H-imidazol ist.

**10.** Verbindung nach Anspruch 9, wobei die Variable $R^3$ die in Anspruch 2 bis 3 angegebene Bedeutung hat.

**11.** Verbindungen nach Anspruch 9 oder 10, wobei die Verbindung die in Anspruch 4 definierten Verbindungen sind.

**12.** Verfahren zur Synthese der Verbindungen gemäß Anspruch 9, umfassend die Suzuki-Kupplung der Verbindung (Ia) worin $R^3$ ein Halogenatom oder Pseudohalogenid ist, und alle anderen Variablen, die in Anspruch 9 angegebene Bedeutung haben mit der Verbindung (II)

$R^3$-$B(OH)_2$ **II;**

wobei $R^3$ die in Anspruch 9 angegebene Bedeutung hat und wobei funktionelle Gruppen in $R^1$-$R^9$ optional mit geeigneten Schutzgruppen versehen sein können.

**13.** Verfahren nach Anspruch 12, wobei $R^3$ Br oder OTf ist.

**14.** Verwendung nach Anspruch 1 bis 4, wobei die genannten Verbindungen

(i) als Einzelverbindungen oder
(ii) als Bestandteil von Mischungen, enthaltend eine oder eine Kombination von zwei und mehr der unter Anspruch 1 bis 4 genannten Verbindungen oder
(iii) in Kombination mit weiteren pharmakologisch aktiven Verbindungen eingesetzt werden.

## Claims

**1.** Use of a compound having a structure of formula (Ia)

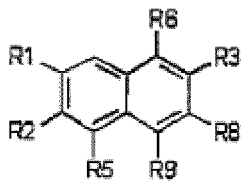

**(Ia)**

wherein

$R^1$ or $R^2$ is hydrogen and the other of substituents $R^1$ or $R^2$ is selected from H, fluorine, chlorine, bromine, CN, $COOR^{11}$, hydroxy, $C_{1-3}$ alkyl and $C_{1-3}$ alkoxy;
$R^3$ is selected from oxazolyl, pyridyl, imidazolyl and pyrimidyl residues;
$R^5$ and $R^6$ are independently selected from H, fluorine, chlorine, bromine;
$R^8$ and $R^9$ are H;
$R^{11}$ is selected from H and $C_{1-3}$ alkyl that may be straight-chain, branched-chain or cyclic, saturated or unsaturated;
or a pharmaceutically acceptable salt thereof
for preparing a medicament for treating hyperaldosteronism, heart failure, and myocardial fibrosis.

**2.** The use according to claim 1, wherein $R^3$ is selected from 3- and 4-pyridyl, 1-imidazolyl, 4-imidazolyl and 5-pyrimidyl.

**3.** The use according to claim 2, wherein $R^3$ is 3-pyridyl.

**4.** The use according to one or more of claims 1 to 3, wherein the compound of formula (Ia) is selected from 3-(2-naphthyl)pyridine; 3-(6-methoxy-2-naphthyl)pyridine; 3-(6-bromo-2-naphthyl)pyridine; 3-(6-ethoxy-2-naphthyl)pyridine; 6-pyridin-3-yl-2-naphthonitrile; 3-(1,5-dichloro-6-methoxy-2-naphthyl)pyridine; and methyl-6-pyridin-3-yl-2-naphthoate.

**5.** A pharmaceutical composition containing a compound having a structure of formula (Ia)

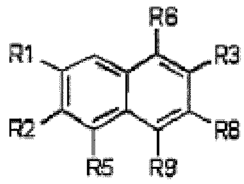

(Ia)

wherein
$R^1$, $R^2$, $R^3$, $R^5$, $R^6$, $R^8$, $R^9$ and $R^{11}$ are as defined in claim 1, or a pharmaceutically acceptable salt thereof, with the proviso that:

(a) $R^3$ is not 1-imidazolyl;
(b) if $R^1$, $R^2$, $R^5$ and $R^6$ are H atoms, then $R^3$ is not 3- or 4-pyridyl; and
(c) if $R^2$, $R^5$ and $R^6$ are H atoms and $R^1$ is COOH, then $R^3$ is not 4-pyridyl.

**6.** The pharmaceutical composition according to claim 5, wherein the variable $R^3$ is as defined in claims 2 to 3.

**7.** The pharmaceutical composition according to claim 5 or 6, wherein said pharmaceutical composition preferably contains one of the compounds as defined in claim 4.

**8.** The pharmaceutical composition according to one or more of claims 5 to 7, which is suitable for the therapy of hyperaldosteronism, heart failure and myocardial fibrosis in mammals and especially in humans.

**9.** A compound of formula (Ia)

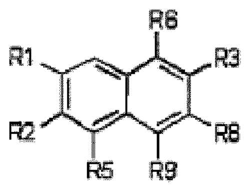

(Ia)

wherein
$R^1$, $R^2$, $R^3$, $R^5$, $R^6$, $R^8$, $R^9$ and $R^{11}$ are as defined in claim 1, or pharmaceutically acceptable salts thereof, with the proviso that:

(a) $R^3$ is not 1-imidazolyl;
(b) if $R^1$, $R^5$ and $R^6$ are H atoms and $R^2$ is H or methoxy, then $R^3$ is not pyridyl, imidazolyl or oxazolyl;
(c) if $R^2$, $R^5$ and $R^6$ are H atoms and $R^1$ is COOH, then $R^3$ is not 4-pyridyl;
(d) if $R^1$, $R^2$, $R^4$, $R^5$ and $R^6$ are H atoms, then $R^3$ is not 3- or 4-pyridyl; and
(e) the compound is not 5-(naphthalene-2-yl)oxazole, 4-naphthalene-2-ylpyrimidine, 4(5)-(2-naphthyl)-1H-imidazole, or 2-(2-naphthyl)-1H-imidazole.

**10.** The compound according to claim 9, wherein the variable $R^3$ is as defined in claims 2 to 3.

**11.** The compound according to claim 9 or 10, wherein the compound are the compounds as defined in claim 4.

**12.** A process for the synthesis of the compounds according to claim 9, comprising the Suzuki coupling of compound (Ia) wherein $R^3$ is a halogen atom or pseudohalide, and all other variables are as defined in claim 9, with compound (II)

R3-$B(OH)_2$ **II** ;

wherein $R^3$ has the meaning as stated in claim 9, and wherein functional groups in $R^1$-$R^9$ may optionally be provided with appropriate protecting groups.

**13.** The process according to claim 12, wherein $R^3$ is Br or OTf.

**14.** The use according to claim 1 to 4, wherein the mentioned compounds are employed

(i) as single compounds; or
(ii) as components of mixtures containing one or a combination of two or more of the compounds as mentioned in claims 1 to 4; or
(iii) in combination with further pharmacologically active compounds.

**Revendications**

**1.** Utilisation d'un composé ayant la structure de formule (Ia)

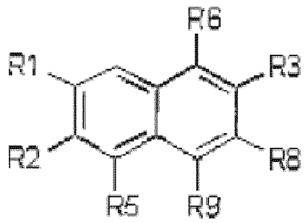

**(Ia)**

dans laquelle $R^1$ ou $R^2$ est un atome d'hydrogène, et l'autre des substituants $R^1$ ou $R^2$ est choisi parmi H, le fluor, le chlore, le brome, CN, $COOR^{11}$, un groupe hydroxy, alkyle en $C_{1-3}$ et alcoxy en $C_{1-3}$ ;
$R^3$ est choisi parmi les groupes oxazolyle, pyridyle, imidazolyle et pyrimidyle;
$R^5$ et $R^6$ sont choisis chacun indépendamment de l'autre parmi H, le fluor, le chlore, le brome ;
$R^8$ et $R^9$ sont H ;
$R^{11}$ est choisi parmi H et un groupe alkyle en $C_{1-3}$, qui peut être à chaîne linéaire, ramifiée ou cyclique, et être saturé ou insaturé;
ou d'un sel pharmaceutiquement acceptable de ce composé,
pour la fabrication d'un médicament destiné au traitement de l'hyperaldostéronisme, de l'insuffisance cardiaque et de la fibrose myocardique.

**2.** Utilisation selon la revendication 1, dans laquelle $R^3$ est choisi parmi les groupes 3- et 4-pyridyle, 1-imidazolyle, 4-imidazolyle et 5-pyrimidyle.

**3.** Utilisation selon la revendication 2, dans laquelle $R^3$ est le groupe 3-pyridyle.

**4.** Utilisation selon une ou plusieurs des revendications 1 à 3, pour laquelle le composé de formule (Ia) est choisi parmi la 3-(2-naphtyl)pyridine, la 3-(6-méthoxy-2-naphtyl)pyridine, la 3-(6-bromo-2-naphtyl)pyridine, la 3-(6-éthoxy-2-naphtyl)pyridine, le 6-pyridin-3-yl-2-naphtonitrile, la 3-(1,5-dichloro-6-méthoxy-2-naphtyl)pyridine et le 6-pyridin-3-yl-2-naphtoate de méthyle.

**5.** Composition pharmaceutique contenant un composé ayant la structure de formule (Ia)

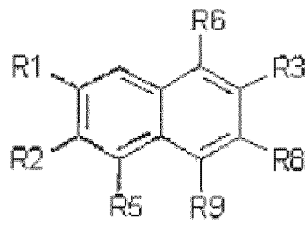

(Ia)

dans laquelle
$R^1$, $R^2$, $R^3$, $R^5$, $R^6$, $R^8$, $R^9$ et $R^{10}$ ont les significations données dans la revendication 1, ou un sel pharmaceutiquement acceptable de ce composé, à la condition que

(a) $R^3$ ne soit pas le groupe 1-imidazolyle;
(b) quand $R^1$, $R^2$, $R^5$ et $R^6$ sont des atomes de H, alors $R^3$ ne soit pas un groupe 3- ou 4-pyridyle ; et
(c) quand $R^2$, $R^5$ et $R^6$ sont des atomes de H et que $R^1$ est COOH, alors $R^3$ ne soit pas le groupe 4-pyridyle.

**6.** Composition pharmaceutique selon la revendication 5, dans laquelle la variable $R^3$ a la signification donnée dans les revendications 2 à 3.

**7.** Composition pharmaceutique selon la revendication 5 ou 6, la composition pharmaceutique contenant de préférence l'un des composés définis dans la revendication 4.

**8.** Composition pharmaceutique selon une ou plusieurs des revendications 5 à 7, qui convient à la thérapie de l'hyperaldostéronisme, de l'insuffisance cardiaque et de la fibrose myocardique chez les mammifères, et en particulier chez les humains.

**9.** Composé de formule (Ia)

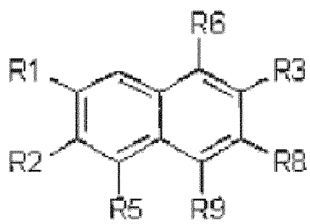

(Ia)

dans laquelle
$R^1$, $R^2$, $R^3$, $R^5$, $R^6$, $R^8$, $R^9$ et $R^{11}$ ont les significations données dans la revendication 1, ou sels pharmaceutiquement acceptables de ce composé, à la condition que

(a) $R^3$ ne soit pas le groupe 1-imidazolyle;
(b) quand $R^1$, $R^5$ et $R^6$ sont des atomes de H et que $R^2$ est H ou le groupe méthoxy, alors $R^3$ ne soit pas un groupe pyridyle, imidazolyle ou oxazolyle ;
(c) quand $R^2$, $R^5$ et $R^6$ sont des atomes H et que $R^1$ est COOH, alors $R^3$ ne soit pas un groupe 4-pyridyle;
(d) quand $R^1$, $R^2$, $R^4$, $R^5$ et $R^6$ sont des atomes de H, alors $R^3$ ne soit pas un groupe 3-ou 4-pyridyle; et
(e) le composé ne soit pas le 5-naphtalén-2-yl)oxazole, la 4-naphtalén-2-yl-pyrimidine, le 4(5)-(2-naphtyl)-1H-imidazole ou le 2-(2-natphyl)-1H-imidazole.

**10.** Composé selon la revendication 9, dans lequel la variable $R^3$ a la signification donnée dans les revendications 2 à 3.

**11.** Composés selon la revendication 9 ou 10, le composé étant les composés définis dans la revendication 4.

**12.** Procédé de synthèse des composés selon la revendication 9, comprenant le couplage de Suzuki du composé (Ia), dans lequel $R^3$ est un atome d'halogène ou un pseudohalogénure, et toutes les autres variables ont les significations données dans la revendication 9, avec le composé (II)

$$R^3\text{-}B(OH)_2 \qquad (II),$$

$R^3$ ayant les significations données dans la revendication 9, et les groupes fonctionnels de $R^1$-$R^9$ pouvant en option être pourvus de groupes protecteurs appropriés.

**13.** Procédé selon la revendication 12, dans lequel $R^3$ est Br ou OTf.

**14.** Utilisation selon la revendication 1 à 4, pour laquelle les composés mentionnés sont utilisés

(i) en tant que composés individuels, ou
(ii) en tant que constituant de mélanges contenant un composé mentionné dans les revendications 1 à 4, ou une combinaison de deux ou plus de ceux-ci, ou
(iii) en association avec d'autres composés pharmacologiquement actifs.

Fig.1A

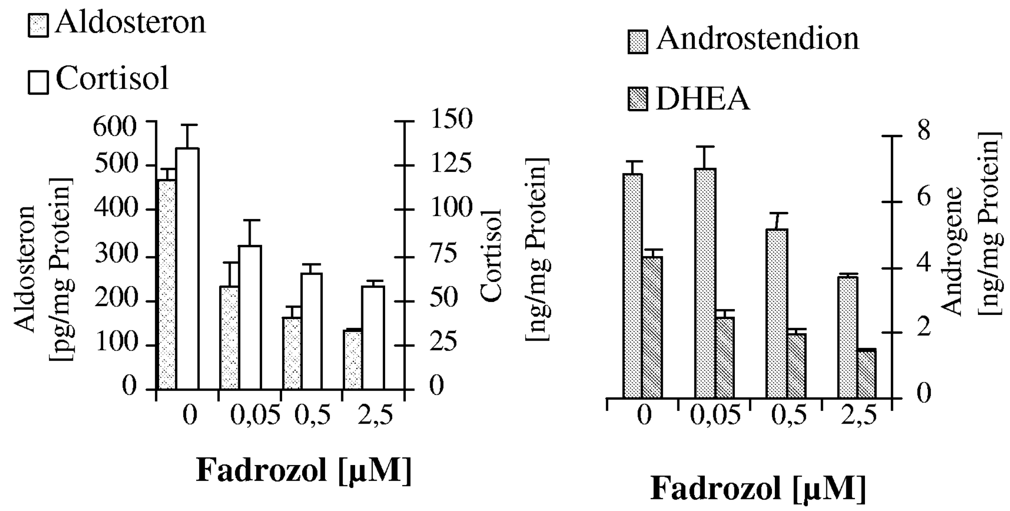


Fig.1B

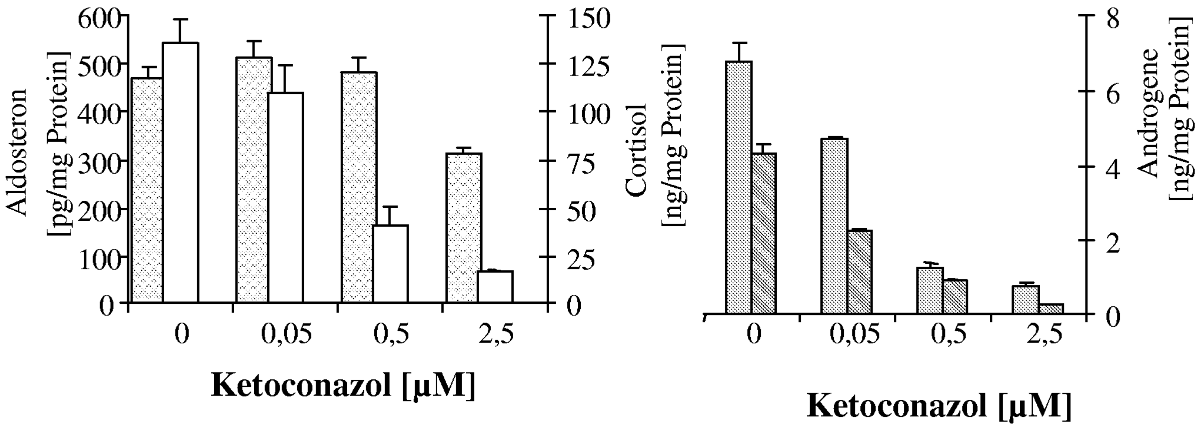


Fig.1C

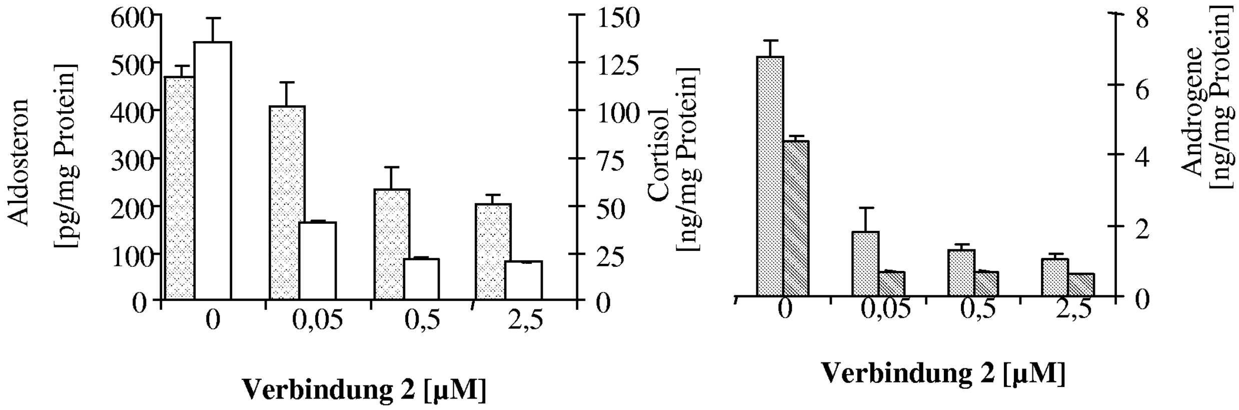

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1461333 A
- US 20020013303 A
- US 6150347 A
- US 6608047 B
- WO 0134132 A
- WO 9640255 A
- US 20020123485 A
- US 20030220312 A
- US 20030220310 A
- WO 0176574 A
- US 3165525 A
- US 3098077 A
- WO 03051805 A


### In der Beschreibung aufgeführte Nicht-Patentliteratur


- **NIEMAN, L. K.** *Pituitary,* 2002, vol. 5, 77-82
- **HEFFELFINGER et al.** *Dev. Psychopathol.,* 2001, vol. 13, 491-513
- **BOLLI et al.** *N. Engl. J. Med.,* 1984, vol. 310, 746-750
- **SHAPIRO et al.** *J. Clin. Endocrinol. Metab.,* 1991, vol. 72, 444-454
- **SCHULTES ; FEHM.** *Der Internist,* 2004, vol. 9, 983-993
- **PHILLIPS et al.** *J. Clin. Endocrinol. Metab.,* 1998, vol. 83, 757-760
- **DEFRONZO et al.** *Diabetes Rev.,* 1997, vol. 5, 177-269
- **STEWART ; KROZOWSKI.** *Vitam. Horm.,* 1999, vol. 57, 249-324
- **ALBERTS et al.** *Diabetologica,* 2002, vol. 45, 1528-1532
- **WEBER, K.T. ; BRILLA, C.G.** *Circulation,* 1991, vol. 83, 1849-1865
- **KAWAMOTO, T. et al.** *Proc. Natl. Acad. Sci. USA,* 1992, vol. 89, 1458-1462
- **BRILLA, C. G.** *Herz,* 2000, vol. 25, 299-306
- **YOUNG, M. ; FUNDER, J.W.** *Trends Endocrinol. Metab.,* 2000, vol. 11, 224-226
- **BRILLA, C. G.** *Cardiovasc. Res.,* 2000, vol. 47, 1-3
- **LIJNEN, P. ; PETROV, V.** *J. Mol. Cell. Cardiol.,* 2000, vol. 32, 865-879
- **PITT, B. et al.** *New Engl. J. Med.,* 1999, vol. 341, 709-717
- **KULBERTUS, H.** *Rev. Med. Liege,* 1999, vol. 54, 770-772
- **PITT, B. et al.** *New Eng. J. Med.,* 1999, vol. 341, 709-717
- **MACFADYEN, R. J. et al.** *Cardiovasc. Res.,* 1997, vol. 35, 30-34
- **SOBERMAN, J.E. ; WEBER, K.T.** *Curr. Hypertens. Rep.,* 2000, vol. 2, 451-456
- **LOSERT, W. et al.** *Drug Res.,* 1986, vol. 36, 1583-1600
- **NICKISCH, K. et al.** *J Med Chem,* 1987, vol. 30 (8), 1403-1409
- **NICKISCH, K. et al.** *J. Med. Chem.,* 1991, vol. 34, 2464-2468
- **AGARWAL, M. K. ; LAZAR, G.** *Renal Physiol. Biochem.,* 1991, vol. 14, 217-223
- **WEINDEL, K. et al.** *Arzneimittelforschung,* 1991, vol. 41 (9), 946-949
- *Eplerenone's Heart Failure Efficacy and Survival Study,* 2003
- **PITT., B. et al.** *N. Eng. J. Med.,* 2003, vol. 348, 1390-1382
- **HARTMANN, R. et al.** *Eur. J. Med. Chem.,* 2003, vol. 38, 363-366
- **EHMER, P. et al.** *J. Steroid Biochem. Mol. Biol.,* 2002, vol. 81, 173-179
- **TAYMANS, S. E. et al.** *J. Clin. Endocrinol. Metab.,* 1998, vol. 83, 1033-1036
- **DEMERS, L.M. et al.** *J. Clin. Endocrinol. Metabol.,* 1990, vol. 70, 1162-1166
- **HARTMANN, R. W. et al.** *Arch. Pharm. Pharm. Med.,* 1996, vol. 339, 251-61
- **HARTMANN, R.W. et al.** *Arch. Pharm. Pharm. Med. Chem.,* 1996, vol. 329, 251-261
- **MORNET, E.** *J. Biol. Chem.,* 1989, vol. 264, 20961-20967
- **JACOBS, C. et al.** *J. Med. Chem.,* 2000, vol. 43, 1841-1851
- **COZZI, P. et al.** *Eur. J. Med. Chem.,* 1991, vol. 26, 423-433
- **BENCZE, W.L. ; BARSKY, L.I.** *J. Med. Pharm. Chem.,* 1962, vol. 5, 1298-1306
- **JOHNSON, A.L. et al.** *J. Med. Chem.,* 1969, vol. 12 (5), 1024-1028
- **HEY, D.H. ; WILLIAMS, J.M.** *J. Chem. Soc.,* 1950, 1678-83

- **BHATNAGAR, A.S. et al.** *J. Steroid Biochem. Mol. Biol.,* 1990, vol. 37, 1021-1027
- **HAUSLER, A. et al.** *J. Steroid Biochem.,* 1989, vol. 34, 567-570
- **DOWSETT, M. et al.** *Clin. Endocrinol. (Oxf.),* 1990, vol. 32, 623-634
- **SANTEN, R.J. et al.** *J. Clin. Endocrinol. Metabol.,* 1991, vol. 73, 99-106
- **FÜRSTNER, A. et al.** *JACS,* 2002, vol. 124, 13856-13863
- **FURUKAWA, N. et al.** *Tet. Lett.,* 1987, vol. 28 (47), 5845-8
- **HEY, D.H. ; WILLIAMS, J.M.** *J.Chem. Soc.,* 1950, 1678-83
- **DUMOUCHEL, S. et al.** *Tetrahedron,* 2003, vol. 59, 8629-8640
- **SARODNICK G. ; KEMPTER G.** *Pharmazie,* 1985, vol. 40 (6), 384-7
- **KELLEY, C.J.** *J.Het.Chem.,* 2001, vol. 38 (1), 11-23
- **HARTMANN, R.W. et al.** *Eur. J. Med. Chem.,* 2003, vol. 38, 363-366
- **KELLEY, C.J. et al.** *J. Het. Chem.,* 2001, vol. 38 (1), 11-23
- **T.W. GREEN.** Protective Groups in Organic Synthesis. Harvard University, John Wiley & Sons, 1981
- **THOMPSON, E.A. JR. ; SITERII, P.K.** *J. Biol. Chem.,* 1974, vol. 249, 5364-5372
- **GAZDAR, A.F. et al.** *Cancer Res.,* 1990, vol. 50, 5488-5496
- **HÄUSLER et al.** *J. Steroid Biochem.,* 1989, vol. 34, 567-570
- **HUISGEN, R. ; SORGE, G.** *Liebigs Ann. Chem.,* 1950, vol. 566, 162-184
- **CHOWDHURY, S. et al.** *Tet. Lett.,* 1999, vol. 40 (43), 7599-7603
- **H.DAHN ; P.ZOLLER.** *Helv.Chim.Acta,* 1952, vol. 35, 1348-1351
- **JAGDMANN, G.E. et al.** *Synth.Comm.,* 1990, vol. 20 (8), 1203-1208
- **LAM, P.Y.S. et al.** *Tet. Lett.,* 1998, vol. 39, 2941-4
- **LAN, J.-B. et al.** *Chem. Commun.,* 2004, 188-9
- **KAUFFMANN, T. et al.** *Chem.Ber.,* 1982, vol. 115, 452-8
- **BREDERECK, H. ; THEILIG, G.** *Chem.Ber.,* 1953, vol. 86, 88-96
- **COZZI, P. et al.** *Eur.J.Med.Chem.,* 1991, vol. 26, 423-433
- **HUTSCHENREUTER, T.U. et al.** *J. Enzyme Inhib. Med. Chem.,* 2004, vol. 19, 17-32
- **HARTMANN, R. W. ; BATZL, C.** *J. Med. Chem.,* 1986, vol. 29, 1362-1369
- **EHMER, P. B. et al.** *J. Steroid Biochem. Mol. Biol.,* 2000, vol. 75, 57-63
- **LOWRY, O. H. et al.** *J Biol Chem,* 1951, vol. 193, 265-275
- **THOMPSON, E. A. ; SIITERI, P. K.** *J. Biol. Chem.,* 1974, vol. 249, 5364-5372
- **LOWRY, O. H. et al.** *J. Biol. Chem.,* 1951, vol. 193, 265-275
- **FOSTER, A. B. et al.** *J Med Chem,* 1983, vol. 26, 50-54
- **GRAVES, P. E. ; SALHANICK, H. A.** *Endocrinology,* 1979, vol. 105, 52-57
- **EHMER, P. B. et al.** *J. Steroid. Biochem. Mol. Biol.,* 2002, vol. 81, 173-179
- **DENNER, K. et al.** *Pharmacogenetics,* 1995, vol. 5, 89-96